# EUROPEAN PATENT APPLICATION

(11) **EP 4 830 787 A1**
(43) Date of publication of application: **28.10.2026**
(21) Application number: 25172739.2
(22) Date of filing: 25.04.2025
(51) Int. Cl.: A61P 35/00, C07K 16/28, C07K 16/30, C07K 16/32, A61K 39/395, A61K 47/68

(54) **COMBINATION THERAPY WITH AN ANTI CD147 ANTIBODY AND AN ANTI TROP2 ANTIBODY DRUG CONJUGATE AND/OR AN ANTI HER2 ANTIBODY DRUG CONJUGATE**

(71) Applicant: Daiichi Sankyo Company, Limited, Tokyo 103-8426 (JP); AstraZeneca UK Limited, Cambridge Biomedical Campus Cambridge CB2 0AA (GB)
(72) Inventor: Watanabe, Sadanori, 103-8426 Tokyo (JP); Hashimoto, Mari, 103-8426 Tokyo (JP); Yokoyama, Miki, 103-8426 Tokyo (JP)
(74) Representative: Marks & Clerk LLP

(57) **Abstract**

The present disclosure relates to an anti-CD147 antibody for use in medicine, and in particular, in the treatment and/or prevention of cancer, by simultaneous or sequential administration with an antibody-drug conjugate comprising a topoisomerase I inhibitor. The disclosure also relates to a pharmaceutical composition comprising an anti-CD147 antibody and an antibody-drug conjugate as above.

## Description

### [Technical Field]

The present disclosure relates to an anti-CD147 antibody for use in a combination therapy.

### [Background Art]

Various approaches have been adopted for the treatment of intractable solid tumor cancers. However, cancers such as pancreatic cancer and liver cancer commonly exhibit resistance to existing treatments. Even with surgical resection or conventional chemotherapy-based treatments, high recurrence rates are observed. Thus, there remains a need for more effective treatments and medications.

It is generally known that combination therapy involving multiple antitumor agents may be effective in the treatment of cancer, and extensive research has been conducted in this area. However, identifying appropriate combinations of drugs that exhibit a synergistic effect, in addition to determining the specific types of cancer against which these combinations are effective, remain a significant challenge.

CD147 has been identified as a potential molecular target for cancer treatment. CD147 is a single-pass transmembrane protein having 2 to 3 immunoglobulin-like domains. CD147 is known to activate downstream signal-related molecules such as MEK, Erk, JAK/STAT, AKT and MAPK family molecules through interactions between multiple CD147 molecules, or through interactions with extracellular or cell membrane surface molecules, such as CD44, integrins, CD98, VEGFR, CypA/B, and MCT1/3/4 (Non-Patent Literature 30). These molecules accelerate the production of proteases such as matrix metalloproteinases, and promote cancer growth, metastasis, and invasion. Moreover, it has been reported that a high level of CD147 expression is associated with a short survival period and a poor prognosis.

Antibodies targeting CD147 such as ABX-CBL and Licartin have been administered clinically to humans. ABX-CBL inhibits binding between CD147 and cyclophilin A, thereby suppressing T-cell activation. Additionally, ABX-CBL demonstrates cytotoxic activity in complement-dependent killing of CD147-positive T cells, along with other normal cells in the bloodstream. Clinical trials targeting graft-versus-host disease (GVHD) have revealed insufficient efficacy of this antibody and a side effect of severe muscle pains. Thus, this antibody has not been approved as a drug (Patent Literature 1, Non- Patent Literature 1).

Licartin is a biopharmaceutical comprising a radioactive isotope, iodine-131, conjugated to the Fab'2 portion of a HAb18 antibody. It has been approved in China for the treatment of liver cancer (Non-Patent Literature 2 and 3). Licartin lacks the Fc region of the antibody responsible for immune-mediated activity, and no immune-mediated toxicity has been reported. However, there are no clinical reports confirming complete eradication of liver cancer.

Other antibodies targeting CD147 have been reported. These include monoclonal antibodies that inhibit biological activities associated with CD147, such as angiogenesis, VEGF production, and matrix metalloproteinases (Patent Literature 2); monoclonal antibodies that suppress T-cell activation (Non-Patent Literature 4); and antibodies binding specifically to CD147 molecules, exhibiting ADCC and CDC activity (Patent Literature 3).

Another potential target for cancer treatment is human TROP2. Immunohistochemical analysis of clinical specimens has shown that TROP2 is overexpressed in various epithelial cell-derived cancers, expression of TROP2 in normal tissues is limited to epithelial cells across various tissues, and the amount of expression is low in normal tissues compared to that in tumor tissues (Non-Patent Literature 10 to 14). It has also been reported that expression of hTROP2 is correlated with poor prognosis in colorectal cancer (Non-Patent literature 10), gastric cancer (Non-Patent Literature 11), pancreatic cancer (Non-Patent Literature 12), oral cancer (Non-Patent Literature 13), and glioma (Non-Patent Literature 14).

Various anti-hTROP2 antibodies have been established to date, and their antitumor effects have been studied. One such antibody exhibits antitumor activity in a nude mouse xenograft model (Patent Literature 6 to 9). However, there remains a need to improve the efficacy of anti-TROP2 antibodies.

HER2 (neu, ErbB-2) is a member of the EGFR (epidermal growth factor receptor) family. HER2 is overexpressed in various cancer types such as breast cancer, gastric cancer and ovarian cancer (Non-patent Literature 15 to 20) and has been reported as a negative prognosis factor for breast cancer (Non-patent Literature 21 and 22). Thus, HER2 is also an attractive target for anti-cancer therapies.

Trastuzumab is a humanized antibody of a mouse anti-HER2 antibody 4D5 (Non-Patent Literature 23 and Patent Literature 10). Trastuzumab binds specifically to the extracellular domain IV of HER2 and induces antibody-dependent cellular cytotoxicity (ADCC) or exerts an anticancer effect via the inhibition of signal transduction from HER2 (Non-Patent Literature 24 and 25). Trastuzumab is highly effective for tumors overexpressing HER2 (Non-Patent Literature 26). Although the therapeutic effect of trastuzumab on breast cancer has been adequately proven (Non-Patent Literature 27), only a minor proportion of patients with breast cancer overexpressing HER2 who have received a wide range of conventional anticancer therapies respond to trastuzumab. The majority of patients in this population have no or only a weak response to trastuzumab treatment.

Antibody-drug conjugates (ADCs) which are composed of a cytotoxic drug conjugated to an antibody enable targeted delivery of the drug to cancer cells, and may thus be expected to cause accumulation of the drug within cancer cells, resulting in the death of the cancer cells (Non-Patent Documents 5 to 9).

One such antibody-drug conjugate is datopotamab deruxtecan, which is composed of a TROP2-targeting antibody and a derivative of exatecan (a topoisomerase I inhibitor). In particular, Patent Literature 4 and 5 provide detailed descriptions of exemplary TROP2-targeting antibody-drug conjugates, including datopotamab deruxtecan (DS-1062). Datopotamab deruxtecan has shown clinical efficacy in multiple tumor types, including lung cancer and breast cancer.

Another antibody-drug conjugate is sacituzumab govitecan (Trodelvy^{®}) which is composed of a TROP2-targeting antibody and SN-38. Sacituzumab govitecan has shown clinical efficacy in the treatment of triple-negative breast cancer. Yet another antibody-drug conjugate is trastuzumab deruxtecan which is composed of a HER2-targeting antibody and a derivative of exatecan (Non-Patent Literature 28 and 29). Trastuzumab deruxtecan (Enhertu^{®}, DS-8201) has shown significant clinical efficacy in HER2-expressing solid tumors, including breast cancer, gastric cancer, colorectal cancer and non-small cell lung cancer. Despite the therapeutic potential of antibody-drug conjugates such as DS-8201, DS-1062 and sacituzumab govitecan in monotherapy, there remains a need to identify combination partners for such antibody-drug conjugates to enhance efficacy of treatment, improve tolerance in patients, reduce dose-dependent toxicity and/or provide an alternative treatment of cancers exhibiting intractability or resistance to existing treatments.

### [Citation List]

### Patent Literature

Patent Literature 1: WO1999/045031
Patent Literature 2: WO2010/036460
Patent Literature 3: WO2017/061602
Patent Literature 4: WO 2015/098099
Patent Literature 5: WO 2020/240467
Patent Literature 6: WO 2008/144891
Patent Literature 7: WO 2011/145744
Patent Literature 8: WO 2011/155579
Patent Literature 9: WO 2013/077458
Patent Literature 10: U.S. Patent No. 5677171

### Non-Patent Literature

Non-Patent Literature 1: Deeg H., et al., J. Blood., pp. 2052-2058, 2001.
Non-Patent Literature 2: Chen Z., et al., Int. J. Radiation Oncology Biol. Phys., pp. 435-444, 2006.
Non-Patent Literature 3: Xu J., et al., Hepatology, pp. 269-276, 2007.
Non-Patent Literature 4: Koch C., et al., International Immunology, pp. 777-786, 1999.
Non-Patent Literature 5: Ducry, L., et al., Bioconjugate Chem. (2010) 21, 5-13.
Non-Patent Literature 6: Alley, S. C., et al., Current Opinion in Chemical Biology (2010) 14, 529-537.
Non-Patent Literature 7: Damle N. K. Expert Opin. Biol. Ther. (2004) 4, 1445-1452.
Non-Patent Literature 8: Senter P. D., et al., Nature Biotechnology (2012) 30, 631-637.
Non-Patent Literature 9: Burris HA., et al., J. Clin. Oncol. (2011) 29(4): 398-405.
Non-Patent Literature 10: Ohmachi T, et al., Clin. Cancer Res., 12(10), 3057-3063 (2006).
Non-Patent Literature 11: Muhlmann G, et al., J. Clin. Pathol., 62(2), 152-158 (2009).
Non-Patent Literature 12: Fong D, et al., Br. J. Cancer, 99(8), 1290-1295 (2008).
Non-Patent Literature 13: Fong D, et al., Mod. Pathol., 21(2), 186-191 (2008).
Non-Patent literature 14: Ning S, et al., Neurol. Sci., 34(10), 1745-1750 (2013).
Non-Patent Literature 15: Hardwick R, et al., Eur. J Surg Oncol. 1997 (23):30-35.
16. Non-Patent Literature 16: Korkaya H, et al., Oncogene. 2008;27(47):6120-6130.
Non-Patent Literature 17: Yano T, et al., Oncol Rep. 2006;15(1):65-71.
Non-Patent Literature 18: Slamon DJ, et al., Science. 1987;235:177-182.
Non-Patent Literature 19: Gravalos C, et al., Ann Oncol 19: 1523-1529, 2008.
Non-Patent Literature 20: Fukushige S et al., Mol Cell Biol 6: 955-958, 1986.
Non-Patent Literature 21: Slamon DJ, et al. Science. 1989;244:707-712.
Non-Patent Literature 22: Kaptain S et al., Diagn Mol Pathol 10:139-152, 2001.
Non-Patent Literature 23: Fendly. et al., Cancer Research 1990(50):1550-1558.
Non-Patent Literature 24: Sliwkowski MX, et al., Semin Oncol. 1999;26(4, Suppl. 12):60-70.
Non-Patent Literature 25: Hudis CA, et al., N Engl J. Med. 357: 39-51, 2007.
Non-Patent Literature 26: Vogel CL, et al., J Clin Oncol. 2002;20(3):719-726.
Non-Patent Literature 27: Baselga et al., J. Clin. Oncol. 14:737-744 (1996).
Non-Patent Literature 28: Ogitani Y. et al., Clinical Cancer Research (2016) 22(20), 5097-5108.
Non-Patent Literature 29: Ogitani Y. et al., Cancer Science (2016) 107, 1039-1046.
Non-Patent Literature 30: K Fukuchi et al., Journal of Oncology, Volume 2022, 17 pages)

### [Summary of Disclosure]

### [Technical Problem]

As described above, antibodies specific for tumor-associated antigens and antibody-drug conjugates are known to have therapeutic effects in cancer patients when used as a monotherapy. However, there is a need for improved therapies for treating cancers, either where such antibodies or antibody-drug conjugates, alone, do not give rise to complete patient remission, or in situations where it is desirable to reduce the necessary dosage of such antibodies or antibody-drug conjugates whilst maintaining therapeutic efficacy.

With particular regard to anti-CD147 antibodies, clinical trial data is scarce, and no results have been reported from clinical trials investigating combination therapies involving anti-CD147 antibodies and other antitumor agents.

Therefore, there is a need to provide further therapies which have superior antitumor effects compared to antibodies and antibody-drug conjugates currently used as a monotherapy. There is also a need to evaluate further the therapeutic potential of anti-CD147 antibodies in combination with other antitumor agents.

### [Solution to Problem]

Through extensive research and analysis, the present inventors have found that a combined administration of an anti-CD147 antibody and an antibody-drug conjugate comprising a topoisomerase I inhibitor, unexpectedly exhibits a superior antitumor effect compared to administration of either the anti-CD147 antibody or the antibody-drug conjugate alone. Specifically, the present inventors have found that combined administration of an anti-CD147 antibody and an antibody-drug conjugate comprising an anti-TROP2 antibody or an anti-HER2 antibody conjugated to a topoisomerase I inhibitor unexpectedly exhibits a superior antitumor effect compared to administration of the anti-CD147 antibody or either of the antibody-drug conjugates alone.

In accordance with the above, the present disclosure generally relates to a combination of i) an anti-CD147 antibody and (ii) an antibody-drug conjugate comprising a topoisomerase I inhibitor for use in medicine, and in particular, for use in the prevention and/or treatment of a cancer. In particular, the present disclosure relates to a combination of i) an anti-CD147 antibody and (ii) an anti-TROP2 antibody-drug conjugate or an anti-HER2 antibody-drug conjugate, the drug comprising a topoisomerase I inhibitor, for use as above. The present disclosure also relates to a pharmaceutical composition comprising i) an anti-CD147 antibody and ii) an anti-hTROP2 antibody-drug conjugate or an anti-HER2 antibody-drug conjugate, the drug comprising a topoisomerase I inhibitor. The present disclosure further relates to a kit comprising components i) and ii) above.

The present disclosure includes the following aspects:
(i) A combination of an anti-CD147 antibody and an antibody-drug conjugate for use in medicine,
   wherein the anti-CD147 antibody is administered to a subject simultaneously or sequentially with an antibody-drug conjugate, and
   wherein the drug moiety of the antibody-drug conjugate comprises a topoisomerase I inhibitor.
(ii) The combination for use according to (i), wherein the use comprises the treatment and/or prevention of a cancer in the subject.
(iii) A therapeutic method comprising administering to a subject in need of treatment an anti-CD147 antibody and an antibody-drug conjugate in combination,
   wherein the anti-CD147 antibody is administered to the subject simultaneously or sequentially with the antibody-drug conjugate, and
   wherein the drug moiety of the antibody-drug conjugate comprises a topoisomerase I inhibitor.
(iv) The combination for use according to (i) or (ii) or the therapeutic method according to (iii), wherein antibody moiety of the antibody-drug conjugate comprises an anti-TROP2 antibody or an anti-HER2 antibody.
More specifically, the present disclosure includes the following aspects:
(1) An anti-CD147 antibody for use in medicine,
   wherein the anti-CD147 antibody is administered to a subject simultaneously or sequentially with an antibody-drug conjugate,
   wherein the antibody moiety of the antibody-drug conjugate comprises an anti-TROP2 antibody, and
   wherein the drug moiety of the antibody-drug conjugate comprises a topoisomerase I inhibitor.
(1a) An antibody-drug conjugate for use in medicine,
   wherein the antibody-drug conjugate is administered to a subject simultaneously or sequentially with an anti-CD147 antibody,
   wherein the antibody moiety of the antibody-drug conjugate comprises an anti-TROP2 antibody, and
   wherein the drug moiety of the antibody-drug conjugate comprises a topoisomerase I inhibitor.
(2) An anti-CD147 antibody for use in medicine,
   wherein the anti-CD147 antibody is administered to a subject simultaneously or sequentially with an antibody-drug conjugate,
   wherein the antibody moiety of the antibody-drug conjugate comprises an anti-HER2 antibody, and
   wherein the drug moiety of the antibody-drug conjugate comprises a topoisomerase I inhibitor.
(2a) An antibody-drug conjugate for use in medicine, wherein the antibody-drug conjugate is administered to a subject simultaneously or sequentially with an anti-CD147 antibody,
   wherein the antibody moiety of the antibody-drug conjugate comprises an anti-HER2 antibody, and
   wherein the drug moiety of the antibody-drug conjugate comprises a topoisomerase I inhibitor;
(3) A combination of an anti-CD147 antibody and an antibody-drug conjugate for use in medicine,
   wherein the anti-CD147 antibody is administered to a subject simultaneously or sequentially with an antibody-drug conjugate,
   wherein the antibody moiety of the antibody-drug conjugate comprises an anti-TROP2 or an anti-HER2 antibody, and
   wherein the drug moiety of the antibody-drug conjugate comprises a topoisomerase I inhibitor.
(4) The anti-CD147 antibody for use according to (1) or (2), the antibody-drug conjugate for use according to (1a) or (2a) or the combination for use according to (3), wherein the use comprises the treatment and/or prevention of a cancer in the subject.
(5) The anti-CD147 antibody for use according to any one of (1), (2) and (4), the antibody-drug conjugate for use according to any one of (1a), (2a) and (4), or the combination for use according to (3) or (4), wherein the anti-CD147 antibody is human or humanised.
(5a) The anti-CD147 antibody for use according to any one of (1), (2), (4) and (5), the antibody-drug conjugate for use according to any one of (1a), (2a), (4) and (5), or the combination for use according to any one of (3) to (5), wherein the anti-CD147 antibody binds to domain 2 of human CD147;
   preferably wherein the anti-CD147 antibody binds to an epitope comprising residues from arginine (Arg) at position 106 to glycine (Gly) at position 165 in the amino acid sequence of SEQ ID NO: 3;
   and more preferably wherein the anti-CD147 antibody binds to an epitope comprising the residues arginine (Arg) at position 106, lysine (Lys) at position 108, alanine (Ala) at position 109, valine (Val) at position 110, lysine (Lys) at position 127, serine (Ser) at position 128, glutamate (Glu) at position 129, serine (Ser) at position 130, valine (Val) at position 131, proline (Pro) at position 132, proline (Pro) at position 133, valine (Val) at position 134, glutamine (Gln) at position 164, and glycine (Gly) at position 165 in the amino acid sequence of SEQ ID NO: 3**.**
(6) The anti-CD147 antibody for use according to any one of (1), (2), (4) to (5) and (5a), the antibody-drug conjugate for use according to any one of (1a), (2a), (4) to (5)and (5a), or the combination for use according to any one of (3) to (5)and (5a), wherein the anti-CD147 antibody comprises a heavy chain variable region and a light chain variable region,
   wherein the heavy chain variable region comprises CDRH1, CDRH2 and CDRH3,
   wherein the CDRH1 consists of an amino acid sequence represented by SEQ ID NO: 7, the CDRH2 consists of an amino acid sequence represented by SEQ ID NO: 8, and the CDRH3 consists of an amino acid sequence represented by SEQ ID NO: 9,
   wherein the light chain variable region comprises CDRL1, CDRL2 and CDRL3, and
   wherein the CDRL1 consists of an amino acid sequence represented by SEQ ID NO: 10, the CDRL2 consists of an amino acid sequence represented by SEQ ID NO: 11, and the CDRL3 consists of an amino acid sequence represented by SEQ ID NO: 12.
(7)The anti-CD147 antibody for use according to any one of (1), (2), (4) to (5), (5a) and (6), the antibody-drug conjugate for use according to any one of (1a), (2a), (4) to (5), (5a) and (6), or the combination for use according to any one of (3) to (5), (5a) and (6), wherein the anti-CD147 antibody comprises the following (a) and (b):
   (a) a heavy chain variable region selected from the group consisting of the following (a1) to (a4):
      (a1) a heavy chain variable region consisting of amino acid residues at positions 20 to 136 in an amino acid sequence represented by SEQ ID NO: 13;
      (a2) a heavy chain variable region consisting of amino acid residues at positions 20 to 136 in an amino acid sequence represented by SEQ ID NO: 14;
      (a3) an amino acid sequence having at least 80% or more sequence identity with the sequence of a framework region outside each CDR sequence in the sequence of (a1) or (a2); and
      (a4) an amino acid sequence in which one or several amino acids are deleted, substituted, or added in the sequence of a framework region outside each CDR sequence in the sequence according to any one of (a1) to (a3); and
   (b) a light chain variable region selected from the group consisting of the following (b1) to (b5):
      (b1) a light chain variable region consisting of amino acid residues at positions 21 to 128 in an amino acid sequence represented by SEQ ID NO: 15;
      (b2) a light chain variable region consisting of amino acid residues at positions 21 to 128 in an amino acid sequence represented by SEQ ID NO: 16;
      (b3) a light chain variable region consisting of amino acid residues at positions 21 to 128 in an amino acid sequence represented by SEQ ID NO: 17;
      (b4) an amino acid sequence having at least 80% or more sequence identity with the sequence of a framework region outside each CDR sequence in the sequence according to any one of (b1) to (b3); and
      (b5) an amino acid sequence in which one or several amino acids are deleted, substituted, or added in the sequence of a framework region outside each CDR sequence in the sequence according to any one of (b1) to (b4).
(8) The anti-CD147 antibody for use according to (7), the antibody-drug conjugate for use according to (7), or the combination for use according to (7), wherein the anti-CD147 antibody comprises a heavy chain variable region and a light chain variable region selected from the following (A) to (C):
   (A) a heavy chain variable region consisting of amino acid residues at positions 20 to 136 in the amino acid sequence represented by SEQ ID NO: 13, and a light chain variable region consisting of amino acid residues at positions 21 to 128 in the amino acid sequence represented by SEQ ID NO: 15;
   (B) a heavy chain variable region consisting of amino acid residues at positions 20 to 136 in the amino acid sequence represented by SEQ ID NO: 14, and a light chain variable region consisting of amino acid residues at positions 21 to 128 in the amino acid sequence represented by SEQ ID NO: 16; and
   (C) a heavy chain variable region consisting of amino acid residues at positions 20 to 136 in the amino acid sequence represented by SEQ ID NO: 14, and a light chain variable region consisting of amino acid residues at positions 21 to 128 in the amino acid sequence represented by SEQ ID NO: 17.
(9) The anti-CD147 antibody for use according to (8), the antibody-drug conjugate for use according to (8) or the combination for use according to (8), wherein the anti-CD147 antibody comprises a heavy chain variable region consisting of amino acid residues at positions 20 to 136 in the amino acid sequence represented by SEQ ID NO: 13, and a light chain variable region consisting of amino acid residues at positions 21 to 128 in the amino acid sequence represented by SEQ ID NO: 15.
(10) The anti-CD147 antibody for use according to (7) or (8), the antibody-drug conjugate for use according to (7) or (8), or the combination for use according to (7) or (8), wherein the anti-CD147 antibody comprises a heavy chain and a light chain selected from one of the following (D) to (F):
   (D) a heavy chain consisting of amino acid residues at positions 20 to 463 in the amino acid sequence represented by SEQ ID NO: 13, and a light chain consisting of amino acid residues at positions 21 to 234 in the amino acid sequence represented by SEQ ID NO: 15;
   (E) a heavy chain consisting of amino acid residues at positions 20 to 463 in the amino acid sequence represented by SEQ ID NO: 14, and a light chain consisting of amino acid residues at positions 21 to 234 in the amino acid sequence represented by SEQ ID NO: 16; and
   (F) a heavy chain consisting of amino acid residues at positions 20 to 463 in the amino acid sequence represented by SEQ ID NO: 14, and a light chain consisting of amino acid residues at positions 21 to 234 in the amino acid sequence represented by SEQ ID NO: 17.
(11) The anti-CD147 antibody for use according to (10), the antibody-drug conjugate for use according to (10) or the combination for use according to (10), wherein the anti-CD147 antibody comprises a heavy chain consisting of amino acid residues at positions 20 to 463 in the amino acid sequence represented by SEQ ID NO: 13, and a light chain consisting of amino acid residues at positions 21 to 234 in the amino acid sequence represented by SEQ ID NO: 15.
(11a) The anti-CD147 antibody for use according to any one of (1), (2), (4) or (5), the antibody-drug conjugate for use according to any one of (1a), (2a), (4) or (5), or the combination for use according to any one of (3) to (5), wherein the anti-CD147 antibody binds to an epitope comprising an amino acid sequence according to SEQ ID NO: 18 or an amino acid sequence in which one or several amino acids are deleted, substituted or added in the sequence of SEQ ID NO: 18.
(11b) The anti-CD147 antibody for use according to any one of (1), (2), (4), (5) or (11a), the antibody-drug conjugate for use according to any one of (1a), (2a), (4), (5) or (11a), or the combination for use according to any one of (3) to (5) or (11a), wherein the anti-CD147 antibody comprises a heavy chain variable region and a light chain variable region,
   wherein the heavy chain variable region comprises CDRH1, CDRH2 and CDRH3,
   wherein the CDRH1 consists of an amino acid sequence represented by SEQ ID NO: 21, the CDRH2 consists of an amino acid sequence represented by SEQ ID NO: 22, and the CDRH3 consists of an amino acid sequence represented by SEQ ID NO: 23,
   wherein the light chain variable region comprises CDRL1, CDRL2 and CDRL3, and
   wherein the CDRL1 consists of an amino acid sequence represented by SEQ ID NO: 24, the CDRL2 consists of an amino acid sequence represented by SEQ ID NO: 25, and the CDRL3 consists of an amino acid sequence represented by SEQ ID NO: 26.
(11c) The anti-CD147 antibody for use according to (11b), the antibody-drug conjugate for use according to (11b), or the combination for use according to (11b), wherein the anti-CD147 antibody comprises the following (c) and (d):
   (c) a heavy chain variable region selected from the group consisting of the following (c1) to (c4):
      (c1) a heavy chain variable region consisting of amino acid residues at positions 20 to 140 in an amino acid sequence represented by SEQ ID NO: 27;
      (c2) a heavy chain variable region consisting of amino acid residues at positions 20 to 140 in an amino acid sequence represented by SEQ ID NO: 28;
      (c3) an amino acid sequence having at least 80% or more sequence identity with the sequence of a framework region outside each CDR sequence in the sequence of (c1) or (c2); and
      (c4) an amino acid sequence in which one or several amino acids are deleted, substituted, or added in the sequence of a framework region outside each CDR sequence in the sequence according to any one of (c1) to (c3); and
   (d) a light chain variable region selected from the group consisting of the following (d1) to (d3):
      (d1) a light chain variable region consisting of amino acid residues at positions 21 to 128 in an amino acid sequence represented by SEQ ID NO: 29;
      (d2) an amino acid sequence having at least 80% or more sequence identity with the sequence of a framework region outside each CDR sequence in the sequence of (d1); and
   (d3) an amino acid sequence in which one or several amino acids are deleted, substituted, or added in the sequence of a framework region outside each CDR sequence in the sequence of (d1) or (d2).
(11d) The anti-CD147 antibody for use according to (11b) or (11c), the antibody-drug conjugate for use according to (11b) or (11c), or the combination for use according to (11b) or (11c), wherein the anti-CD147 antibody comprises a heavy chain variable region and a light chain variable region selected from one of the following (G) and (H):
   (G) a heavy chain variable region consisting of amino acid residues at positions 20 to 136 in the amino acid sequence represented by SEQ ID NO: 27, and
      a light chain variable region consisting of amino acid residues at positions 21 to 128 in the amino acid sequence represented by SEQ ID NO: 29; or
   (H) a heavy chain variable region consisting of amino acid residues at positions 20 to 136 in the amino acid sequence represented by SEQ ID NO: 28, and
      a light chain variable region consisting of amino acid residues at positions 21 to 128 in the amino acid sequence represented by SEQ ID NO: 29.
(11e) The anti-CD147 antibody for use according to any one of (11b) to (11d), the antibody-drug conjugate for use according to any one of (11b) to (11d), or the combination for use according to any one of (11b) to (11d), wherein the anti-CD147 antibody comprises a heavy chain and a light chain selected from one of the following (I) and (J):
   (I) a heavy chain consisting of amino acid residues at positions 20 to 466 in the amino acid sequence represented by SEQ ID NO: 27, and
      a light chain consisting of amino acid residues at positions 21 to 234 in the amino acid sequence represented by SEQ ID NO: 29; or
   (J) a heavy chain consisting of amino acid residues at positions 20 to 467 in the amino acid sequence represented by SEQ ID NO: 28, and
      a light chain consisting of amino acid residues at positions 21 to 234 in the amino acid sequence represented by SEQ ID NO: 29.
(12) The anti-CD147 antibody for use according to any one of (1), (2), (4) and (5), the antibody-drug conjugate for use according to any one of (1a), (2a), (4) and (5), or the combination for use according to any one of (3) to (5), wherein the anti-CD147 antibody competes with at least one antibody selected from the group consisting of the following (K) and (L) for binding to human CD147:
   (K) an anti-CD147 antibody comprising a heavy chain variable region consisting of an amino acid sequence represented by SEQ ID NO: 5, and a light chain variable region consisting of an amino acid sequence represented by SEQ ID NO: 6; and
   (L) an anti-CD147 antibody comprising a heavy chain variable region consisting of an amino acid sequence represented by SEQ ID NO: 19, and a light chain variable region consisting of an amino acid sequence represented by SEQ ID NO: 20.
(13) The anti-CD147 antibody for use according to any one of (1), (2), (4) and (5), the antibody-drug conjugate for use according to any one of (1a), (2a), (4) and (5), or the combination for use according to any one of (3) to (5), wherein the anti-CD147 antibody binds to an epitope to which at least one antibody selected from the group consisting of the following (K) and (L) binds:
   (K) an anti-CD147 antibody comprising a heavy chain variable region consisting of an amino acid sequence represented by SEQ ID NO: 5, and a light chain variable region consisting of amino acid sequence represented by SEQ ID NO: 6; and
   (L) an anti-CD147 antibody comprising a heavy chain variable region consisting of an amino acid sequence represented by SEQ ID NO: 19, and a light chain variable region consisting of an amino acid sequence represented by SEQ ID NO: 20.
(14) The anti-CD147 antibody for use according to any one of (1), (2), (4) to (5), (5a), (6) to (9), (11a) to (11d), and (12) to (13), the antibody-drug conjugate for use according to any one of (1a), (2a), (4) to (5), (5a), (6) to (9), (11a) to (11d), and (12) to (13, or the combination for use according to any one of (3) to (5), (5a), (6) to (9), (11a) to (11d), and 12 to (13), wherein the anti-CD147 antibody comprises an antigen-binding fragment that is selected from the group consisting of Fab, F(ab')2, Fab', diabody and Fv.
(15) The anti-CD147 antibody for use according to any one of (1), (2), (4) to (5), (5a), (6) to (9), (11a) to (11d), and (12) to (13), the antibody-drug conjugate for use according to any one of (1a), (2a), (4) to (5), (5a), and (6) to (9), (11a) to (11d), and (12) to (13), or the combination for use according to any one of (3) to (5), (5a), and (6) to (9), (11), (11a) to (11d), and (12) to (13), wherein the anti-CD147 antibody comprises an antigen-binding fragment that is scFv.
(16) The anti-CD147 antibody for use according to any one of (1), (2), (4) to (5), (5a), (6) to (9), (11a) to (11d),and (12) to (13), the antibody-drug conjugate for use according to any one of (1a), (2a), (4) to (5), (5a), (6) to (9), (11a) to (11d),and (12) to (13), or the combination for use according to any one of (3) to (5), (5a), (6) to (9), (11a) to (11d), and (12) to (13), wherein the anti-CD147 antibody comprises an antigen-binding fragment that is VHH.
(17) The anti-CD147 antibody for use according to any one of (1), (2), (4) to (5), (5a), (6) to (11), (11a) to (11e), and (12) to (16), the antibody-drug conjugate for use according to any one of (1a), (2a), (4) to (5), (5a), (6) to (11), (11a) to (11e), and (12) to (16), or the combination for use according to any one of (3) to (5), (5a), (6) to (11), (11a) to (11e), (12) to (16), wherein a topoisomerase I inhibitor represented by the following formula is released from the antibody-drug conjugate to express an antitumor activity: 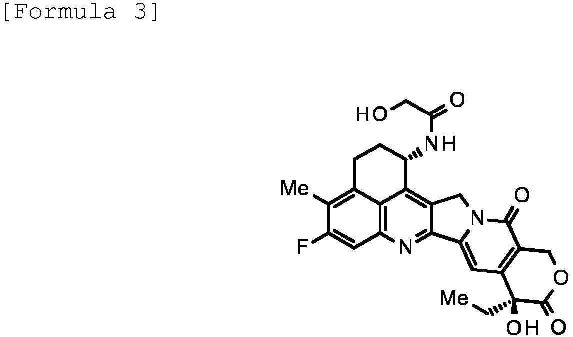
(18) The anti-CD147 antibody for use according to any one of (1), (2), (4) to (5), (5a), (6) to (11), (11a) to (11e), and (12) to (17), the antibody-drug conjugate for use according to any one of (1a), (2a), (4) to (5), (5a), (6) to (11), (11a) to (11e), and (12) to (17), or the combination for use according to any one of (3) to (5), (5a), (6) to (11), (11a) to (11e), (12) to (17), wherein antibody-drug conjugate comprises a linker and a drug, the linker and drug forming a drug-linker,
   wherein the drug represented by the following formula is conjugated to the antibody of the antibody-drug conjugate via the linker with the nitrogen atom of the amino group at position 1 as a connecting position,
   preferably, wherein the drug-linker is conjugated to the antibody via a thioether bond,
   and more preferably, wherein the linker comprises a tetrapeptide amino acid sequence according to SEQ ID NO: 53: 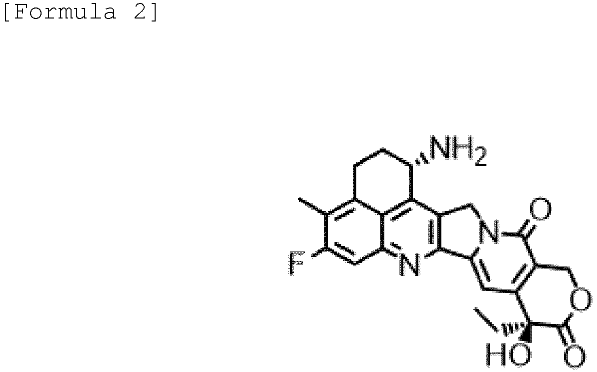
(19) The anti-CD147 antibody for use according to any one of (1), and (4) to (5), (5a), (6) to (11), (11a) to (11e), and (12) to (18) as dependent on (1), the antibody-drug conjugate for use according to any one of (1a) and (4) to (5), (5a), (6) to (11), (11a) to (11e), and (12) to (18) as dependent on (1a), or the combination for use according to any one of (3) to (5), (5a), (6) to (11), (11a) to (11e), and (12) to (18) as dependent on (3), wherein the anti-TROP2 antibody-drug conjugate comprises a linker and a drug, the anti-TROP2 antibody being connected to the drug via the linker, the linker and drug forming a drug-linker, and wherein the drug-linker is represented by the following formula: 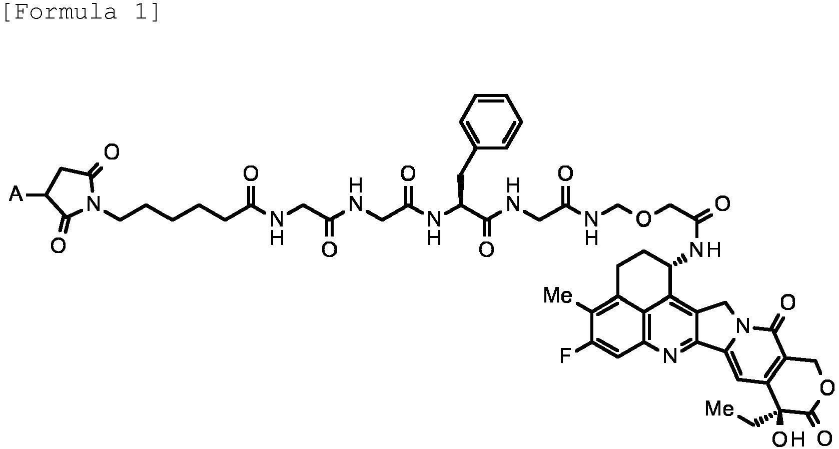 wherein A represents a connecting position to the anti-TROP antibody, and wherein the drug-linker is conjugated to the anti-TROP2 antibody via a thioether bond.
(20) The anti-CD147 antibody for use according to any one of (1), and (4) to (5), (5a), (6) to (11), (11a) to (11e), and (12) to (18) as dependent on (1), and (19), the antibody-drug conjugate for use according to any one of (1a), and (4) to (5), (5a), (6) to (11), (11a) to (11e), and (12) to (18) as dependent on (1a), and 19, or the combination for use according to any one of (3), and (4) to (5), (5a), (6) to (11), (11a) to (11e), and (12) to (18) as dependent on (3), and (19), wherein the anti-TROP2 antibody comprises a heavy chain variable region and a light chain variable region,
   wherein the heavy chain variable region comprises CDRH1, CDRH2 and CDRH3,
   wherein the CDRH1 consists of an amino acid sequence represented by SEQ ID NO: 30, the CDRH2 consists of an amino acid sequence represented by SEQ ID NO: 31, and the CDRH3 consists of an amino acid sequence represented by SEQ ID NO: 32,
   wherein the light chain variable region comprises CDRL1, CDRL2 and CDRL3, and
   wherein the CDRL1 consists of an amino acid sequence represented by SEQ ID NO: 33, the CDRL2 consists of an amino acid sequence represented by SEQ ID NO: 34, and the CDRL3 consists of an amino acid sequence represented by SEQ ID NO: 35.
(21) The anti-CD147 antibody for use according to (20), the antibody-drug conjugate for use according to (20), or the combination for use according to (20), wherein the anti-TROP2 antibody comprises:
   a heavy chain variable region consisting of an amino acid sequence represented by SEQ ID NO: 36, and
   a light chain variable region consisting of an amino acid sequence represented by SEQ ID NO: 37.
(22) The anti-CD147 antibody for use according to (20) or (21), the antibody-drug conjugate for use according to(20) or (21), or the combination for use according to (20) or (21), wherein the anti-TROP2 antibody or comprises:
   a heavy chain consisting of an amino acid sequence represented by SEQ ID NO: 38 and a light chain consisting of an amino acid sequence represented by SEQ ID NO: 39.
(23) The anti-CD147 antibody for use according to any one of (19) to (22), the antibody-drug conjugate for use according to any one of (19) to (22), or the combination for use according to any one of (19) to (22), wherein the average number of units of the drug-linker conjugated per anti-TROP2 antibody molecule in the anti-TROP2 antibody-drug conjugate is in the range of from 2 to 8, and preferably, in the range of from 3.5 to 4.5.
(24) The anti-CD147 antibody for use according to any one of (19) to (23), the antibody-drug conjugate for use according to any one of (19) to (23), or the combination for use according to any one of (19) to (23), wherein the anti-TROP2 antibody-drug conjugate is datopotamab deruxtecan.
(25) The anti-CD147 antibody for use according to any one of (2), and (4) to (5), (5a), (6) to (11), (11a) to (11e), and (12) to (18) as dependent on (2), the antibody-drug conjugate for use according to any one of (2a) and (4) to (5), (5a), (6) to (11), (11a) to (11e), and (12) to (18) as dependent on (2a), or the combination for use according to any one of (3), and (4) to (5), (5a), (6) to (11), (11a) to (11e), and (12) to (18)as dependent on (3), wherein the anti-HER2 antibody-drug conjugate comprises a linker and a drug, the anti-HER2 antibody being connected to the drug via the linker, the linker and drug forming a drug-linker, and wherein the drug-linker is represented by the following formula: 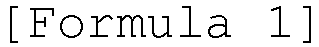 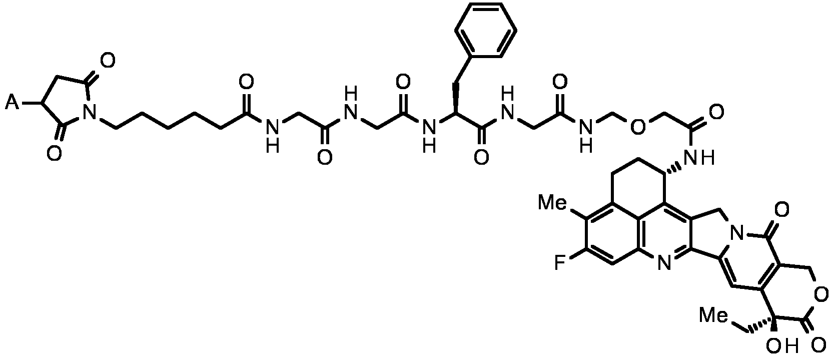 wherein A represents the connecting position to the anti-HER2 antibody, and wherein the drug-linker is conjugated to the anti-HER2 antibody via a thioether bond.
(26) The anti-CD147 antibody for use according to any one of (2), and (4) to (5), (5a), (6) to (11), (11a) to (11e), and (12) to (18) as dependent on (2), and (25), the antibody-drug conjugate for use according to any one of (2a), and (4) to (5), (5a), (6) to (11), (11a) to (11e), and (12) to (18) as dependent on (2a), and (25), or the combination for use according to any one of (3), and (4) to (5), (5a), (6) to (11), (11a) to (11e), and (12) to (18) as dependent on (3), and (25), wherein the anti-HER2 antibody comprises a heavy chain variable region and a light chain variable region,
   wherein the heavy chain variable region comprises CDRH1, CDRH2 and CDRH3,
   wherein the CDRH1 consists of an amino acid sequence represented by SEQ ID NO: 43, the CDRH2 consists of an amino acid sequence represented by SEQ ID NO: 44, and the CDRH3 consists of an amino acid sequence represented by SEQ ID NO: 45,
   wherein the light chain variable region comprises CDRL1, CDRL2 and CDRL3, and
   wherein the CDRL1 consists of an amino acid sequence represented by SEQ ID NO: 46, the CDRL2 consists of an amino acid sequence represented by SEQ ID NO: 47, and the CDRL3 consists of an amino acid sequence represented by SEQ ID NO: 48.
(27) The anti-CD147 antibody for use according to (26), the antibody-drug conjugate for use according to (26), or the combination for use according to (26), wherein the anti-HER2 antibody comprises:
   a heavy chain variable region consisting of an amino acid sequence represented by SEQ ID NO: 49, and
   a light chain variable region consisting of an amino acid sequence represented by SEQ ID NO: 50.
(28) The anti-CD147 antibody for use according to (26) or (27), the antibody-drug conjugate for use according to (26) or (27), or the combination for use according to (26) or (27), wherein the anti-HER2 antibody comprises:
   a heavy chain consisting of an amino acid sequence represented by SEQ ID NO: 51 and a light chain consisting of an amino acid sequence represented by SEQ ID NO: 52.
(29) The anti-CD147 antibody for use according to any one of (25) to (28), the antibody-drug conjugate for use according to any one of (25) to (28), or the combination for use according to any one of (25) to (28), wherein the average number of units of the drug-linker conjugated per anti-HER2 antibody molecule in the anti-HER2 antibody-drug conjugate is in the range of from 2 to 8, preferably in the range of from 7 to 8, and more preferably, in the range of from 7.5 to 8.
(30) The anti-CD147 antibody for use according to any one of (25) to (29), the antibody-drug conjugate for use according to any one of (25) to (29), or the combination for use according to any one of (25) to (29), wherein the anti-HER2 antibody-drug conjugate is trastuzumab deruxtecan.
(31) The anti-CD147 antibody for use according to any one of (1), (2,) (4) to (5), (5a), (6) to (11), (11a) to (11e), (12) to (16), (20) to (23) and (26) to (29), the antibody-drug conjugate for use according to any one of (1a), (2a,) (4) to (5), (5a), (6) to (11), (11a) to (11e), (12) to (16), (20) to (23) and (26) to (29), or the combination for use according to any one of (3) to (5), (5a), (6) to (11), (11a) to (11e), (12) to (16), (20) to (23) and (26) to (29), wherein a topoisomerase I inhibitor represented by the following formula is released from the antibody-drug conjugate to express an antitumor activity: 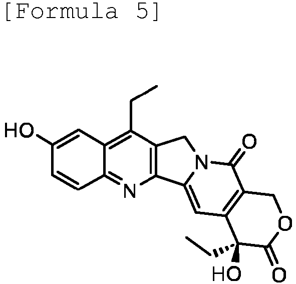
(31a) The anti-CD147 antibody for use according to any one of (1), (2,) (4) to (5), (5a), (6) to (11), (11a) to (11e), (12) to (16), (20) to (23), (26) to (29) and (31), the antibody-drug conjugate for use according to any one of (1a), (2a,) (4) to (5), (5a), (6) to (11), (11a) to (11e), (12) to (16), (20) to (23), (26) to (29) and (31), or the combination for use according to any one of (3) to (5), (5a), (6) to (11), (11a) to (11e), (12) to (16), (20) to (23), (26) to (29) and (31), wherein the anti-TROP2 antibody-drug conjugate or the anti-HER2 antibody drug conjugate comprises a linker and a drug, the anti-TROP2 antibody or the anti-HER2 antibody being connected to the drug via the linker, the linker and drug forming a drug-linker, and wherein the drug-linker is represented by the following formula: 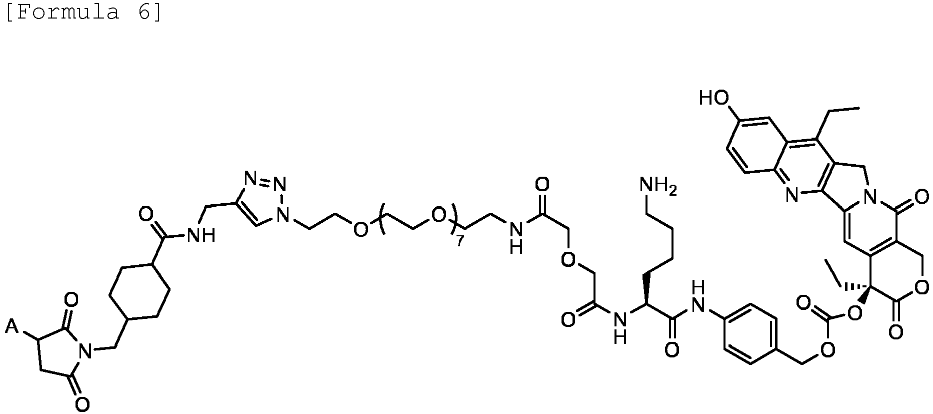 wherein A represents a connecting position to the anti-TROP antibody or the anti-HER2 antibody, and wherein the drug-linker is conjugated to the anti-TROP2 antibody or the anti-HER2 antibody via a thioether bond.
(32) The anti-CD147 antibody for use according to (31a), the antibody-drug conjugate for use according to (31a), or the combination for use according to (31a), wherein A represents the connecting position to an anti-TROP antibody, and wherein the anti-TROP2 antibody-drug conjugate comprises a heavy chain amino acid sequence according to SEQ ID NO**:** 54 and a light chain amino acid sequence according to SEQ ID NO: 55 (sacituzumab govitecan).
(33) The anti-CD147 antibody for use according to any one of (1), (2) (4) to (5), (5a), (6) to (11), (11a) to (11e), (12) to (31), (31a) and (32), the antibody-drug conjugate for use according to any one of (1a), (2a) (4) to (5), (5a), (6) to (11), (11a) to (11e), (12) to (31), (31a) and (32), or the combination for use according to any one of (3) to (5), (5a), (6) to (11), (11a) to (11e), (12) to (31), (31a) and (32), wherein the anti-CD147 antibody, anti-TROP2 antibody and/or anti-HER2 antibody each comprises a heavy chain, and wherein 1 or 2 amino acid residues are deleted from the carboxyl terminus of the heavy chain,
   optionally, wherein the anti-CD147 antibody, anti-TROP2 antibody and/or anti-HER2 antibody each comprises a first and a second heavy chain, and wherein each of the first and the second heavy chains has a deletion of one amino acid residue at the carboxyl terminus.
(34) The anti-CD147 antibody for use according to any one of (1), (2), (4) to (5), (5a), (6) to (11), (11a) to (11e), (12) to (31), (31a) and (32) to (33), the antibody-drug conjugate for use according to any one of (1a), (2a), (4) to (5), (5a), (6) to (11), (11a) to (11e), (12) to (31), (31a) and (32) to (33), or the combination for use according to any one of (3) to (5), (5a), (6) to (11), (11a) to (11e), (12) to (31), (31a) and (32) to (33), wherein the anti-CD147 antibody, anti-TROP2 antibody and/or anti-HER2 antibody each comprises a heavy chain, and wherein an amino acid residue at the carboxyl terminus of the heavy chain is amidated.
(35) The anti-CD147 antibody for use according to any one of (4) to (5), (5a), (6) to (11), (11a) to (11e), (12) to (24), (31), (31a) and (32) to (34), the antibody-drug conjugate for use according to any one of (4) to (5), (5a), (6) to (11), (11a) to (11e), (12) to (24), (31), (31a) and (32) to (34), or the combination for use according to any one of (4) to (5), (5a), (6) to (11), (11a) to (11e), (12) to (24), (31), (31a) and (32) to (34), wherein the antibody-drug-conjugate comprises an anti-TROP2 antibody drug conjugate, and wherein the cancer is selected from the group consisting of: breast cancer (including triple negative breast cancer and hormone receptor (HR)-positive, HER2-negative breast cancer), lung cancer (including small cell lung cancer and non-small cell lung cancer), colorectal cancer (also called colon and rectal cancer, and including colon cancer and rectal cancer), gastric cancer (also called gastric adenocarcinoma), esophageal cancer, head-and-neck cancer (including salivary gland cancer and pharyngeal cancer), esophagogastric junction adenocarcinoma, biliary tract cancer (including bile duct cancer), Paget's disease, pancreatic cancer (including pancreatic ductal adenocarcinoma) , ovarian cancer, uterine carcinosarcoma, urothelial cancer, prostate cancer, bladder cancer, gastrointestinal stromal tumor, cervical cancer (including uterine cervix cancer), squamous cell carcinoma, peritoneal cancer, liver cancer, hepatocellular cancer, corpus uteri carcinoma, kidney cancer, vulval cancer, thyroid cancer, penis cancer, leukemia, malignant lymphoma, plasmacytoma, myeloma, glioblastoma multiforme, osteosarcoma, sarcoma, endometrial cancer, and melanoma;
   preferably wherein the cancer is selected from non-small cell lung cancer, triple negative breast cancer, hepatocellular cancer, pancreatic ductal adenocarcinoma, and colorectal cancer;
   and more preferably, wherein the cancer is selected from triple negative breast cancer and non-small cell lung cancer.
(36) The anti-CD147 antibody for use according to any one of (4) to (5), (5a), (6) to (11), (11a) to (11e), (12) to (18), (25) to (31), (31a) and (33) to (34), the antibody-drug conjugate for use according to any one of ( (4) to (5), (5a), (6) to (11), (11a) to (11e), (12) to (18), (25) to (31), (31a) and (33) to (34), or the combination for use according to any one of (4) to (5), (5a), (6) to (11), (11a) to (11e), (12) to (18), (25) to (31), (31a) and (33) to (34), wherein the antibody-drug-conjugate comprises an anti-HER2 antibody drug conjugate, and wherein the cancer is selected from the group consisting of: breast cancer (including hormone receptor (HR)-positive breast cancer), lung cancer (including small cell lung cancer and non-small cell lung cancer), colorectal cancer (also called colon and rectal cancer, and including colon cancer and rectal cancer), gastric cancer (also called gastric adenocarcinoma), esophageal cancer, head-and-neck cancer (including salivary gland cancer and pharyngeal cancer), esophagogastric junction adenocarcinoma, biliary tract cancer (including bile duct cancer), Paget's disease, pancreatic cancer including pancreatic ductal adenocarcinoma, ovarian cancer, uterine carcinosarcoma, urothelial cancer, prostate cancer, bladder cancer, gastrointestinal stromal tumor, cervical cancer (including uterine cervix cancer), squamous cell carcinoma, peritoneal cancer, liver cancer, hepatocellular cancer, corpus uteri carcinoma, kidney cancer, vulval cancer, thyroid cancer, penis cancer, leukemia, malignant lymphoma, plasmacytoma, myeloma, glioblastoma multiforme, osteosarcoma, sarcoma, endometrial cancer, and melanoma;
   preferably wherein the cancer is selected from the group consisting of HER2-positive breast cancer, lung cancer including non-small cell lung cancer, gastric cancer, hepatocellular cancer, pancreatic ductal adenocarcinoma, and colorectal cancer;
   and more preferably, wherein the cancer is gastric cancer.
(37) The anti-CD147 antibody for use according to any one of (4) to (5), (5a), (6) to (11), (11a) to (11e), (12) to (31), (31a) and (32) to (34), the antibody-drug conjugate for use according to (4) to (5), (5a), (6) to (11), (11a) to (11e), (12) to (31), (31a) and (32) to (34), or the combination for use according to any one of (4) to (5), (5a), (6) to (11), (11a) to (11e), (12) to (31), (31a) and (32) to (34), wherein the cancer is selected from: non-small cell lung cancer; breast cancer, preferably triple negative breast cancer, and more preferably, triple negative breast cancer without a BRACA1 mutation; and gastric cancer.
(37a) The anti-CD147 antibody for use according to any one of (4) to (5), (5a), (6) to (11), (11a) to (11e), (12) to (31), (31a) and (32) to (37), the antibody-drug conjugate for use according to any one of (4) to (5), (5a), (6) to (11), (11a) to (11e), (12) to (31), (31a) and (32) to (37), or the combination for use according to any one of (4) to (5), (5a), (6) to (11), (11a) to (11e), (12) to (31), (31a) and (32) to (37), wherein the cancer is a CD147-expressing cancer.
(38) The anti-CD147 antibody for use according to any one of (1), (2), (4) to (5), (5a), (6) to (11), (11a) to (11e), (12) to (31), (31a), (32) to (37) and (37a), the antibody-drug conjugate for use according to any one of (1a), (2a), (4) to (5), (5a), (6) to (11), (11a) to (11e), (12) to (31), (31a), (32) to (37) and (37a), or the combination for use according to any one of (3) to (5), (5a), (6) to (11), (11a) to (11e), (12) to (31), (31a), (32) to (37) and (37a), wherein the anti-CD147 antibody and the antibody-drug conjugate are contained separately in different formulations for simultaneous or sequential administration, or wherein the anti-CD147 antibody and the antibody-drug conjugate are contained in the same formulation for simultaneous administration.
(39) The anti-CD147 antibody for use according to any one of (1), (2), (4) to (5), (5a), (6) to (11), (11a) to (11e), (12) to (31), (31a), (32) to (37), (37a) and (38), the antibody-drug conjugate for use according to any one of (1a), (2a), (4) to (5), (5a), (6) to (11), (11a) to (11e), (12) to (31), (31a), (32) to (37), (37a) and (38), or the combination for use according to any one of (3) to (5), (5a), (6) to (11), (11a) to (11e), (12) to (31), (31a), (32) to (37), (37a) and (38), wherein the anti-CD147 antibody is administered to the subject at a dosage of from about 0.1 to about 50 mg/kg, or from about 1 to about 40 mg/kg, or about 20 mg/kg, or about 40 mg/kg, optionally,
   wherein the anti-CD147 antibody is administered to the subject daily, every other day, twice per week, three times per week, four times per week, five times per week, six times per week, once per week, once every two weeks, once every three weeks, once every four weeks, once every five weeks, once every six weeks, once every seven weeks, once every eight weeks, once every nine weeks, once every ten weeks, once every eleven weeks, once every twelve weeks, twice per year, or once per year.
(40) A pharmaceutical composition comprising:
   i) an anti-CD147 antibody; and
   ii) an antibody-drug conjugate;
      wherein the antibody moiety of the antibody-drug conjugate comprises an anti-TROP2 antibody or an anti-HER2 antibody, and
      wherein the drug moiety of the antibody-drug conjugate comprises a topoisomerase I inhibitor.
(40a) A pharmaceutical combination comprising:
   i) an anti-CD147 antibody; and
   ii) an antibody-drug conjugate;
      wherein the antibody moiety of the antibody-drug conjugate comprises an anti-TROP2 antibody or an anti-HER2 antibody, and
      wherein the drug moiety of the antibody-drug conjugate comprises a topoisomerase I inhibitor.
(40b) The pharmaceutical composition according to (40) or the pharmaceutical combination according to (40a) for use in the treatment and/or prevention of a cancer in a subject.
(41) A kit suitable for the prevention and/or treatment of cancer, the kit comprising:
   i) an anti-CD147 antibody or; and
   ii) an antibody-drug conjugate;
      wherein the antibody moiety of the antibody-drug conjugate comprises an anti-TROP2 antibody or an anti-HER2 antibody, and
      wherein the drug moiety of the antibody-drug conjugate comprises a topoisomerase I inhibitor.
(42) The pharmaceutical composition according to (40), the pharmaceutical combination according to (40a), the pharmaceutical composition for use according to (40b), the pharmaceutical combination for use according to (40b), or the kit according to (41), wherein the anti-CD147 antibody is as defined in any one of (5), (5a), (6) to (11), (11a) to (11e), (12) to (16), and (33) to (34) , wherein the anti-TROP2 antibody-drug conjugate is as defined in any one of (17) to (24) (31), (31a), and (32) to (34), and/or wherein the anti-HER2 antibody-drug conjugate is as defined in any one of (17), (18),(25) to (31), (31a), and (33) to (34).
(42a) The pharmaceutical composition for use according to (40b) or (42), the pharmaceutical combination for use according to (40b) or (42), or the kit according to (41) or (42), wherein the cancer is as defined in any one of (35) to (37)and (37a)).
The present disclosure also includes the following aspects:
(A1) A therapeutic method comprising administering an anti-CD147 antibody and an antibody-drug conjugate in combination to a subject in need thereof,
   wherein the anti-CD147 antibody is administered to the subject simultaneously or sequentially with the antibody-drug conjugate,
   wherein the antibody moiety of the antibody-drug conjugate comprises an anti-TROP2 antibody, and
   wherein the drug moiety of the antibody-drug conjugate comprises a topoisomerase I inhibitor.
(A2) A therapeutic method comprising administering an anti-CD147 antibody and an antibody-drug conjugate in combination to a subject in need thereof,
   wherein the anti-CD147 antibody is administered to the subject simultaneously or sequentially with the antibody-drug conjugate,
   wherein the antibody moiety of the antibody-drug conjugate comprises an anti-HER2 antibody, and
   wherein the drug moiety of the antibody-drug conjugate comprises a topoisomerase I inhibitor.
(A3) The method according to (A1) or (A2), wherein the method is for preventing or treating cancer in the subject.
(A4) The method according to (A3), wherein the antibody-drug conjugate comprises an anti-TROP2 antibody-drug conjugate, and the cancer is as defined in any one of (35), (37) and (37a), or wherein the antibody-drug conjugate comprises an anti-HER2 antibody-drug conjugate, and the cancer is as defined in any one of (36), (37) and (37a).
(A5) The method according to any one of (A1) to (A4), wherein the anti-CD147 antibody is as defined in any one of (5), (5a), (6) to (11), (11a) to (11e), (12) to (16), and (33) to (34), wherein the anti-TROP2 antibody-drug conjugate is as defined in any one of (17) to (24) (31), (31a), and (32) to (34), and/or wherein the anti-HER2 antibody-drug conjugate is as defined in any one of (17), (18), (25) to (31), (31a), and (33) to (34).
(B1) The anti-CD147 antibody for use according to any one of (4) to (5), (5a), (6) to (11), (11a) to (11e), (12) to (31), (31a), (32) to (37), (37a) and (38) to (39), the antibody-drug conjugate for use according to any one of (4) to (5), (5a), (6) to (11), (11a) to (11e), (12) to (31), (31a), and (32) to (37), (37a) and (38) to (39), the combination for use according to any one of (4) to (5), (5a), (6) to (11), (11a) to (11e), (12) to (31), (31a), (32) to (37), (37a) and (38) to (39), or the therapeutic method according to any one of (A3) to (A5), wherein the anti-CD147 antibody in combination with the antibody-drug conjugate produce a synergistic effect in the treatment of cancer.

### [Advantageous Effects]

The present disclosure provides a combination therapy using an anti-CD147 antibody and an antibody-drug conjugate comprising a topoisomerase I inhibitor. The antibody which is conjugated to the topoisomerase I inhibitor is preferably an anti-TROP2 antibody or an anti-HER2 antibody. The present disclosure arises from the unexpected finding that the antitumor effects of the combination therapy are significantly enhanced as compared to the corresponding monotherapy with either the anti-CD147 antibody or antibody-drug conjugate. The present inventors have found that the anti-CD147 antibody and the antibody-drug conjugate produce a synergistic effect. The combination therapy may advantageously be used in the treatment of a wide range of cancers with improved clinical efficacy compared to existing treatments, and may be used to treat otherwise intractable cancers which are resistant to existing treatments.

### [Brief Description of Drawings]

Figure 1 is a diagram showing the amino acid sequence of variant 1 of human CD147 (SEQ ID NO: 1).
Figure 2 is a diagram showing the nucleotide sequence of variant 1 of human CD147 (SEQ ID NO: 2).
Figure 3 is a diagram showing the amino acid sequence of variant 2 of human CD147 (SEQ ID NO: 3).
Figure 4 is a diagram showing the nucleic acid sequence of variant 2 of human CD147 (SEQ ID NO: 4)
Figure 5 is a diagram showing the amino acid sequence of the heavy chain variable region of rat_CD147_#110(r#110) (SEQ ID NO: 5).
Figure 6 is a diagram showing the amino acid sequence of the light chain variable region of rat_CD147_#110(r#110) (SEQ ID NO: 6)
Figure 7 is a diagram showing the amino acid sequence of CDRH1 of rat_CD147_#110(r#110) (SEQ ID NO: 7)
Figure 8 is a diagram showing the amino acid sequence of CDRH2 of rat_CD147_#110(r#110) (SEQ ID NO: 8)
Figure 9 is a diagram showing the amino acid sequence of CDRH3 of rat _CD147_#110(r#110 (SEQ ID NO: 9)
Figure 10 is a diagram showing the amino acid sequence of CDRL1 of rat_CD147_#110(r#110) (SEQ ID NO: 10)
Figure 11 is a diagram showing the amino acid sequence of CDRL2 of rat_CD147#110(r#110) (SEQ ID NO: 11)
Figure 12 is a diagram showing the amino acid sequence of CDRL3 of rat_CD147_#110(r#110) (SEQ ID NO: 12)
Figure 13 is a diagram showing the amino acid sequence of #110H1hIgG4P (SEQ ID NO: 13)
Figure 14 is a diagram showing the amino acid sequence of #110H13hIgG4P (SEQ ID NO: 14)
Figure 15 is a diagram showing the amino acid sequence of #110L4h (SEQ ID NO: 15)
Figure 16 is a diagram showing the amino acid sequence of #110L2h (SEQ ID NO: 16)
Figure 17 is a diagram showing the amino acid sequence of #110L12h (SEQ ID NO: 17)
[Experimental]:
   Figure 18 shows the antitumor response to monotherapy with either #110H1L4hIgG4P (anti-CD147 antibody) or Dato-DXd (datopotamab deruxtecan) alone, or combination therapy with both #110H1L4hIgG4P and Dato-DXd, in a mouse EBC-1 xenograft model. Figure 18A is a line graph showing the tumor volume in response to the monotherapy and combination therapy.
   Figure 18B shows the level of expression of CD147 in tumor samples in response to the monotherapy and combination therapy. Figure 18C shows the level of expression of ABCC1 in tumor samples in response to the monotherapy and combination therapy. Figure 18D shows the level of DXd (deruxtecan)in tumor samples in response to the monotherapy and combination therapy.
   Figure 19 shows the antitumor response to monotherapy with either #110H1L4hIgG4P (anti-CD147 antibody) or Dato-DXd (datopotamab deruxtecan) alone, or combination therapy with both #110H1L4hIgG4P and Dato-DXd, in a mouse NCI-H358 xenograft model. Figure 19A is a line graph showing the tumor volume in response to the monotherapy and combination therapy. Figure 19B shows the level of DXd (deruxtecan)in tumor samples in response to the monotherapy and combination therapy.
   Figure 20 is a line graph showing the antitumor response to monotherapy with either #110H1L4hIgG4P (anti-CD147 antibody) or Dato-DXd (datopotamab deruxtecan) alone, or combination therapy with both #110H1L4hIgG4P and Dato-DXd, in a mouse HCC-1937 xenograft model. Figure 20 specifically shows the tumor volume in response to the monotherapy and combination therapy.
   Figure 21 is a line graph showing the antitumor response to monotherapy with either #110H1L4hIgG4P (anti-CD147 antibody) or Dato-DXd (datopotamab deruxtecan) alone, or combination therapy with both #110H1L4hIgG4P and Dato-DXd, in a mouse HCC-1806 xenograft model. Figure 21 specifically shows the tumor volume in response to the monotherapy and combination therapy.
   Figure 22 is a line graph showing the antitumor response to monotherapy with either #110H1L4hIgG4P (anti-CD147 antibody) or sacituzumab govitecan (anti-TROP2 antibody-drug conjugate) alone, or combination therapy with both #110H1L4hIgG4P and Dato-DXd, in a mouse HCC-1806 xenograft model. Figure 22 specifically shows the tumor volume in response to the monotherapy and combination therapy.
   Figure 23 is a line graph showing the antitumor response to combination therapy with both #110H1L4hIgG4P (anti-CD147 antibody) and a control anti-LPS antibody-DXd conjugate, combination therapy with both T-DXd (anti-HER2 antibody-drug conjugate (trastuzumab deruxtecan)) and a control anti-LPS antibody with hIgG4P constant region, and combination therapy with both #110H1L4hIgG4P and T-DXd in a mouse SNU-16 xenograft model. Figure 23 specifically shows the tumor volume in response to the combination therapies.

### [Definitions]

The terms "cancer (s)" and "tumor (s)" as used herein are used as synonyms, and unless otherwise indicated, each term is used to refer to solid cancer, non-solid cancer, or both.

The term "gene(s)" as used herein includes not only DNA, but also mRNA thereof, and cDNA and cRNA thereof.

The term "polynucleotide (s)" or "nucleotide (s)" are used herein synonymously with "nucleic acid" and examples thereof include DNA, RNA, probes, oligonucleotides, and primers.

The terms "polypeptide (s)" and "protein(s)" are used herein indistinguishably.

The term "CD147" as used herein is synonymous with CD147 protein", the term "TROP2" as used herein is synonymous with TROP2 protein", and the term "HER2" as used herein is synonymous with HER2 protein".

The term "antibody" as used herein encompasses various antibody structures including but not limited to a full-length antibody comprising two heavy chains and two light chains, a monoclonal antibody, a polyclonal antibody, a multispecific antibody (for example, a bispecific antibody) and an antigen-binding fragment of a full-length antibody.

The term "antigen-binding fragment" of an antibody as used herein means a partial fragment of a full-length antibody having an antigen-binding activity and which retains all or a part of the functions of a full-length antibody molecule. An antigen-binding fragment includes Fab, F(ab')2, Fv, scFv, a diabody, VHH and the like. The term also encompasses Fab' which is a monovalent fragment in a variable region of an antibody obtained by treating F(ab')2 under reducing conditions. However, the term is not limited to these molecules as long as the fragment has a binding affinity for an antigen. Further, these antigen-binding fragments include not only a fragment obtained by treating a full-length molecule of an antibody protein with an appropriate enzyme, but also a protein produced in an appropriate host cell using a genetically modified antibody gene.

The term "subject" as used herein in the context of prophylaxis or therapy is not limited but preferably refers to an animal, more preferably a mammal (including mice, rats, rabbits, dogs, monkeys, e.g., cynomolgus monkeys, and humans), and most preferably, a human.

The term "effector activity" as used herein refers to one or more of the following: antibody-dependent cellular cytotoxicity (ADCC), complement-dependent cytotoxicity (CDC), or antibody-dependent cellular phagocytosis (ADCP). The term "effector function" as used herein indicates the manifestation of one or more "effector activities."

The terms "combination", "pharmaceutical combination" and "combination therapy" as used herein, refer to a method or associated products where multiple active ingredients (for example, an anti-CD147 antibody and antibody-drug conjugate etc.) are contained or encapsulated in separate formulations and are administered simultaneously or sequentially in any order, or are contained or encapsulated in the same formulation and administered (simultaneously). Similarly, in this specification, the terms "used in combination with" and "administered in combination with", refer to a method or associated product where multiple active ingredients are contained or encapsulated in separate formulations and are administered simultaneously or sequentially in any order, or are contained or encapsulated in the same formulation and administered (simultaneously).

The term "pharmaceutical composition" as used herein refers to a formulation or preparation containing one or more active ingredients. In the case of a combination therapy, the pharmaceutical composition may comprise two or more active ingredients (or all the active ingredients) for simultaneous administration of the active ingredients. The pharmaceutical composition permits biological activity of the active ingredients and typically does not contain additional components which are unacceptably toxic to a subject receiving the composition. Such a composition may be sterile.

By "simultaneous administration" is meant that active ingredients are administered at the same time. By "sequential administration" is meant that active ingredients are administered one after the other, in any order, with a time interval between the individual administrations. The time interval can be, for example, less than 24 hours, preferably less than 6 hours, more preferably less than 2 hours.

The term "epitope" used herein refers to a partial peptide of, or a partial three-dimensional structure of, the target antigen (e.g. CD147), to which a specific antibody binds. The above epitope that is a partial peptide of the target antigen can be determined by a method well known by persons skilled in the art, such as an immunoassay method. First, various partial structures of antigens are prepared. Upon preparation of the partial structures, known oligonucleotide synthesis techniques can be employed. For example, a series of polypeptides are prepared by gene recombination techniques known by persons skilled in the art in such a manner that lengths of polypeptides from the C-terminus or the N-terminus of the target antigen are sequentially shortened, the reactivity of the antibody to these polypeptides is examined, recognition sites are roughly determined, peptides that are even shorter than the polypeptides are synthesized, and then the reactivity to these peptides is examined, so that an epitope can be determined. Further, when the epitope of an antibody, which binds to a membrane protein consisting of plurality of extracellular domains, is a three-dimensional structure consisting of a plurality of domains, the amino acid sequence of a specific extracellular domain can be modified to modify the three-dimensional structure, so that the specific domain to which the antibody binds can be determined. An epitope that is a partial three-dimensional structure of an antigen, to which a specific antibody binds, can also be determined by specifying the amino acid residues of an antigen adjacent to the above antibody by X-ray structure analysis.

If a second antibody binds to a partial peptide or a partial three-dimensional structure, to which a first antibody binds, it can be determined that the first antibody and the second antibody share a common epitope. Through confirmation of a second antibody cross-competing with a first antibody in terms of its binding to the antigen (specifically, the second antibody inhibits the binding of the first antibody to the antigen), it can be determined that the first antibody and the second antibody bind to the same epitope, even if the specific sequence or structure of the epitope has not determined. When a first antibody and a second antibody bind to the same epitope, and the first antibody has a special effect such as anti-tumor efficacy, the second antibody can be expected to have a similar activity.

In this specification, and unless otherwise stated, "binding" of an antibody to a given antigen or epitope includes specific binding such that the antibody binds to the target antigen or epitope contained therein preferentially as compared with unrelated target antigens or epitopes.

Each of the heavy chains and the light chains of an antibody molecule is known to have 3 complementarity determining regions (CDRs). The complementarity determining regions are also referred to as hypervariable regions (hypervariable domains), are located in the heavy chain and the light chain variable regions of an antibody, are sites where the variability of the primary structure is particularly high, and are located separately in the primary structure of the polypeptide chains of the heavy chains and the light chains. Regarding the term "complementarity determining regions of an antibody" as used herein, the heavy chain complementarity determining regions are denoted as CDRH1, CDRH2, and CDRH3 from the amino terminal side of the heavy chain amino acid sequence, and the light chain complementarity determining regions are denoted as CDRL1, CDRL2, and CDRL3 from the amino terminal side of the light chain amino acid sequence. These sites are adjacent to each other on the three-dimensional structure, and determine the specificity to an antigen to which the antibody binds.

The term "several" as used herein in the expressions "1 to several" and "1 or several" refers to 2 to 10, preferably, 10 or less, more preferably, 5 or 6 or less, and most preferably, 1, 2 or 3. The term may also refer to 1 to 2, 1 to 3, 1 to 4, or 1 to 5.

### [Description of Embodiments]

Hereinafter, preferred modes for carrying out aspects of the present disclosure are described. The embodiments described below are given merely for illustrating one example of a typical embodiment of the present disclosure and are not intended to limit the scope of the present disclosure.

### 1. CD147

In preferred embodiments, the anti-CD147 antibody used in embodiments of the present disclosure binds specifically to human CD147.

Three variants of human CD147 are known. Variant 1 is expressed specifically in the retina and is a single-pass transmembrane protein having three immunoglobulin-like domains (these domains may be referred to as D0, D1 and D2, respectively). Variant 2 is expressed in T cells and various other normal cells, and is a single-pass transmembrane protein having two immunoglobulin-like domains (D1, D2), the increased expression of which in various cancer tissues has been reported. Variant 3 is a single-pass transmembrane protein having one immunoglobulin-like domain.

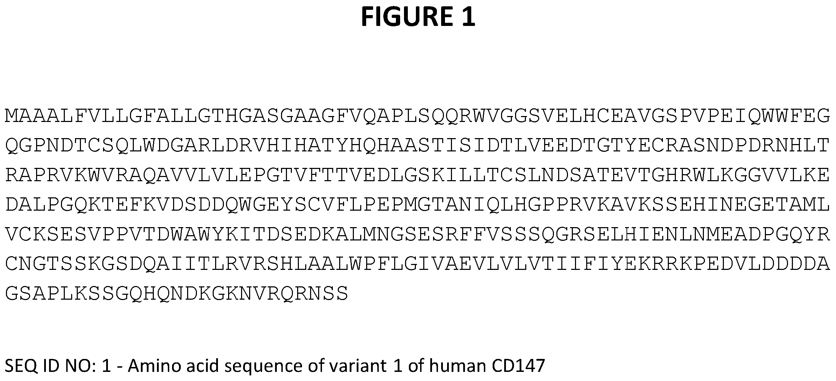

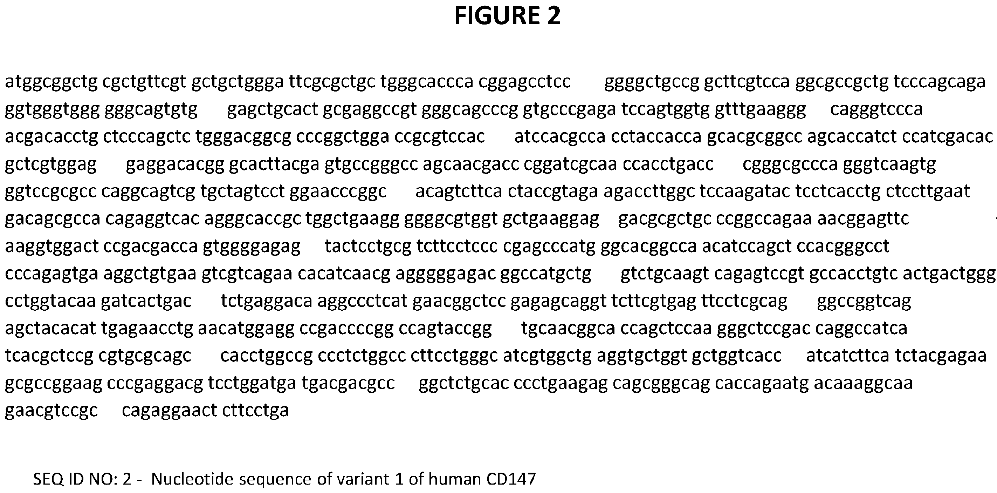

The amino acid sequence and the nucleotide sequence of variant 1 of human CD147 are available with reference to GenBank Accession Nos. NP_001719.2 and NM_001728.3. The amino acid sequence is disclosed herein as SEQ ID NO: 1 (Fig. 1), and the nucleotide sequence is disclosed herein as SEQ ID NO: 2 (Fig. 2). The three immunoglobulin-like domains of variant 1 range from amino acid residues 22 to 138 (D0), amino acid residues 140 to 218 (D1), and amino acid residues 223 to 323 (D2), respectively, in SEQ ID NO: 1, (Redzic, J., J. Mol. Biol., 2011, 68-82) (Grass et al., Biosol. Rep, 2016, 1-16). The transmembrane region of variant 1 ranges from amino acid residues 324 to 344 in SEQ ID NO: 1.

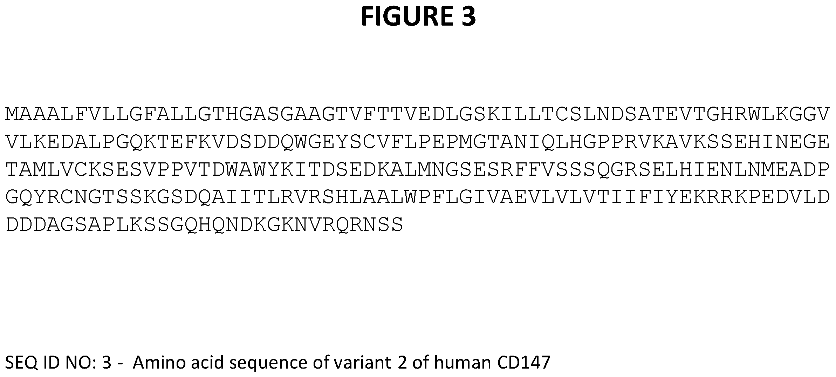

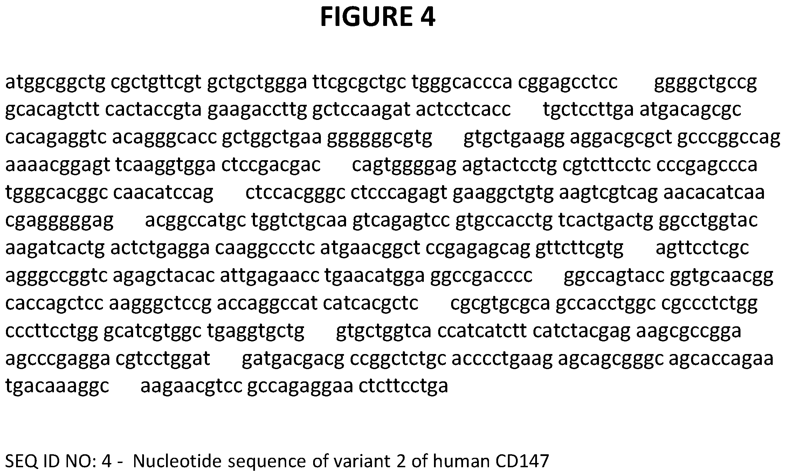

The amino acid sequence and the nucleotide sequence of variant 2 of human CD147 are available with reference to GenBank Accession Nos. NP_940991.1 and NM _198589.2. The amino acid sequence is disclosed herein as SEQ ID NO: 3 (Fig. 3), and the nucleotide sequence is disclosed herein as SEQ ID NO: 4 (Fig. 4). The two immunoglobulin-like domains (D1, D2) of variant 2 range from amino acid residues 24 to 102 (D1) and amino acid residues 107 to 207 (D2), respectively, in SEQ ID NO: 3. The transmembrane region of variant 2 ranges from amino acid residues 208 to 228 in SEQ ID NO: 3 (Grass et al., Biosol. Rep, 2016, 1-16).

The amino acid sequence and the nucleotide sequence of variant 3 of human CD147 are available with reference to GenBank Accession Nos. NP_940992.1 and NM_198590.2. The human CD147 gene is also available from a commercial source.

The anti-CD147 antibody used in embodiments of the present disclosure may bind to cynomolgous monkey CD147, preferably in addition to human CD147. Thus, the anti-CD147 antibody may exhibit cross-reactivity to CD147 derived from different species. The amino acid sequence and the nucleotide sequence of cynomolgus monkey CD147 (also referred to as monkey CD147 herein) are available with reference to GenBank Accession Nos. XP_005587354.1 and XM_005587297.1. In other embodiments, the anti-CD147 antibody binds to mouse CD147. The amino acid sequence and the nucleotide sequence of mouse CD147 are available with reference to GenBank Accession Nos. NP_001070652.1 and NM_001077184. 1. Mouse and monkey CD147 are also available from commercial sources.

CD147 that is to be used in connection with the present disclosure (for example, to determine the binding activity of an anti-CD147 antibody or to use as an immunogen to obtain anti-CD147 antibodies) can be obtained by synthesizing CD147 *in vitro,* or by using genetically engineered host cells to produce CD147. Specifically, CD147 cDNA is incorporated into a vector capable of expressing it, and then CD147 is synthesized in a solution containing the appropriate enzymes, substrates and energy sources required for transcription and translation. Alternatively, prokaryotic or eukaryotic host cells are transformed with the vector to express CD147, thus producing the protein.

The cDNA of CD147 can be obtained by a polymerase chain reaction (hereinafter, referred to as "PCR"), using a cDNA library expressing the cDNA of CD147 as a template, and primers for specific amplification of the cDNA of CD147 (Saiki, R. K., et al., Science, (1988)239, 487-49), for example.

Examples of the CD147 cDNA include a polynucleotide hybridizing under stringent conditions to a polynucleotide consisting of a nucleotide sequence complementary to a nucleotide sequence encoding human, monkey or mouse CD147, and, encoding a protein having biological activity equivalent to that of CD147. Further, examples of the cDNA of CD147 include a polynucleotide that is a splicing variant transcribed from the human, monkey or mouse CD147 gene locus or a polynucleotide hybridizing under stringent conditions thereto, and encoding a protein having biological activity equivalent to that of CD147.

A further example of CD147 includes an amino acid sequence derived from human, monkey or mouse CD147 in which the signal sequence has been removed, or an amino acid sequence in which one or several amino acids are substituted, deleted, or added in the amino acid sequence of human, monkey or mouse CD147. A further example of CD147 includes an amino acid sequence that corresponds to a splice variant transcribed from the human, monkey or mouse CD147 gene locus, optionally in which one or several amino acids are substituted, deleted, or added. These sequences desirably maintain the biological activity of CD147.

### 2. Anti-CD147 antibody

Preferably, the anti-CD147 antibody used in embodiments of the present disclosure binds to mammalian CD147 (for example, mouse, rat, cynomolgus monkey, or human CD147). More preferably, the anti-CD147 antibody binds to human CD147. Known antibodies may be used as long as they achieve the desired effects. Specific anti-CD147 antibodies which may be used are disclosed in Patent Literature 1 to 3; Non-Patent Literature 1 to 4; WO2019/022187; WO2015/160853; WO2023/174147; and CN110642947A.

The anti-CD147 antibody used in embodiments of the present disclosure can be obtained by procedures known in the art. For example, an anti-CD147 antibody can be obtained using a standard method which involves immunizing animals with an antigenic CD147 polypeptide and collecting and purifying antibodies produced *in vivo.* The origin of the CD147 antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen may be fused with myeloma cells according to a method known in the art (for example, Kohler and Milstein, Nature (1975) 256, p.495-497; Kennet, R. ed., Monoclonal Antibodies, p.365-367, Plenum Press, N.Y. (1980)). This method establishes hybridomas producing monoclonal antibodies. Specific examples of monoclonal antibodies thus obtained include, for instance, the rat _CD147_#84 (r#84) antibody and the rat_CD147_#110 (r#84) antibody. These antibodies and their methods of production are described in WO2019/022187 which is incorporated herein by reference.

The amino acid sequence of the heavy chain variable region of the rat _CD147_#84 antibody (also referred to as r#84) is shown in SEQ ID NO: 19, and the amino acid sequence of the light chain variable region is shown in SEQ ID NO: 20. Furthermore, the amino acid sequences of CDRH1, CDRH2, and CDRH3 are shown in SEQ ID NOs: 21, 22, and 23, respectively, and the amino acid sequences of CDRL1, CDRL2, and CDRL3 are shown in SEQ ID NOs: 24, 25, and 26, respectively.

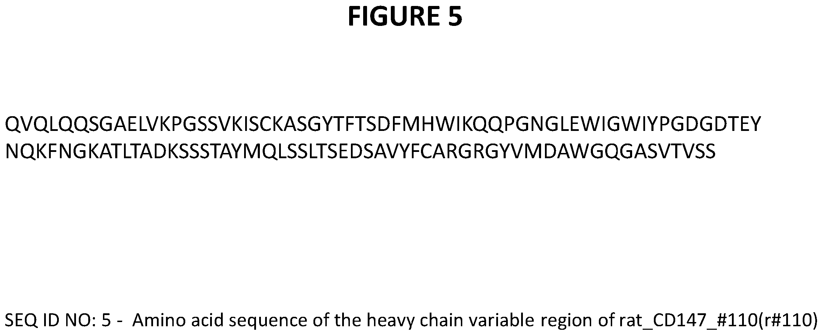

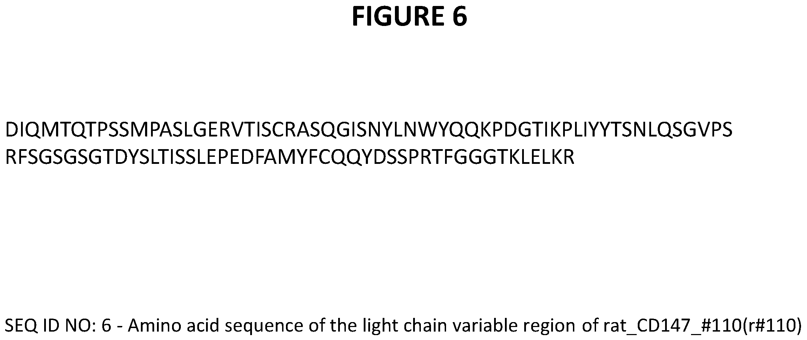

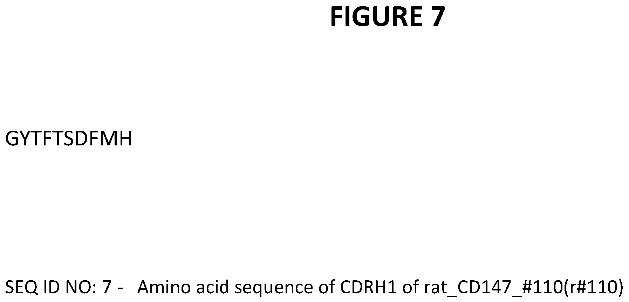

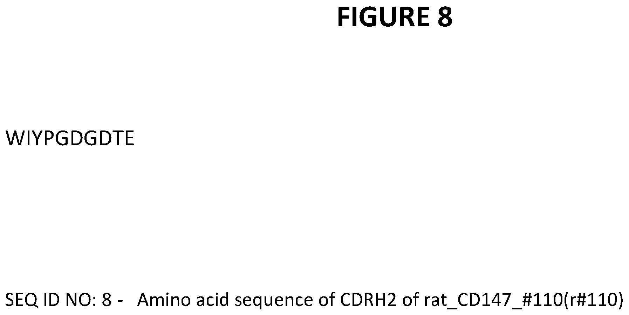

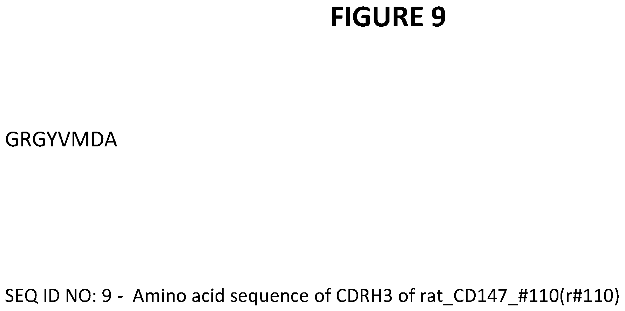

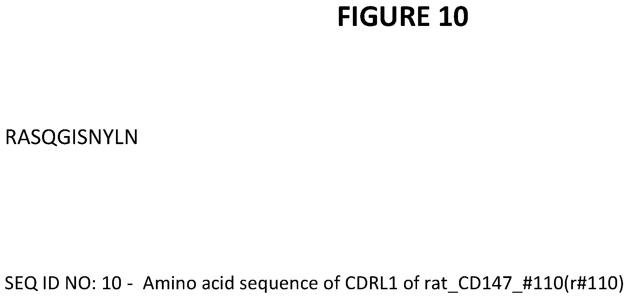

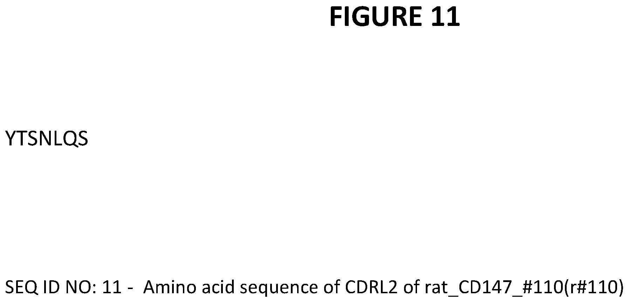

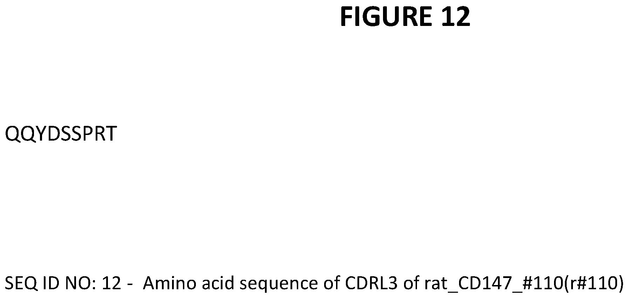

The amino acid sequence of the heavy chain variable region of rat_CD147_#110 antibody (also referred to as r#110) is shown in SEQ ID NO: 5 (Fig. 5), and the amino acid sequence of the light chain variable region is shown in SEQ ID NO: 6 (Fig. 6). Additionally, the amino acid sequences of CDRH1, CDRH2, and CDRH3 are presented in SEQ ID NOs: 7 (Fig. 7), 8 (Fig. 8), and 9 (Fig. 9), respectively, while the amino acid sequences of CDRL1, CDRL2, and CDRL3 are presented in SEQ ID NOs: 10 (Fig. 10), 11 (Fig. 11), and 12 (Fig. 12), respectively.

Anti-CD147 antibodies used in embodiments of the present disclosure include, in addition to the above-mentioned monoclonal antibodies against CD147, genetically engineered antibodies that are modified artificially to reduce xenogeneic antigenicity to humans. Examples include chimeric antibodies, humanized antibodies, and human antibodies. These antibodies can be produced using known methods.

Chimeric antibodies are antibodies in which the variable regions and constant region originate from different species. For example, the variable region of a mouse- or rat-derived antibody may be fused with a constant region of a human-derived antibody to create a chimeric antibody (Proc. Natl. Acad. Sci. USA, 81, 6851-6855 (1984)). Specific examples include chimeric versions of rat _CD147_#84 (r#84) and rat_CD147_#110 (r110) antibodies, as described in WO2019/022187 which is incorporated herein by reference.

A humanised antibody may be obtained by integrating only the complementarity determining regions (CDRs) of a heterologous antibody into a human-derived antibody (Nature (1986) 321, pp. 522-525), by grafting some of the amino acid residues of the framework regions and the amino acid residues of the CDR sequences of a heterologous antibody on to a human antibody using a CDR-grafting method (WO 90/07861), or by using a gene conversion mutagenesis strategy (U.S. Patent No. 5821337).

Human antibodies contain gene sequences exclusively derived from human chromosomes. Human anti-CD147 antibodies may be obtained by genetically engineering mice to produce human antibodies according to methods known in the art (e.g., Tomizuka, K. et al., Nature Genetics (1997) 16:133-143; Kuroiwa, Y. et al., Nucleic Acids Res. (1998) 26:3447-3448; Yoshida, H. et al., Animal Cell Technology: Basic and Applied Aspects vol. 10:69-73; Tomizuka, K. et al., Proc. Natl. Acad. Sci. USA (2000) 97:722-727).

Such human antibody-producing mice may be created as genetically engineered animals in which the endogenous immunoglobulin heavy chain and light chain loci are disrupted and, in their place, human immunoglobulin heavy chain and light chain loci are introduced via vectors such as human artificial chromosome (HAC) vectors or mouse artificial chromosome (MAC) vectors. These genetically engineered animals can be produced by combining knockout animals and transgenic animals, as well as by crossing these animals with one another.

Additionally, through genetic engineering, a recombinant human monoclonal antibody can also be obtained from culture supernatants by transfecting eukaryotic cells with cDNA, preferably vectors containing cDNA, encoding each of the heavy and light chains of the human antibody, and by culturing transformed cells capable of producing this antibody. Examples of host cells include mammalian cells such as CHO cells, lymphocytes, or myeloma cells.

Another known method for obtaining human antibodies of fragments of human antibodies is a phage display from human antibody libraries (e.g., Wormstone, I.M. et al., Investigative Ophthalmology & Visual Science (2002) 43(7):2301-2308; Carmen, S. et al., Briefings in Functional Genomics and Proteomics (2002) 1(2):189-203; Siriwardena, D. et al., Ophthalmology (2002) 109(3):427-431). In the phage display method, the variable regions of a human antibody are expressed on the surface of phages as single-chain antibodies (scFvs). Phages binding to the target antigen are selected, and the gene of the selected phage is analyzed to determine the DNA sequence encoding the scFv. Once the sequence is identified, an expression vector carrying the DNA sequence is introduced into a suitable host to produce human antibodies (e.g., WO92/01047; WO92/20791; WO93/06213; WO93/11236; WO93/19172; WO95/01438; WO95/15388; Annu. Rev. Immunol(1994) 12:433-455; Nature Biotechnology (2005) 23(9):1105-1116).

Once produced, the antigen-binding fragment may be separated and purified by methods well known to those skilled in the art. For instance, column chromatography, filter filtration, ultrafiltration, salting-out, dialysis, preparative polyacrylamide gel electrophoresis, isoelectric focusing electrophoresis, or appropriate combinations of these methods may be used to isolate and purify the antibody, but the separation and purification methods are not limited to these examples.

Although the aforementioned methods of antibody generation and isolation are described in relation to the anti-CD147 antibody used in embodiments of the present disclosure, they are also applicable to the anti-TROP2 and anti-HER2 antibodies of the antibody-binding conjugates described herein.

In one embodiment, the anti-CD147 antibody used in embodiments of the present disclosure is an anti-CD147 antibody described in the following (C1) and (C2):
(C1) An anti-CD147 antibody comprising a heavy chain variable region that includes CDRH1 described in SEQ ID NO: 7, CDRH2 described in SEQ ID NO: 8, and CDRH3 described in SEQ ID NO: 9, and a light chain variable region that includes CDRL1 described in SEQ ID NO: 10, CDRL2 described in SEQ ID NO: 11, and CDRL3 described in SEQ ID NO: 12; and
(C2) An anti-CD147 antibody comprising a heavy chain variable region that includes CDRH1 described in SEQ ID NO: 21, CDRH2 described in SEQ ID NO: 22, and CDRH3 described in SEQ ID NO: 23, and a light chain variable region that includes CDRL1 described in SEQ ID NO: 24, CDRL2 described in SEQ ID NO: 25, and CDRL3 described in SEQ ID NO: 26.

The antibody according to (C1) may compete with an anti-CD147 antibody according to (C1a) or bind to the epitope to which the anti-CD147 antibody according to (C1a) binds:
(C1a) an anti-CD147 antibody comprising a heavy chain variable region consisting of an amino acid sequence represented by SEQ ID NO: 5, and a light chain variable region consisting of an amino acid sequence represented by SEQ ID NO: 6.

The antibody according to (C2) may compete with an anti-CD147 antibody according to (C2a) or bind to the epitope to which the anti-CD147 antibody according to (C2a) binds:
(C2a) an anti-CD147 antibody comprising a heavy chain variable region consisting of an amino acid sequence represented by SEQ ID NO: 19, and a light chain variable region consisting of an amino acid sequence represented by SEQ ID NO: 20.

Humanized antibodies derived from rat _CD147_#84 (r#84) antibody and rat_CD147_#110 antibody may be used in embodiments of the present disclosure. The humanized antibodies may retain each of the six CDR sequences and possess binding activity to CD147. Alternatively, CDR-modified humanized antibodies in which one to three amino acid residues in any of the CDRs are substituted with other amino acid residues, provided they maintain binding activity to CD147, are envisaged. These modified antibodies also fall under the scope of anti-CD147 antibodies in this disclosure.

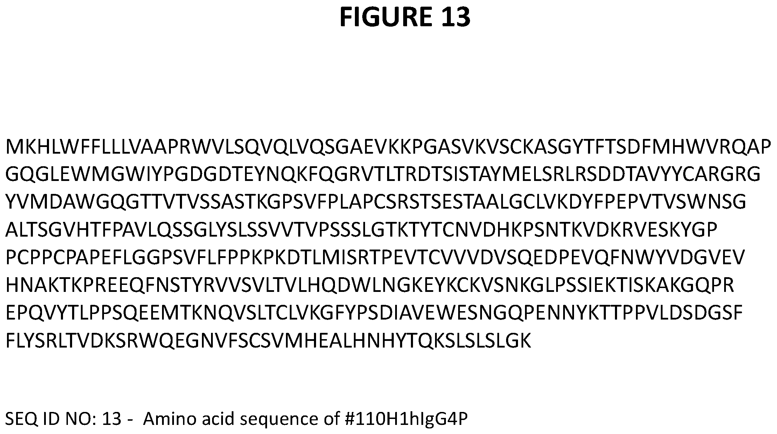

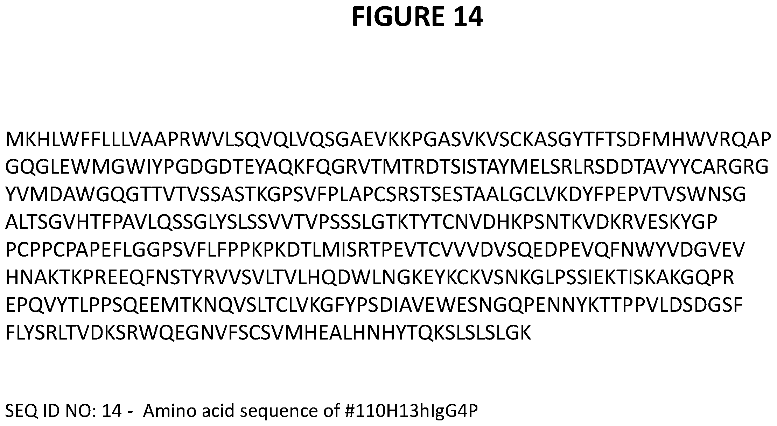

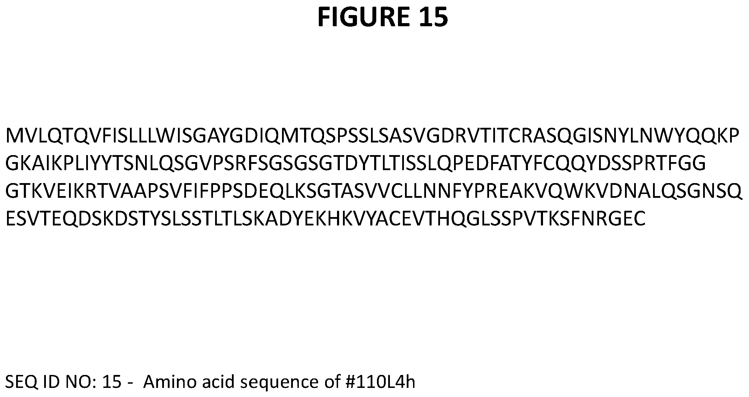

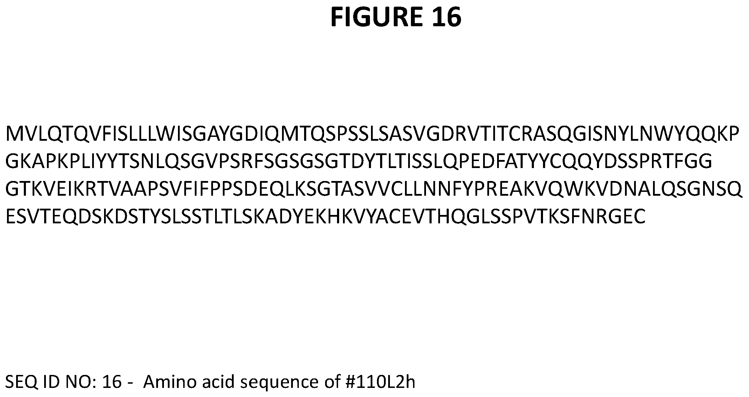

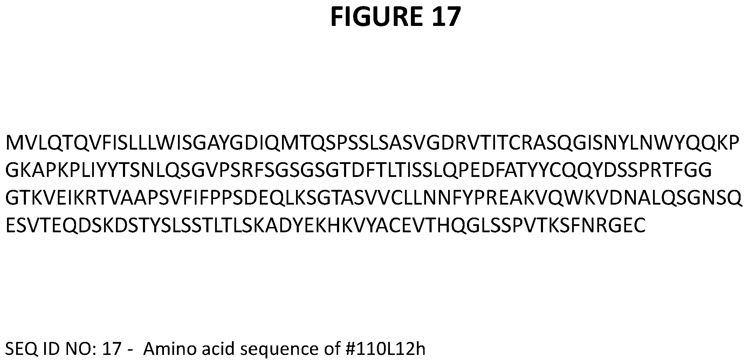

Humanized antibodies derived from rat_CD147_#110 antibody are preferred. These include, but are not limited to, antibodies described in Example 6 of WO2019/022187 which is incorporated herein by reference. The following humanised anti-CD147 antibodies may be used in embodiments of the disclosure:
(D1) An anti-human CD147 antibody containing the heavy chain variable region of #110H1hIgG4P (composed of amino acid residues 20-136 of the amino acid sequence shown in SEQ ID NO: 13 (Fig. 13)) and the light chain variable region of #110L4h (composed of amino acid residues 21-128 of the amino acid sequence shown in SEQ ID NO: 15(Fig. 15));
(D1-1) A humanized antibody #110H1L4hIgG4P, which is an anti-human CD147 antibody containing the heavy chain of #110H1hIgG4P (composed of amino acid residues 20-463 of the amino acid sequence shown in SEQ ID NO: 13) and the light chain of #110L4h (composed of amino acid residues 21-234 of the amino acid sequence shown in SEQ ID NO: 15);
(D2) An anti-human CD147 antibody containing the heavy chain variable region of #110H13hIgG4P (composed of amino acid residues 20-136 of the amino acid sequence shown in SEQ ID NO: 14 (Fig. 14)) and the light chain variable region of #110L2h (composed of amino acid residues 21-128 of the amino acid sequence shown in SEQ ID NO: 16 (Fig. 16));
(D2-1) A humanized antibody #110H13L2hIgG4P, which is an anti-human CD147 antibody containing the heavy chain of #110H13hIgG4P (composed of amino acid residues 20-463 of the amino acid sequence shown in SEQ ID NO: 14 (Fig. 14)) and the light chain of #110L2h (composed of amino acid residues 21-234 of the amino acid sequence shown in SEQ ID NO: 16);
(D3) An anti-human CD147 antibody containing the heavy chain variable region of #110H13hIgG4P (composed of amino acid residues 20-136 of the amino acid sequence shown in SEQ ID NO: 14(Fig. 14)) and the light chain variable region of #110L12h (composed of amino acid residues 21-128 of the amino acid sequence shown in SEQ ID NO: 17 (Fig. 17));
(D3-1) A humanized antibody #110H13L12hIgG4P, which is an anti-human CD147 antibody containing the heavy chain of #110H13hIgG4P (composed of amino acid residues 20-463 of the amino acid sequence shown in SEQ ID NO: 14) and the light chain of #110L12h (composed of amino acid residues 21-234 of the amino acid sequence shown in SEQ ID NO: 17).

In yet another embodiment, humanized antibodies derived from rat_CD147_#110 include the following components (D4) and (D5) :
(D4) a heavy chain variable region selected from the group consisting of the following (D4-1) to (D4-4):
   (D4-1) a heavy chain variable region consisting of amino acid residues at positions 20 to 136 in an amino acid sequence represented by SEQ ID NO: 13;
   (D4-2) a heavy chain variable region consisting of amino acid residues at positions 20 to 136 in an amino acid sequence represented by SEQ ID NO: 14;
   (D4-3) an amino acid sequence having at least 80%, 85%, 90%, 95%, or 98% sequence identity with the sequence of a framework region outside each CDR sequence in the sequence of (D4-1) or (D4-2); and
   (D4-4) an amino acid sequence in which one or several amino acids are deleted, substituted, or added in the sequence of a framework region outside each CDR sequence in the sequence according to any one of (D4-1) to (D4-3); and
(D5) a light chain variable region selected from the group consisting of the following (b1) to (b5):
   (D5-1) a light chain variable region consisting of amino acid residues at positions 21 to 128 in an amino acid sequence represented by SEQ ID NO: 15;
   (D5-2) a light chain variable region consisting of amino acid residues at positions 21 to 128 in an amino acid sequence represented by SEQ ID NO: 16;
   (D5-3) a light chain variable region consisting of amino acid residues at positions 21 to 128 in an amino acid sequence represented by SEQ ID NO: 17;
   (D5-4) an amino acid sequence having at least 80%, 85%, 90%, 95%, or 98% sequence identity with the sequence of a framework region outside each CDR sequence in the sequence according to any one of (D5-1) to (D5-3); and
   (D5-5) an amino acid sequence in which one or several amino acids are deleted, substituted, or added in the sequence of a framework region outside each CDR sequence in the sequence according to any one of (D5-1) to (D5-4).

Humanized antibodies derived from the rat _CD147_#84 antibody are, without limitation, exemplified in Example 6 of WO2019/022187 which is incorporated herein by reference. Such antibodies as used in present disclosure may include:
(D6) An anti-human CD147 antibody comprising a heavy chain variable region consisting of amino acid residues 20-140 of SEQ ID NO: 27 (#84H1hIgG2), and a light chain variable region consisting of amino acid residues 21-128 of SEQ ID NO: 29 (#84L2h);
(D6-1) A humanized antibody (#84H1L2hIgG2) comprising a heavy chain consisting of amino acid residues 20-466 of SEQ ID NO: 27 (#84H1hIgG2), and a light chain consisting of amino acid residues 21-234 of SEQ ID NO: 29 (#84L2h);
(D7) An anti-human CD147 antibody comprising a heavy chain variable region consisting of amino acid residues 20-140 of SEQ ID NO: 28 (#84H1hIgG4P), and a light chain variable region consisting of amino acid residues 21-128 of SEQ ID NO:29 (#84L2h);
(D7-1) A humanized antibody (#84H1L2hIgG4P) comprising a heavy chain consisting of amino acid residues 20-467 of SEQ ID NO: 28 (#84H1hIgG4P), and a light chain consisting of amino acid residues 21-234 of SEQ ID NO: 29 (#84L2h).

In another embodiment, humanized antibodies derived from rat _CD147_#84 may include the following components (D8) and (D9):
(D8) a heavy chain variable region selected from the group consisting of the following (D8-1) to (D8-4):
   (D8-1) a heavy chain variable region consisting of amino acid residues at positions 20 to 140 in an amino acid sequence represented by SEQ ID NO: 27;
   (D8-2) a heavy chain variable region consisting of amino acid residues at positions 20 to 140 in an amino acid sequence represented by SEQ ID NO: 28;
   (D8-3) an amino acid sequence having at least 80%, 85%, 90%, 95%, or 98% sequence identity with the sequence of a framework region outside each CDR sequence in the sequence of (D8-1) or (D8-2); and
   (D8-4) an amino acid sequence in which one or several amino acids are deleted, substituted, or added in the sequence of a framework region outside each CDR sequence in the sequence according to any one of (D8-1) to (D8-3); and
(D9) a light chain variable region selected from the group consisting of the following (9-1) to (9-3):
   (D9-1) a light chain variable region consisting of amino acid residues at positions 21 to 128 in an amino acid sequence represented by SEQ ID NO: 29;
   (D9-2) an amino acid sequence having at least 80%, 85%, 90%, 95%, or 98% sequence identity with the sequence of a framework region outside each CDR sequence in the sequence of (D9-1); and
   (D9-3) an amino acid sequence in which one or several amino acids are deleted, substituted, or added in the sequence of a framework region outside each CDR sequence in the sequence of (D9-1) or (D9-2).

Anti-CD147 antibodies binding to the same epitope as the anti-CD147 antibodies disclosed herein may also be used. For example, Example 17 of WO2019/022187 describes X-ray crystallographic analysis of the Fab' fragment of humanized #110H1L4hIgG4P (obtained via pepsin digestion) in complex with human CD147 variant 2 (hCD147v2). The analysis confirmed binding to a specific region of domain 2.
Thus, in one embodiment of the disclosure, the anti-CD147 antibody binds to domain 2 of human CD147. Furthermore, the antibody may bind to an epitope comprising residues Arg106 to Gly165 within the amino acid sequence of human CD147 variant 2, as described in SEQ ID NO: 3. Additionally, the antibody may bind to an epitope comprising specific residues such as Arg106, Lys108, Ala109, Val110, Lys127, Ser128, Glu129, Ser130, Val131, Pro132, Pro133, Val134, Gln164, and Gly165 within variant 2. Preferably, the binding is specific such that the antibody binds to the target epitope preferentially as compared with an unrelated epitope.

The anti-CD147 antibody as described herein may be modified to obtain a modified variant. The modified variant may be obtained by subjecting the antibody to chemical or biological modification. Examples of chemical modification include linking a chemical moiety (for example, polyethylene glycol) to an amino acid skeleton or linking a chemical moiety to an N-linked or O-linked carbohydrate chain, etc. Examples of a biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N-terminus by virtue of being expressed in a prokaryotic host cell. Further, an antibody may be labelled to enable the detection or isolation of the antibody. For example, an enzyme-labeled antibody, a fluorescence-labeled antibody, and an affinity-labeled antibody are also included in the meaning of the modified variant. Such a modified variant of the antibody for use according to the present disclosure may be useful for improving the stability and blood retention of the antibody, reducing the antigenicity thereof, and/or detecting or isolating an antibody or an antigen.

Further, by regulating the modification of a glycan which is linked to the antibody used the present disclosure (glycosylation, defucosylation, etc.), it is possible to enhance antibody-dependent cellular cytotoxic activity. Methods for regulating the modification of a glycan of antibodies are known from WO 99/54342, WO 00/61739, WO 02/31140, WO 2007/133855, and WO 2013/120066 which are incorporated herein by reference. However, the other standard methods may be used.

Additionally, it is known that a lysine residue at the carboxyl terminus of the heavy chain of an antibody produced in a cultured mammalian cell is deleted (Tsubaki et al., Int. J. Biol. Macromol, 139-147, 2013). It has also been reported that two amino acid residues (glycine and lysine) at the carboxyl terminus of the heavy chain of an antibody produced in a cultured mammalian cell are deleted and a proline residue newly-located at the carboxyl terminus is amidated (Analytical Biochemistry, 360: 75-83 (2007)). However, deletion and modification of these heavy chain sequences do not affect the antibody's ability to bind to an antigen and effector functions (e.g., activation of complement and antibody-dependent cellular cytotoxicity). Therefore, embodiments of the present disclosure include an antibody subjected to modification. For example, an antibody in which 1 or 2 amino acids are deleted at the heavy chain carboxyl terminus, and an antibody subjected to amidation (for example, a heavy chain of the antibody in which a proline residue at the carboxyl terminal site is amidated) are included. As long as the antigen-binding capability and the function of activating signal-related molecules located downstream of CD147 are maintained, a heavy chain carboxyl terminal-deficient antibody is not limited to the above types.

In some embodiments, the anti-CD147 antibody comprises two heavy chains. The two heavy chains may consist of two full-length heavy chains, two truncated/modified variants, or a combination of the full-length heavy chain and a truncated/modified variant. The ratio of carboxyl terminal-deficient heavy chains can be affected by the type and culture conditions of the cultured mammalian cells producing the antibody. In a preferred embodiment, the major component of the anti-CD147 antibody comprises two heavy chains, each having a single amino acid deletion at the carboxyl terminus.

Although the above modifications to the antibody above are described in relation to the anti-CD147 antibody, the modifications are applicable to each of the antibodies disclosed herein, including the anti-TROP2 antibody and anti-HER2 antibody moieties of the antibody-drug conjugates described herein.

The isotype of the anti-CD147 antibody used in embodiments of the present disclosure is not limited and may include, for example, IgG (IgG1, IgG2, IgG3, IgG4), IgM, IgA (IgA1, IgA2), IgD, or IgE, amongst others. Preferably, the isotype is IgG or IgM, more preferably, IgG, and even more preferably, human IgG (e.g., human IgG1, IgG2, IgG3, or IgG4). The amino acid sequence of IgG can also be modified as necessary.

When IgG1 is used as an isotype of the antibody of the present disclosure, the IgG1 antibody may have one or more mutation. Specifically, effector functions can be adjusted by substitution of some of the amino acid residues of the constant regions (see WO88/007089, WO94/28027, WO94/29351 which are incorporated herein by reference). Examples of a variant of IgG1 with attenuated effector functions include IgG1 LALA (IgG1-L234A, L235A), and IgG1 LAGA (IgG1-L235A, G237A).

When IgG4 is used as an isotype, the IgG4 antibody may have one or more mutations. IgG4 variants may include, for example, the substitution of phenylalanine with alanine at position 234 (F234A) and the substitution of leucine with alanine at position 235 (L235A) as indicated by the EU index (Proc Natl Acad Sci U.S.A. 1969, 63(1), 78-85; Kabat et al., Sequences of Proteins of Immunological Interest, 1991, Fifth Edition; Parekh et al., mAbs, 310-318, 2012). These modifications are known collectively as the FALA mutations.

The disulphide bonds which form between antibody heavy chains in IgG4 are unstable. Mutations may be introduced to to enhance stability by promoting disulfide bond formation. For instance, substituting serine at position 228 with proline (S228P) as indicated by the EU index is known to stabilize the antibody (ANGAL et al., Molecular Immunology, 105-108, 1993). This mutation is referred to as the Pro mutation.

The constant region of an anti-CD147 antibody in the present disclosure may simultaneously include both the FALA mutation and the Pro mutation (see Vafa et al., Methods, 65, 114-126, 2014). IgG4 heavy chains with the FALA mutation are referred to as "IgG4FALA" heavy chains, those with the Pro mutation as "IgG4P" heavy chains, and those with both mutations as "IgG4PFALA" heavy chains.

The constant region of the antibody heavy chain consists of the CH1, hinge, CH2, and CH3 regions. CH1 is defined as EU index 118 to 215, the hinge as EU index 216 to 230, CH2 as EU index 231 to 340, and CH3 as EU index 341 to 446. In one embodiment, the isotype of the anti-CD147 antibody used in embodiments of the present disclosure includes IgG1, IgG2, IgG4, IgG4P, or IgG4PFALA, preferably IgG2, IgG4P, or IgG4PFALA, and more preferably, IgG2 or IgG4P.

In one embodiment, the anti-CD147 antibody used in embodiments of the present disclosure may include, as the heavy chain, the constant region or a fragment thereof of a human IgG1 heavy chain, human IgG2 heavy chain, human IgG3 heavy chain, or human IgG4 heavy chain. Similarly, it may include, as the light chain, the constant region or a fragment thereof of a human κ chain or λ chain.

In embodiments of the present disclosure, an antigen-binding fragment of a CD147 antibody is used. As described above, the term "antigen-binding fragment" refers to a partial fragment of an antibody that has antigen-binding activity, without being limited, as long as it retains all or a part of the functions of a full-length antibody molecule. In some embodiments, the antigen-binding fragment of the anti-CD147 antibody includes Fab, F(ab')2, Fab', Fv, VHH, diabody, or a linear antibody such as scFv or single-chain immunoglobulin. Alternatively, the antigen-binding fragment may be a peptide that mimics fully or partially and/or functionally the antigen-binding portion of the anti-CD147 antibody. Such peptides are not particularly limited and may be, for instance, linear or cyclic peptides. In the case where the epitope on CD147 is a linear epitope, the mimicking peptide may be one that can bind to all or part of this linear epitope. In the case where the epitope is a non-linear epitope, the mimicking peptide may bind to all or part of the partial three-dimensional structure formed by the non-linear epitope. Furthermore, such mimicking peptides may bind to the part of the linear or non-linear epitope that exhibits the main activity. If the portion with the main activity is linear, the mimicking peptide may be a linear peptide; if it is non-linear, the mimicking peptide may be a cyclic peptide.

Antigen-binding fragments include not only those derived by processing full-length antibody molecules with appropriate enzymes but also proteins produced in suitable host cells using genetically engineered modified antibody genes. In some embodiments, the anti-CD147 antibody is a multi-specific antigen-binding molecule formed from two or more of these antigen-binding fragments.

The anti-CD147 antibody used in embodiments of the present disclosure may also be an antibody possessing a single heavy chain variable domain (VH) without a light chain. These antibodies, known as VHH, single-domain antibodies (sdAb), or nanobodies, have been observed in camelids such as camels and llamas, and retain antigen-binding ability. Such antibodies are envisaged in the present disclosure as one type of antigen-binding fragment.

The antibody of the present disclosure may be a single chain antibody (scFv). A single chain antibody is obtained by connecting an antibody heavy chain variable region (VH) and a light chain variable region (VL) with a polypeptide linker (Pluckthun, The Pharmacology of Monoclonal Antibodies, 113; Rosenberg and Moore, eds., Springer Verlag, New York, pp. 269-315, 1994; Nature Biotechnology [2005] 23:1126-1136). Additionally, bispecific scFvs (BiscFvs), created by linking two scFvs via a peptide linker, can function as bispecific antibodies. In a scFv, the VH and VL regions are connected via a non-conjugating linker, preferably a polypeptide linker (Huston, J.S. et al., Proc. Natl. Acad. Sci. USA [1988] 85:5879-5883). The VH and VL regions in the scFv may originate from the same antibody or from different antibodies. A single-chain peptide composed of, for example, 12 to 19 amino acid residues may be used as a polypeptide linker for connecting variable regions.

Methods for obtaining single-chain immunoglobulins-formed by linking the full-length heavy chain and light chain sequences with suitable linkers-are well-known (Lee, H.-S., et al., Molecular Immunology [1999] 36:61-71; Shirrmann, T. et al., mAbs [2010] 2(1):1-4). Single-chain immunoglobulins may dimerize to mimic the structure and activity of native tetrameric antibodies.

The DNA encoding a scFv may be generated by amplifying DNA fragments encoding the VH and VL regions by PCR using primers that define the ends of the heavy and light chain sequences. Subsequently, DNA encoding the peptide linker, with primers designed to connect the VH and VL regions in an appropriate manner, can be combined and further amplified to create the full-length scFv-encoding sequence.

Once DNA encoding scFv is available, an expression vector containing the DNA and a host transformed with the expression vector may be prepared using standard methods. Subsequent expression of the scFv gene in the host cell allows the scFv fragment to be obtained.

In another embodiment, the anti-CD147 antibody used in embodiments of the present disclosure may be a multispecific antibody with specificity for CD147 and at least one additional antigen. Examples include bispecific or trispecific antibodies. Alternatively, the anti-CD147 antibody may be a multiparatopic antibody, such as a biparatopic antibody, which is engineered to bind to two or more distinct epitopes on CD147. Such multispecific or multiparatopic antibodies may be full-length antibodies or fragments thereof, such as bispecific antigen-binding molecules in the form of F(ab')2 fragments. Bispecific or biparatopic antibodies can be produced by combining the heavy and light chains (HL pairs) of two different antibodies or by fusing hybridomas that produce distinct monoclonal antibodies (Milstein et al., Nature [1983] 305:537-539).

In another embodiment, the anti-CD147 antibody may be a polyclonal antibody, which is a mixture of multiple types of anti-CD147 antibodies with different amino acid sequences. One example of a polyclonal antibody is a mixture of antibodies having different CDRs. Such polyclonal antibodies may be obtained by culturing a mixture of cells producing different antibodies and purifying the antibodies from the culture in accordance with standard methods (see WO2004/061104).

In another embodiment, the anti-CD147 antibody of the present disclosure may be multimerized to enhance its affinity for antigens. Multimerized antibodies may consist of a single antibody type or multiple antibodies recognizing distinct epitopes on the same antigen. Methods for multimerizing antibodies include the fusion of IgG CH3 domains with two scFvs, conjugation with streptavidin, and the incorporation of helix-turn-helix motifs, amongst others.

In another embodiment, the anti-CD147 antibody of the present disclosure may exhibit 80-99% sequence identity (e.g., at least 80%, 85%, 90%, 95%, or 98%) with the heavy and/or light chain sequences of the anti-CD147 antibodies disclosed herein. By providing sequences that exhibit high sequence identity with the aforementioned heavy and/or light chain sequences, antibodies possessing equivalent or even improved antigen-binding capabilities and CD147-mediated signal transduction activities such as MAPK activation or the activation of downstream signaling molecules of MAPK, may be selected.

The threshold of sequence identity is not limited but it is preferably at least 80%, and more preferably at least 85%, 90%, 95%, or 98%. Furthermore, antibodies may also include sequences where one or more amino acid residues (e.g., up to 10 residues, preferably 5-6, more preferably 2-3, and most preferably a single residue) have been substituted, deleted, or added, provided they retain equivalent functional activities.

Analogous sequence variation is possible with respect to the anti-TROP2 and anti-HER2 antibody sequences described herein.

Sequence identity or homology between two amino acid sequences can be calculated using the BLAST algorithm version 2.2.2 (Altschul, Stephen F., Thomas L. Madden, Alejandro A. Schaffer, Jinghui Zhang, Zheng Zhang, Webb Miller, and David J. Lipman [1997], "Gapped BLAST and PSI-BLAST: a new generation of protein database search programs," Nucleic Acids Res. 25:3389-3402) with default settings. The BLAST algorithm can also be accessed online at [www.ncbi.nlm.nih.gov/blast] (http://www.ncbi.nlm.nih.gov/bl ast). The BLAST algorithm calculates two percentage values: Identity (or Identities) and Positivity (or Positivities). Identity reflects the percentage of exact matches between amino acid residues in the two sequences (sequence identity), whilst Positivity is based on residues with similar chemical structures (sequence homology).

### 3. Antibody-drug conjugate

In some embodiments of the present disclosure, the anti-CD147 antibody described above is administered in combination with an antibody-drug conjugate as a combination therapy. The drug moiety of the antibody-drug conjugate comprises a topoisomerase I inhibitor. The antibody may be selected depending on the desired target cell for drug delivery.

In preferred embodiments, the antibody in the antibody-drug conjugate is an anti-TROP2 antibody. The term "anti-TROP2 antibody" refers to an antibody which binds specifically to TROP2 (TACSTD2: Tumor-associated calcium signal transducer 2; EGP-1), and preferably has an activity of internalization in TROP2-expressing cells by binding to TROP2.

In other embodiments, the antibody in the antibody-drug conjugate is an anti-HER2 antibody. The term "anti-HER2 antibody" refers to an antibody which binds specifically to HER2 (neu, ErbB-2), and preferably has an activity of internalization in HER2-expressing cells by binding to HER2.

Preferably, the TROP2 or HER2 to which the antibody-drug conjugate binds is derived from a human. The amino acid sequence of human TROP2 is available with reference to the National Centre for Biotechnology Information (NCBI) accession no. NP_002344 (Entrez Gene ID 4070) . The nucleotide (mRNA) sequence of human TROP2 is available with reference to NCBI accession no. NM_002353. The amino acid sequence of human HER2 is available with reference to NCBI accession no. NP_004439 (Entrez Gene ID 2064). The nucleotide (mRNA) sequence of human HER2 is available with reference to GenBank accession no. M11730. However, alternatively or additionally, the antibody-drug conjugate may bind to TROP2 or HER2 derived from other mammals (e.g. mouse, rat, and monkey) .Sequences of TROP2 or HER2 derived from non-human animals are also publicly available.

In some embodiments, the antibody-drug conjugate comprises a linker and a drug, the antibody being connected to the drug via the linker, and the linker and drug forming a drug-linker. (That is to say, the partial structure consisting of a linker and a drug in an antibody-drug conjugate is referred to as a "drug-linker"). In some embodiments, the drug-linker is represented by the following formula: 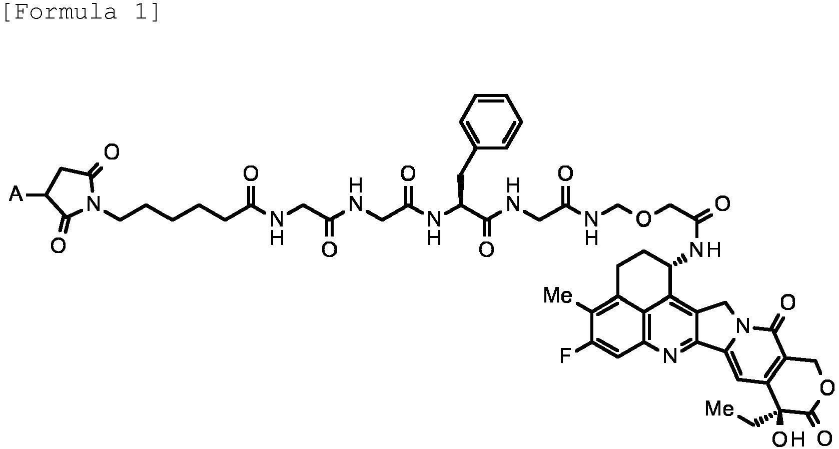 wherein A represents a connecting position to the antibody, and wherein the drug-linker is conjugated to the antibody, preferably an anti-TROP2 antibody or an anti-HER2 antibody, via a thioether bond.

The drug-linker is preferably connected to a thiol group (that is to say, the sulphur atom of a cysteine residue) formed at a disulphide bond site, such as an interchain disulfide bond site (two sites between heavy chains, and two sites between a heavy chain and a light chain) in the antibody.

The drug-linker of antibody-drug conjugate used in embodiments of the present disclosure (and as exemplified by Formula 1) includes exatecan as a drug component (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H,12H-benzo[de]pyrano[3',4':6,7]indolizino[1,2-b]quinolin-10,13(9H,15H)-dione)). Exatecan is a topoisomerase I inhibitor. More specifically, exatecan is a camptothecin derivative having an antitumor effect, represented by the following formula:

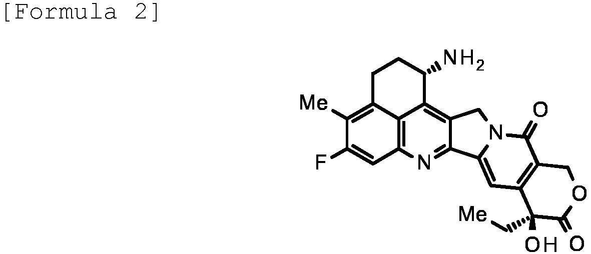

Accordingly, in some embodiments of the present disclosure, exatecan according to Formula 2 is conjugated to an anti-TROP2 antibody or an anti-HER2 antibody as described herein via the linker with the nitrogen atom of the amino group at position 1 as a connecting position. As described above, the drug-linker is preferably connected to a thiol group formed at a disulfide bond site, for example, an interchain disulphide bond site. In some embodiments, the linker comprises a tetrapeptide sequence of -Gly-Gly-Phe-Gly - (SEQ ID NO: 53), as depicted in Formula 1 above. However, other linker sequences are also envisaged.

In embodiments of the present disclosure, a topoisomerase I inhibitor represented by the following formula is released from the antibody-drug conjugate to express an antitumor activity:

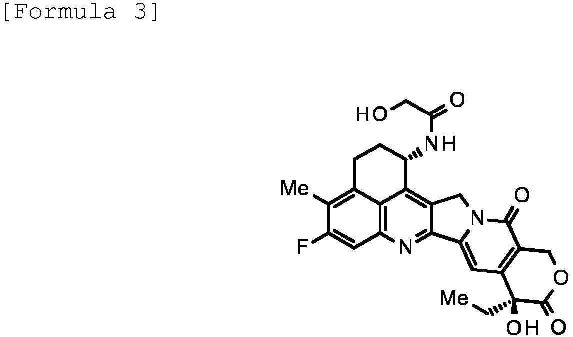

The release of the compound according to Formula 3 may occur after migration of the antibody-drug conjugate into a target cancer cell, and preferably after cleavage of the antibody-drug conjugate at the linker portion. In these embodiments, the compound according to Formula 3 is inferred to be the original source of the antitumor activity of the antibody-drug conjugate.

The antibody-drug conjugate used in embodiments of the present disclosure can be also represented by the following formula:

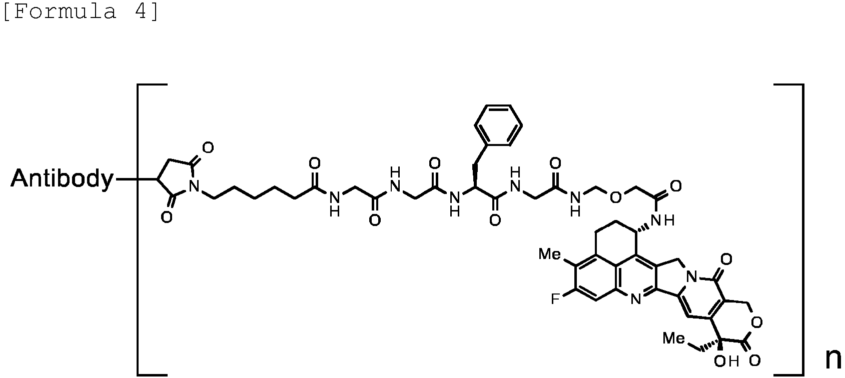

In this structure, the drug-linker is conjugated to an antibody via a thioether bond. The meaning of n is the average number of conjugated drug molecules (DAR; Drug-to-Antibody Ratio), and indicates the average number of units of the drug-linker conjugated per antibody molecule. The DAR of the antibody-drug conjugates which are used in embodiments of the disclosure is described in more detail below.

Other antibody-drug conjugates may be used in embodiments of the present disclosure. In some embodiments, a compound according to Formula 5 below is released from the antibody-drug conjugate after migration into a target cancer cell, preferably after cleavage of the antibody-drug conjugate at the linker portion: 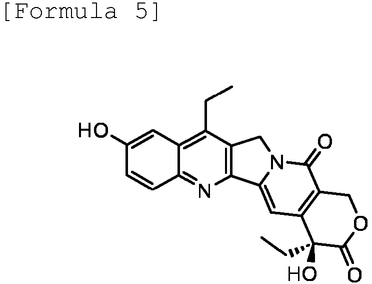 In these embodiments, the compound according to Formula 5 (SN-38) is inferred to be the original source of the antitumor activity of the antibody-drug conjugate.

In some embodiments, the anti-TROP2 antibody-drug conjugate or the anti-HER2 antibody drug conjugate comprises a linker and a drug, the anti-TROP2 antibody or the anti-HER2 antibody being connected to the drug via the linker, the linker and drug forming a drug-linker, and wherein the drug-linker is represented by the following formula: 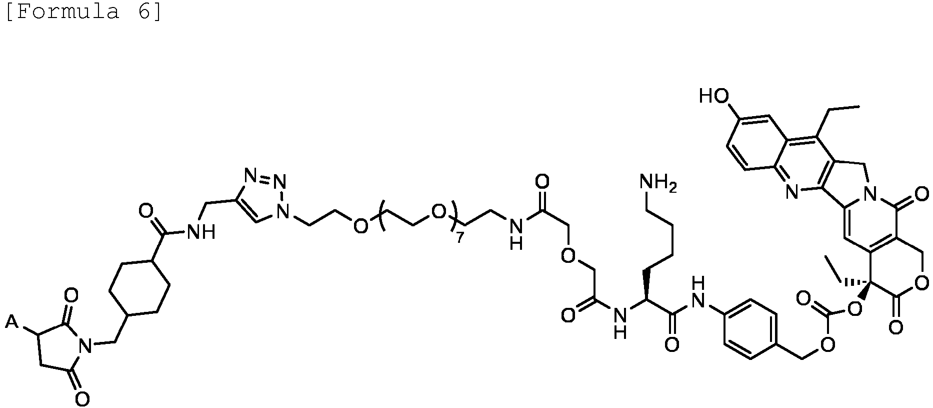 wherein A represents a connecting position to the anti-TROP antibody or the anti-HER2 antibody, and wherein the drug-linker is conjugated to the anti-TROP2 antibody or the anti-HER2 antibody via a thioether bond. For example, an anti-TROP2 antibody-drug conjugate according to the above structure of Formula 6 is sacituzumab govitecan (Trodelvy^{®}, Gilead Sciences).

### 4. Antibody in antibody-drug conjugate

As mentioned above, in preferred embodiments, the antibody in the antibody-drug conjugate used in the present disclosure is an anti-TROP2 antibody or an anti-HER2 antibody. The anti-TROP2 or anti-HER2 antibody in the antibody-drug conjugate used in embodiments of the present disclosure may be derived from any species and is preferably an antibody derived from a human, a rat, a mouse, or a rabbit. In cases when the antibody is derived from species other than human species, it is preferably chimerized or humanized using well-known techniques. The antibody may be a polyclonal antibody or a monoclonal antibody, preferably a monoclonal antibody. Methods of chimerization and humanization discussed above in relation to the anti-CD147 antibody apply by analogy.

The antibody in the antibody-drug conjugate used in embodiments of the present disclosure is an antibody preferably having the characteristic of being able to target cancer cells enabling targeted drug delivery, and is preferably an antibody possessing, for example, the property of being able to recognize a cancer cell, the property of being able to bind to a cancer cell, the property of being internalized in a cancer cell, and/or cytocidal activity against cancer cells. The binding activity of the antibody against cancer cells can be confirmed using flow cytometry. The internalization of the antibody into tumor cells can be confirmed using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labelled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

Since the topoisomerase I inhibitor that is conjugated in the antibody-drug conjugate exerts an antitumor effect, it is preferred but not essential that the antibody itself should have an antitumor effect. In order to specifically and selectively exert cytotoxic activity of the topoisomerase I inhibitor against cancer cells, it is preferred that the antibody should have the property of being internalized to migrate into cancer cells.

The antitumor activity of the antibody can be confirmed *in vitro* by determining inhibitory activity against cell growth. For example, a cancer cell line overexpressing a target protein for the antibody is cultured, and the antibody is added at varying concentrations into the culture system to determine inhibitory activity against focus formation, colony formation, and spheroid growth. The antitumor activity can be confirmed *in vivo,* for example, by administering the antibody to a nude mouse with a transplanted cancer cell line highly expressing the target protein, and determining changes in the cancer cells.

The antibody in the antibody-drug conjugate used in embodiments of the present disclosure can be obtained by a procedure known in the art, as described above in relation to the anti-CD147 antibody.

The antibody of the antibody-drug conjugate used in embodiments of the present disclosure may be modified as described above in relation to the anti-CD147 antibody. In some embodiments, the antibody in the antibody-drug conjugate comprises a heavy chain, from which 1 or 2 amino acid residues are deleted at the carboxyl terminus. In preferred embodiments, the antibody in the antibody-drug conjugate may comprise a first and a second heavy chain, wherein each of the first and the second heavy chain has a deletion of one amino acid residue at the carboxyl terminus. In other embodiments, an amino acid residue at the carboxyl terminus of the heavy chain is amidated.

The isotype of the antibody in the antibody-drug conjugate used in embodiments of the present disclosure is not limited and may be as described above in relation to the anti-CD147 antibody.

### 5. Production of antibody-drug conjugate

A drug-linker intermediate for use in the production of the antibody-drug conjugate according to embodiments of the present disclosure is represented by the following formula.

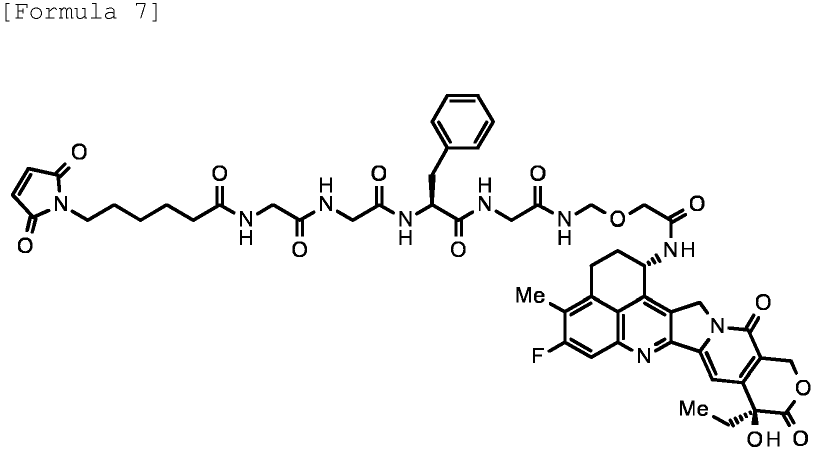

The drug-linker intermediate has the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to the methods described in WO 2014/057687, WO 2015/098099, WO 2015/115091, WO 2015/155998, and WO 2019/044947, each of which is incorporated herein by reference.

The antibody-drug conjugate used in embodiments of the present disclosure may be produced by reacting the above-described drug-linker intermediate and an anti-TROP2 antibody or an anti-HER2 antibody having a thiol group (alternatively referred to as a sulfhydryl group).

The anti-TROP2 antibody or anti-HER2 antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the antibody in a buffer solution containing a chelating agent such as ethylenediamine tetraacetic acid (EDTA), an antibody having a sulfhydryl group with partially or completely reduced interchain disulfides within the antibody can be obtained.

Further, by using 2 to 20 molar equivalents of the drug-linker intermediate per the antibody having a sulfhydryl group, an antibody-drug conjugate in which 2 to 8 drug molecules are conjugated per antibody molecule can be produced.

The average number of conjugated drug molecules per antibody molecule of the antibody-drug conjugate produced can be determined, for example, by a method of calculation based on measurement of UV absorbance for the antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

Conjugation between the antibody and the drug-linker intermediate and calculation of the average number of conjugated drug molecules per antibody molecule of the antibody-drug conjugate can be performed using standard methods as set out in WO2014/057687, WO2017/002776 or WO2022/014698, which are incorporated herein by reference.

### 6. Anti-TROP2 antibody in the antibody-drug conjugate

Preferably, the anti-TROP2 antibody moiety of the antibody-drug conjugate used in embodiments of the present disclosure is an antibody comprising a heavy chain comprising CDRH1 consisting of an amino acid sequence represented by SEQ ID NO: 30 [equivalent to an amino acid sequence consisting of amino acid residues 50 to 54 of SEQ ID NO: 38], CDRH2 consisting of an amino acid sequence represented by SEQ ID NO: 31 equivalent to an amino acid sequence consisting of amino acid residues 69 to 85 of SEQ ID NO: 38], and CDRH3 consisting of an amino acid sequence represented by SEQ ID NO: 32 [equivalent to an amino acid sequence consisting of amino acid residues 118 to 129 of SEQ ID NO: 38], and a light chain comprising CDRL1 consisting of an amino acid sequence represented by SEQ ID NO: 33 [equivalent to an amino acid sequence consisting of amino acid residues 44 to 54 of SEQ ID NO: 39], CDRL2 consisting of an amino acid sequence represented by SEQ ID NO: 34 [equivalent to an amino acid sequence consisting of amino acid residues 70 to 76 of SEQ ID NO: 39], and CDRL3 consisting of an amino acid sequence represented by SEQ ID NO: 35 [equivalent to an amino acid sequence consisting of amino acid residues 109 to 117 of SEQ ID NO: 39].

More preferably, the anti-TROP2 antibody in the antibody-drug conjugate used in embodiments of the present disclosure is an antibody comprising a heavy chain comprising a heavy chain variable region consisting of an amino acid sequence represented by SEQ ID NO: 36 [equivalent to an amino acid sequence consisting of amino acid residues 20 to 140 of SEQ ID NO: 38], and a light chain comprising a light chain variable region consisting of an amino acid sequence represented by SEQ ID NO: 37 [equivalent to an amino acid sequence consisting of amino acid residues 21 to 129 of SEQ ID NO: 39].

Most preferably, the anti-TROP2 antibody in the antibody-drug conjugate used in embodiments of the present disclosure is an antibody comprising a heavy chain consisting of an amino acid sequence represented by SEQ ID NO: 40 [equivalent to an amino acid sequence consisting of amino acid residues 20 to 470 of SEQ ID NO: 38] and a light chain consisting of an amino acid sequence represented by SEQ ID NO: 41 [equivalent to an amino acid sequence consisting of amino acid residues 21 to 234 of SEQ ID NO: 39], or an antibody comprising a heavy chain consisting of an amino acid sequence represented by SEQ ID NO: 42 [equivalent to an amino acid sequence consisting of amino acid residues 20 to 469 of SEQ ID NO: 38] and a light chain consisting of an amino acid sequence represented by SEQ ID NO: 41 [equivalent to an amino acid sequence consisting of amino acid residues 21 to 234 of SEQ ID NO: 39].

In preferred embodiments, the anti-TROP2 antibody in the antibody-drug conjugate comprises a full-length antibody. This facilitates drug conjugation and improves the stability of the antibody-drug conjugate. However, the anti-TROP2 antibody may also comprise an antigen-binding fragment of a full-length antibody. The antigen-binding fragment may be as described above in relation to the anti-CD147 antibody.

The average number of units of the drug-linker conjugated per antibody molecule in the anti-TROP2 antibody-drug conjugate (DAR) is preferably 2 to 8, more preferably 3 to 5, even more preferably 3.5 to 4.5, and most preferably about 4.

In preferred embodiments, the anti-TROP2 antibody-drug conjugate is datopotamab deruxtecan (DS-1062).

The anti-TROP2 antibody-drug conjugate can be produced with reference to the methods set out in WO 2015/098099 and WO 2017/002776 which are incorporated herein by reference.

Other anti-TROP2 antibody-drug conjugates may also be used in embodiments of the present disclosure. As described above, in some embodiments, the anti-TROP2 antibody-drug conjugate is sacituzumab govitecan (Trodelvy^{®}, Gilead Sciences). The anti-TROP2 antibody in this antibody-drug conjugate comprises a heavy chain amino acid sequence according to SEQ ID NO: 54 and a light chain amino acid sequence according to SEQ ID NO: 55.

### 7. Anti-HER2 antibody in the antibody-drug conjugate

Preferably, the anti-HER2 antibody is an antibody comprising a heavy chain comprising CDRH1 consisting of an amino acid sequence according to SEQ ID NO: 43, CDRH2 consisting of an amino acid sequence according to SEQ ID NO: 44 and CDRH3 consisting of an amino acid sequence according to SEQ ID NO: 45, and a light chain comprising CDRL1 consisting of an amino acid sequence according to SEQ ID NO: 46, CDRL2 consisting of an amino acid sequence according to SEQ ID NO: 47 and CDRL3 consisting of an amino acid sequence according to SEQ ID NO: 48.

More preferably, the anti-HER2 antibody is an antibody comprising a heavy chain comprising a heavy chain variable region consisting of an amino acid sequence according to SEQ ID NO: 49, and a light chain comprising a light chain variable region consisting of an amino acid sequence according to SEQ ID NO: 50.

Even more preferably, the anti-HER2 antibody is an antibody comprising a heavy chain consisting of an amino acid sequence according to SEQ ID NO: 51 and a light chain consisting of an amino acid sequence according to SEQ ID NO: 52, or an antibody comprising a heavy chain consisting of amino acid residues 1 to 449 of SEQ ID NO: 51 and a light chain consisting of an amino acid sequence according to SEQ ID NO: 52.

In preferred embodiments, the anti-HER2 antibody in the antibody-drug conjugate comprises a full-length antibody. This facilitates drug conjugation and improves the stability of the antibody-drug conjugate. However, the anti-HER2 antibody may also comprise an antigen-binding fragment of a full-length antibody. The antigen-binding fragment may be as described above in relation to the anti-CD147 antibody.

The average number of units of the drug-linker conjugated per antibody molecule in the anti-HER2 antibody-drug conjugate (DAR) is preferably 2 to 8, more preferably 3 to 8, even more preferably 7 to 8, even more preferably 7.5 to 8, and even more preferably about 8.

The anti-HER2 antibody-drug conjugate used in embodiments of the present disclosure can be produced with reference to the method set out in WO2015/115091 which is incorporated herein by reference.

In preferred embodiments, the anti-HER2 antibody-drug conjugate is trastuzumab deruxtecan (DS-8201).

Other antibody-drug conjugates may be used in embodiments of the present disclosure. In some embodiments, the drug moiety of the anti-HER2 antibody-drug conjugate comprises SN-38 such that a compound according to Formula 5 above is released from the antibody-drug conjugate after migration of the antibody-drug conjugate into a target cancer cell, and preferably after cleavage of the antibody-drug conjugate at the linker portion. The anti-HER2 antibody drug conjugate may have the drug-linker structure according to Formula 6.

### 8. Therapeutic use and pharmaceutical formulations

In accordance with some embodiments of the present disclosure, an anti-CD147 antibody and an antibody-drug conjugate as described herein, are administered to a subject in combination for the prevention or treatment of cancer in the subject. In preferred embodiments, the antibody-drug conjugate is an anti-TROP2 antibody-topoisomerase I inhibitor conjugate or an anti-HER2 antibody- topoisomerase I inhibitor conjugate.

In some embodiments of the present disclosure, the anti-CD147 antibody and the antibody-drug conjugate are separately contained as active ingredients in different formulations. In some embodiments, the anti-CD147 antibody and the antibody-drug conjugate are administered simultaneously, whereas in other embodiments they are administered at different times (i.e. sequentially). In still further embodiments, the anti-CD147 antibody and the antibody-drug conjugate are contained as active components in a single formulation. As such, in one embodiment, the present disclosure provides a pharmaceutical composition comprising the anti-CD147 antibody and an antibody-drug conjugate as described herein. In other embodiments, the present disclosure provides a kit comprising the anti-CD147 antibody and an antibody-drug conjugate as described herein. In the kit, the anti-CD147 antibody and the antibody-drug conjugate may be provided as separate pharmaceutical formulations. Preferably, the kit is suitable for the prevention or treatment of cancer.

In some embodiments of the disclosure, the cancer to be prevented or treated by a combination therapy using an anti-CD147 antibody and an anti-TROP2 antibody-drug conjugate as described herein may be at least one cancer selected from the group consisting of: breast cancer (including triple negative breast cancer and hormone receptor (HR)-positive, HER2-negative breast cancer), lung cancer (including small cell lung cancer and non-small cell lung cancer), colorectal cancer (also called colon and rectal cancer, and including colon cancer and rectal cancer), gastric cancer (also called gastric adenocarcinoma), esophageal cancer, head-and-neck cancer (including salivary gland cancer and pharyngeal cancer), esophagogastric junction adenocarcinoma, biliary tract cancer (including bile duct cancer), Paget's disease, pancreatic cancer including pancreatic ductal adenocarcinoma, ovarian cancer, uterine carcinosarcoma, urothelial cancer, prostate cancer, bladder cancer, gastrointestinal stromal tumor, cervical cancer (including uterine cervix cancer), squamous cell carcinoma, peritoneal cancer, liver cancer, hepatocellular cancer, corpus uteri carcinoma, kidney cancer, vulval cancer, thyroid cancer, penis cancer, leukemia, malignant lymphoma, plasmacytoma, myeloma, glioblastoma multiforme, osteosarcoma, sarcoma, endometrial cancer, and melanoma.

In preferred embodiments of the present disclosure, the cancer to be prevented or treated by a combination therapy using an anti-CD147 antibody and an anti-TROP2 antibody-drug conjugate as described herein may be selected from the group consisting of non-small cell lung cancer, triple negative breast cancer, hepatocellular cancer, pancreatic ductal adenocarcinoma, and colorectal cancer.

In more preferred embodiments of the present disclosure, the cancer to be prevented or treated by a combination therapy using an anti-CD147 antibody and an anti-TROP2 antibody-drug conjugate as described herein may be selected from triple negative breast cancer and non-small cell lung cancer.

In some embodiments of the present disclosure, the cancer to be prevented or treated by a combination therapy using an anti-CD147 antibody and an anti-HER2 antibody-drug conjugate as described herein may be at least one cancer selected from the group consisting of breast cancer (including hormone receptor (HR)-positive breast cancer), lung cancer (including small cell lung cancer and non-small cell lung cancer), colorectal cancer (also called colon and rectal cancer, and including colon cancer and rectal cancer), gastric cancer (also called gastric adenocarcinoma), esophageal cancer, head-and-neck cancer (including salivary gland cancer and pharyngeal cancer), esophagogastric junction adenocarcinoma, biliary tract cancer (including bile duct cancer), Paget's disease, pancreatic cancer including pancreatic ductal adenocarcinoma, ovarian cancer, uterine carcinosarcoma, urothelial cancer, prostate cancer, bladder cancer, gastrointestinal stromal tumor, cervical cancer (including uterine cervix cancer), squamous cell carcinoma, peritoneal cancer, liver cancer, hepatocellular cancer, corpus uteri carcinoma, kidney cancer, vulval cancer, thyroid cancer, penis cancer, leukemia, malignant lymphoma, plasmacytoma, myeloma, glioblastoma multiforme, osteosarcoma, sarcoma, endometrial cancer, and melanoma.

In preferred embodiments of the present disclosure, the cancer to be prevented or treated by a combination therapy using an anti-CD147 antibody and an anti-HER2 antibody-drug conjugate as described herein may be selected from the group consisting of HER2-positive breast cancer, lung cancer including non-small cell lung cancer, gastric cancer, hepatocellular cancer, pancreatic ductal adenocarcinoma, and colorectal cancer.

In more preferred embodiments of the disclosure, the cancer to be prevented or treated by a combination therapy using an anti-CD147 antibody and an anti-HER2 antibody-drug conjugate as described herein may be gastric cancer.

In some embodiments of the combination therapy of the present disclosure (using an anti-CD147 antibody and an antibody-drug conjugate as described herein), the cancer cells may have a BRCA1 and/or a BRCA2 deficient phenotype i.e. BRCA1 and/or BRCA2 activity is reduced or abolished in the cancer cells. This may arise by means of mutation or polymorphism in the encoding nucleic acid, or by means of amplification, mutation or polymorphism in a gene encoding a regulatory factor, for example the EMSY gene which encodes a BRCA2 regulatory factor (Hughes-Davies, et al., Cell, 115, 523-535). BRCA1 and BRCA2 are known tumor suppressors whose wild-type alleles are frequently lost in tumors of heterozygous carriers (Jasin M., Oncogene, 21(58), 8981-93 (2002); Tutt, et al., Trends Mol Med., 8 (12), 571-6, (2002)). The association of BRCA1 and/or BRCA2 mutations with breast cancer is well-characterised in the art (Radice, P.J., Exp Clin Cancer Res., 21(3 Suppl) , 9-12 (2002)). Amplification of the EMSY gene, which encodes a BRCA2 binding factor, is also known to be associated with breast and ovarian cancer. Carriers of mutations in BRCA1 and/or BRCA2 are also at elevated risk of certain cancers, including breast, ovary, pancreas, prostate, hematological, gastrointestinal and lung cancer. In some embodiments, the subject to be treated is heterozygous for one or more variations, such as mutations and polymorphisms, in BRCA1 and/or BRCA2 or a regulator thereof. The detection of variation in BRCA1 and BRCA2 is well-known in the art and is described, for example in EP 699 754, EP 705 903, Neuhausen, S.L. and Ostrander, E.A., Genet. Test, 1, 75-83 (1992); Chappnis, P.O. and Foulkes, W.O., Cancer Treat Res, 107, 29-59 (2002); Janatova M., et al., Neoplasma, 50(4), 246-505 (2003); Jancarkova, N., Ceska Gynekol., 68{1), 11-6 (2003)). Determination of amplification of the BRCA2 binding factor EMSY *is* described in Hughes-Davies, et al., Cell, 115, 523-535). Furthermore, the cancer may or may not be deficient in homologous recombination-dependent DNA double-stranded break repair.

In preferred embodiments of the combination therapy of the present disclosure (using an anti-CD147 antibody and an anti-antibody-drug conjugate as described herein), the cancer is selected from: non-small cell lung cancer; breast cancer, preferably triple negative breast cancer, and more preferably, triple negative breast cancer without a BRACA1 mutation; and gastric cancer.

In some embodiments of the combination therapy of the present disclosure (using an anti-CD147 antibody and an antibody-drug conjugate as described herein), the cancer cells express CD147. In other embodiments, the cancer cells express TROP2 and/or HER2, preferably, in addition to CD147. In some embodiments, the combination therapy described herein is used to prevent or treat cancer in a mammal, and more preferably, in a human.

An antitumor effect of the combination of the anti-CD147 antibody and antibody-drug conjugate according to embodiments of the present disclosure can be confirmed by generating a model in which cancer cells are transplanted into a test animal, and measuring the reduction of tumor volume and/or life-prolonging effect. Furthermore, comparison to antitumor effects obtained by single administrations of each of the anti-CD147 antibody and antibody-drug conjugate may provide confirmation of a combined or synergistic effect of the anti-CD147 antibody and antibody-drug conjugate.

In addition, an antitumor effect of the combination of the anti-CD147 antibody and antibody-drug conjugate according to embodiments of the present disclosure can be confirmed, in a clinical study, with Response Evaluation Criteria in Solid Tumors (RECIST) evaluation method, WHO's evaluation method, Macdonald's evaluation method, measurement of body weight, and other methods. The antitumor effect can also be determined by indicators such as Complete response (CR), Partial response (PR), Progressive disease (PD), Objective response rate (ORR), Duration of response (DoR), Progression-free survival (PFS), and Overall survival (OS) .

The foregoing methods can provide confirmation of superiority in terms of the antitumor effect of the combination of embodiments of the present disclosure with respect to existing pharmaceutical products and treatment methods for cancer therapy.

The combination of the anti-CD147 antibody and antibody-drug conjugate according to embodiments of the present disclosure can retard cancer cell growth, suppress its proliferation, and further can disrupt cancer cells. These effects can allow cancer patients to be free from symptoms caused by cancer or achieve improvement in QOL of a cancer patients, and attain a therapeutic effect by sustaining the lives of the cancer patients.

The combination of the anti-CD147 antibody and antibody-drug conjugate according to embodiments of the present disclosure can exert a therapeutic effect by application as systemic therapy to patients, and additionally, by local application to cancer tissues.

In the following, the term "pharmaceutical formulation" refers to the preparations containing the anti-CD147 antibody and/or antibody-drug conjugate. Thus, the term includes a pharmaceutical composition comprising both the anti-CD147 antibody and antibody-drug conjugate as described above; or separate preparations comprising the anti-CD147 antibody and antibody-drug conjugate (but not both). The pharmaceutical formulations which are administered in embodiments of the present disclosure may be contain at least one pharmaceutically acceptable ingredient. The pharmaceutically acceptable ingredient can be suitably selected and applied from formulation additives or the like that are generally used in the art, in accordance with the dosage, administration concentration or the like of the anti-CD147 antibody and antibody-drug conjugate used in embodiments of the present disclosure. For example, the pharmaceutical formulation used in embodiments of the present disclosure can be administered as a preparation containing a buffer such as a histidine buffer, an excipient such as sucrose or trehalose, and a surfactant such as Polysorbate 80 or 20. The pharmaceutical formulation may preferably be used as an injection, for example, as an aqueous injection or a lyophilized injection. In the case that the pharmaceutical formulation containing the anti-CD147 antibody, anti-TROP2 antibody-drug conjugate and/or anti-HER2 antibody drug conjugate used in embodiments of the present disclosure is an aqueous injection, it can be preferably diluted with a suitable diluent and then given as an intravenous infusion. For the diluent, a dextrose solution, physiological saline, and the like, can be exemplified. A dextrose solution (for example, 5% concentration) is preferred.

Examples of the administration route which may be used to administer the pharmaceutical formulation of the present disclosure include intravenous, intradermal, subcutaneous, intramuscular, and intraperitoneal routes, and preferably include an intravenous route.

The anti-CD147 antibody used in embodiments of the present disclosure can be administered to a human at a dose of about 0.1 to 100 mg/kg, about 0.1 to 50 mg/kg, about 1 to 40 mg/kg, about 20 to 50 mg/kg, or about 20 to 30 mg/kg. In some embodiments, the anti-CD147 antibody used in embodiments of the present disclosure can be administered to a human at a dose of about 20 mg/kg, 40 mg/kg, 45 mg/kg or 50 mg/kg. The antibody can be administered to a human daily, every other day, twice per week, three times per week, four times per week, five times per week, six times per week, once per week, once every two weeks, once every three weeks, once every four weeks, once every five weeks, once every six weeks, once every seven weeks, once every eight weeks, once every nine weeks, once every ten weeks, once every eleven weeks, once every twelve weeks, twice per year, or once per year. The antibody can preferably be administered once a week, once every 2 weeks, once every 3 weeks, or once every 4 weeks. More preferably, it can be administered once every 2 weeks, once every 3 weeks, or once every 4 weeks.

The anti-TROP2 antibody-drug conjugate used in embodiments of the present disclosure can be administered to a human once at intervals of 1 to 180 days, and can preferably be administered once a week, once every 2 weeks, once every 3 weeks, or once every 4 weeks. More preferably, it can be administered once every 3 weeks. The anti-TROP2 antibody-drug conjugate used in embodiments of the present disclosure can be administered at a dose of about 0.001 to 100 mg/kg, and preferably, at a dose of 0.8 to 12.4 mg/kg per administration. For example, the anti-TROP2 antibody-drug conjugate can be administered once every 3 weeks at a dose of 0.27 mg/kg, 0.5 mg/kg, 1.0 mg/kg, 2.0 mg/kg, 3.0 mg/kg, 4.0 mg/kg, 6.0 mg/kg, or 8.0 mg/kg, and can preferably be administered once every 3 weeks at a dose of 6.0 mg/kg.

The anti-HER2 antibody-drug conjugate used in embodiments of the present disclosure can be administered to a human once at intervals of 1 to 180 days, and can preferably be administered once a week, once every 2 weeks, once every 3 weeks, or once every 4 weeks. More preferably, it can be administered once every 3 weeks. The anti-HER2 antibody-drug conjugate used in embodiments of the present disclosure can be administered at a dose of about 0.001 to 100 mg/kg per administration, and can preferably be administered at a dose of 0.8 to 12.4 mg/kg per administration. For example, the anti-HER2 antibody-drug conjugate can be administered once every three weeks at a dose of 0.8 mg/kg, 1.6 mg/kg, 3.0 mg/kg, 3.2 mg/kg, 5.4 mg/kg, 6.4 mg/kg, 7.4 mg/kg, 8 mg/kg or 10 mg/kg, and can preferably be administered once every three weeks at a dose of 5.4 mg/kg or 6.4 mg/kg.

However, the dosage and the frequency of administration should generally be determined in consideration of the gender, body weight, and age of a patient, symptoms, severity, any adverse reactions, and the like, and thus doses and methods therefor are not limited to the above examples.

The combination therapy according to embodiments of the present disclosure may be used as an adjuvant therapy in addition to surgery. The pharmaceutical formulations comprising the anti-CD147 antibody and anti-TROP2 antibody-drug conjugate or anti-HER2 antibody-drug conjugate may be administered for the purpose of reducing tumor size before surgical operation (referred to as preoperative adjuvant therapy or neoadjuvant therapy), or may be administered for the purpose of preventing recurrence of tumor after surgical operation (referred to as postoperative adjuvant therapy or adjuvant therapy).

The anti-CD147 antibody and antibody-drug conjugate used in embodiments of the present disclosure may be administered in combination with another anti-cancer agent, thereby enhancing an antitumor effect. The other anti-cancer agent to be used for such purpose may be administered to a subject simultaneously, separately, or sequentially with the anti-CD147 antibody and antibody-drug conjugate. Such anti-cancer agents are not limited as long as they are agents having antitumor activity, and would be known to a person skilled in the art. The anti-cancer agent may be a chemotherapeutic agent other than a topoisomerase I inhibitor. In some embodiments, the anti-cancer agent may comprise an immune checkpoint inhibitor. The immune checkpoint inhibitor may be an anti-PD1 antibody (including but not limited to nivolumab, pembrolizumab, spartalizumab, semiplimab), an anti-PD-L1 antibody (including but not limited to avelumab, atezolizumab, and durvalumab), and/or an anti-CTLA4 antibody (including but not limited to tremelimumab and ipilimumab). In other embodiments, the anti-cancer agent may comprise an EZH1/2 inhibitor (including but not limited to valemetstat).

In some embodiments, the anti-CD147 antibody and/or antibody-drug conjugate are formulated together with a pharmaceutically acceptable carrier, diluent, solubilizer, emulsifier, preservative, auxiliary agent or others. The "pharmaceutically acceptable carrier" and others can be appropriately selected from a wide range in accordance with the type of target disease and the dosage form of a drug.

An appropriate method for administering the anti-CD147 antibody and/or antibody-drug conjugate according to the present disclosure can be selected, for example, administration by injection. Examples of the injection that can be employed include local injection, intraperitoneal injection, selective intravenous injection, intravenous injection, subcutaneous injection and organ-perfusate injection. The injection solution can be formulated by using a carrier consisting of a salt solution, a glucose solution, or a mixture of a salt solution and a glucose solution, and any one of the buffers. Alternatively, the injection solution may be prepared by formulating a powder preparation and mixing the powder preparation with the aforementioned liquid carrier, when used.

Other administration methods can be selected appropriately together with the development of formulations. For example, for oral administration, oral liquids, powders, pills, capsules and tablets can be applied. In the case of an oral liquid, an oral liquid preparation such as a suspension and a syrup can be produced by using water; a sugar such as sucrose, sorbitol and fructose; a glycol such as polyethylene glycol; an oil such as sesame oil and soybean oil; a preservative such as alkyl parahydroxybenzoate; and a flavor such as strawberry flavor and peppermint flavor. Powders, pills, capsules and tablets can be formulated by using, e.g., an excipient such as lactose, glucose, sucrose and mannitol; a disintegrant such as starch and soda alginate; a lubricant such as magnesium stearate and talc; a binder such as polyvinyl alcohol, hydroxypropyl cellulose and gelatin; a surfactant such as a fatty acid ester; and a plasticizer such as glycerin. Tablets and capsules are easily administered. In this respect, they are preferable unit dosage forms of the composition of the present disclosure. In producing tablets and capsules, a solid production carrier is used.

### 9. Synergistic effect

The anti-CD147 antibody and the anti-TROP2 antibody-topoisomerase I inhibitor drug conjugate or the anti-HER2 antibody-topoisomerase I inhibitor drug conjugate described herein produce a synergistic effect in the treatment of cancer.
Without wishing to be bound by theory, it is believed that the anti-CD147 antibody may cause a reduction in CD147 expression on cancer cells. CD147 acts as a molecular chaperone and stabilises various protein molecules in the cell membrane with which it interacts. The reduction in CD147 expression may cause a loss of stabilisation of transporters which are involved in the efflux of drug molecules. As such, it is believed there may be an increased retention of the topoisomerase I inhibitor which is delivered to the cancer cells by the anti-TROP2 antibody moiety or anti-HER2 antibody moiety of the antibody-drug conjugate, resulting in increased cell death.

It is to be appreciated that the present disclosure is particularly useful in patient groups in which the patient has had an insufficient clinical response to existing therapies.

### [Examples]

The following examples describe various embodiments of the present disclosure. However, the disclosure is not limited to these embodiments, and the examples are not intended to be interpreted in any limiting manner.

With reference to the following examples, the humanised #110H1L4hIgG4P antibody comprises a heavy chain consisting of amino acid residues at positions 20 to 463 in the amino acid sequence represented by SEQ ID NO: 13, and a light chain consisting of amino acid residues at positions 21 to 234 in the amino acid sequence represented by SEQ ID NO: 15. This antibody was prepared according to the methods described in WO2019/022187 which is incorporated herein by reference (see in particular, Example 6).

The anti-TROP2 antibody drug conjugate used in the following Examples 1 to 4 is datopotamab deruxtecan (Dato-DXd, DS-1062). The DAR of the antibody-drug conjugate is ~4. The anti-TROP2 antibody in this antibody-drug conjugate comprises a heavy chain consisting of an amino acid sequence represented by SEQ ID NO: 40 [equivalent to amino acid residues 20 to 470 of SEQ ID NO: 38] and a light chain consisting of an amino acid sequence represented by SEQ ID NO: 41 [equivalent to amino acid residues 21 to 234 of SEQ ID NO: 39. The drug-linker in the antibody-drug conjugate is represented by Formula (1) as described above. Datopotamab deruxtecan was prepared as described in Example 1 of WO2023/089527 which is incorporated herein by reference.

The anti-TROP2 antibody drug conjugate used in Example 5 is sacituzumab govitecan (Trodelvy^{®}). The drug-linker in the antibody-drug conjugate is represented by Formula (6) as described above. The anti-TROP2 antibody in this antibody-drug conjugate comprises a heavy chain amino acid sequence according to SEQ ID NO: 54 and a light chain amino acid sequence according to SEQ ID NO: 55. Sacituzumab govitecan was prepared according to the methods described in WO2015098099 which is incorporated herein by reference (see in particular, paragraphs [0205]-[0208] describing the preparation of the hRS7 (anti-TROP2) antibody and paragraph [0266] describing the preparation of the antibody-drug conjugate). The DAR of sacituzumab govitecan is ~8.

The anti-HER2 antibody drug conjugate used in the following Example 6 is trastuzumab deruxtecan (T-DXd, DS-8201). The anti-HER2 antibody in the antibody-drug conjugate comprises a heavy chain consisting of an amino acid sequence represented by SEQ ID NO: 51 and a light chain consisting of the amino acid sequence represented by SEQ ID NO: 52. The drug-linker in the antibody-drug conjugate is represented by Formula (1) as described above. The DAR of the antibody-drug conjugate is 7.7 or 7.8. Trastuzumab deruxtecan was prepared by the methods set out in Example 50 (paragraphs [0326] and [0327]) of WO2015/115091 which is incorporated herein by reference.

### Example 1)- Antitumor responses of monotherapy and combination therapy using #110H1L4hIgG4P and Dato-DXd in a EBC-1 xenograft model (non-small cell lung cancer)

### 1)-1 Rationale and objectives

The inventors investigated whether a combination therapy using an anti-CD147 antibody and anti-TROP2 antibody-drug conjugate could suppress tumor growth to a greater extent than the corresponding monotherapies in an EBC-1 xenograft model.

### 1)-2 Materials and Methods

EBC-1 cells (JCRB, catalogue #JCRB0820) (a squamous non-small cell lung cancer cell line) were cultured in E-MEM with 10% FBS. One million cells were injected subcutaneously into the right flank of female, 5-week-old NODscid mice for tumor implantation. Following tumor randomization, the Dato-DXd was administered once intravenously at a dose of 1 mg/kg on day 7 post tumor inoculation when the tumor reached a volume of about 130 mm³ (n=6). The #110H1L4hIgG4P was administered intravenously at a dose of 20 mg/kg weekly for two weeks, starting on day 8 post tumor inoculation (n=6). The tumor volume in each group of mice was determined twice per week. To analyse the expression of CD147 and ABCC1, and to determine the amount of DXd (deruxtecan) in the tumor, tumor samples were obtained from mice treated with Dato-DXd and #110H1L4hIgG4P on day 10 post tumor inoculation.

The tumor samples were lysed using GentleMACS M-tubes (Miltenyi Biotec, catalogue number 130096335) in M-PER Mammalian Protein Extraction Reagent (Thermo Fisher Scientifics, catalogue number 78501) supplemented with Pierce Protease and Phosphate Inhibitor Mini Tablets, EDTA-free (Thermo Fisher Scientific, catalogue number A32961), and stored at -80°C until use. The protein concentration was measured by using Pierce BCA Protein Assay Kit (Thermo Fisher Scientifics, catalogue number 23225) in Envision Multilabel Reader (Perkin Elmer, catalogue number 2104). The protein expression was examined by using Abby, Simple Western system (Bio-Techne, catalogue number 004-680) using standard separation module and detection module with anti-CD147 antibody (R&D, catalogue number AF972), anti-MRP1/ABCC1 antibody (CST, catalogue number72202), anti-rabbit secondary antibody (protein simple, catalogue number 042-206) and anti-goat secondary antibody (R&D, catalogue number HAF109).

The tumor samples were ground immediately after the collection and stored at -80°C for measurement of intratumor DXd levels. The DXd levels were determined using LC-MS/MS method with Nexera X2 (SHIMADZU) and Triple Quad 6500+ (AB Sciex Pte. Ltd.). The lower limit of quantification was 0.0400 ng/g tissue.

Any synergy between #110H1L4hIgG4P and Dato-DXd was evaluated in this and subsequent Examples by calculating the combination index based on the Bliss independent model (Duarte D and Vale N, Current Research in Pharmacology and Drug Discovery (2022)). Specifically, if the effects of Drug A and Drug B are represented as Ea and Eb, respectively, and the effect of their combination is represented as Eab, the Combination Index (CI) can be calculated using the formula (Ea + Eb - EaEb) / Eab. A CI of 1 indicates an additive effect, a CI greater than 1 indicates an antagonistic effect, and a CI less than 1 indicates a synergistic effect.

### 1)-3 Results

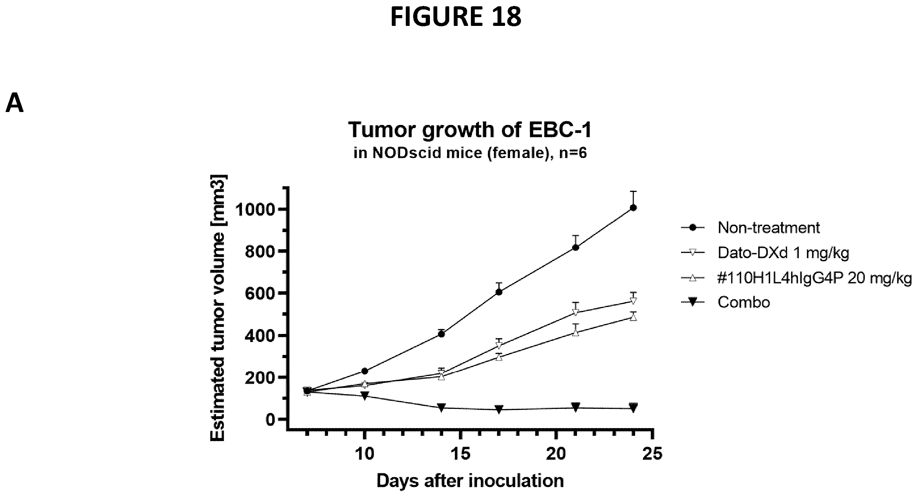

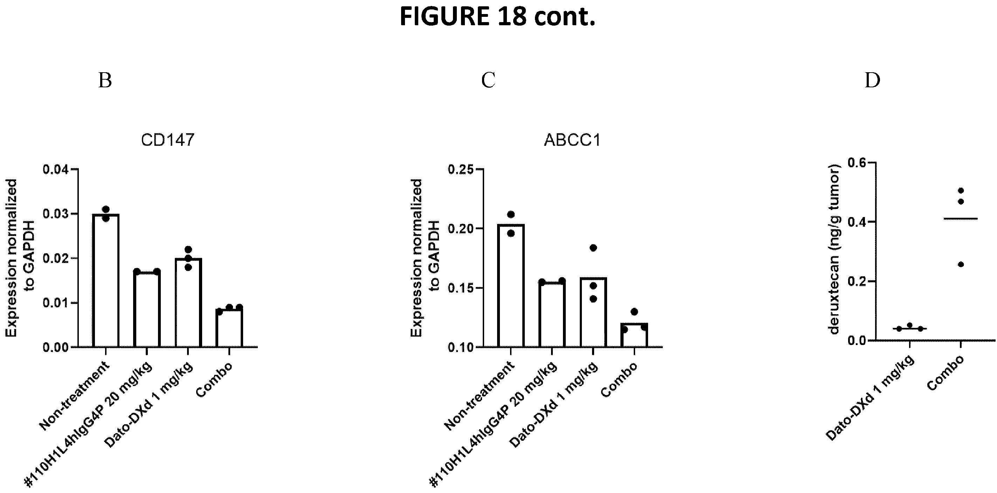

The in vivo efficacy of the combination therapy of #110H1L4hIgG4P and Dato-DXd was evaluated in the EBC-1 subcutaneous xenograft model. Treating tumor-bearing mice with #110H1L4hIgG4P, Dato-DXd, and the combination resulted in tumor growth inhibition of 52%, 44%, and 95%, respectively, compared to the non-treatment control on day 24, as shown in Fig. 18A. The combination therapy synergistically enhanced the individual antitumor effects of the #110H1L4hIgG4P and Dato-DXd, as indicated by a CI of 0.77. Expression of CD147, a target of #110H1L4hIgG4P, was lower in mice treated with #110H1L4hIgG4P and Dato-DXd monotherapy compared to the non-treatment group, while the expression of CD147 showed further reduction in mice receiving combination treatment, as shown in Fig. 18B. This suggests that CD147 expression was decreased by #110H1L4hIgG4P and Dato-DXd monotherapy and the reduction of CD147 expression was enhanced in the combination treatment. Furthermore, the expression of ABCC1, an efflux transporter of intracellular drugs including ADC payloads such as DXd, showed a similar expression pattern to that of CD147 (Fig. 18C). Consistent with the above, the DXd levels in the tumor samples with Dato-DXd monotherapy were either 0.0524 ng/g of tissue (n=1) or lower than detection limit (0.0400 ng/g of tissue) (n=2). In contrast, the DXd levels in the tumor samples from mice receiving the combination therapy increased to mean value of 0.411 ng/g of tissue with a standard deviation of 0.134 (n=3) (Fig. 18D).

CD147 is known to function as a molecular chaperone and contributes to the stabilization of various proteins with which it interacts. Without wishing to be bound by theory, we postulate that the enhanced reduction in CD147 expression achieved by the #110H1L4hIgG4P and Dato-DXd combination therapy consequently leads to an enhanced reduction in ABCC1 expression. This is turn, increases the amount of DXd available in the tumor cells and extends DXd retention, and results in the synergistically-enhanced antitumor effects observed with the combination therapy. However, transporters other than ABCC1 may be involved in the efflux of DXd, and different mechanisms may be involved depending on the cell type and cancer type.

### Example 2 - Antitumor responses of monotherapy and combination therapy using #110H1L4hIgG4P and Dato-DXd in an NCI-H358 xenograft model (non-small cell lung cancer)

### 2)-1 Rationale and objectives

The inventors investigated whether a combination therapy using an anti-CD147 antibody and anti-TROP2 antibody-drug conjugate could suppress tumor growth to a greater than the corresponding monotherapies in an NCI-H358 xenograft model.

### 2)-2 Materials and Methods

NCI-H358 cells (ATCC, catalogue#CRL-5807) (adenocarcinoma cell line of non-small cell lung cancer) were cultured in ATCC-modified RPMI1640. Three million cells were injected subcutaneously into the right flank of female, 6-week-old NODscid mice for tumor implantation. Following tumor randomization, the Dato-DXd was administered once intravenrously at a dose of 1 mg/kg on day 7 post tumor inoculation when tumor reached the volume about 200 mm³(n=6). The #110H1L4hIgG4P was administered intravenously at a dose of 20 mg/kg weekly for two weeks, starting on day 8 post tumor inoculation (n=6). For DXd measurements, tumor samples were obtained from mice treated with Dato-DXd and #110H1L4hIgG4P on day 10 post tumor inoculation. The tumor volume in each group of mice was determined twice per week.

The tumor samples were ground immediately after the collection and stored at -80°C for measurement of intratumor DXd levels. The DXd levels were determined according to the method described in the Example 1.

### 2)-3 Results

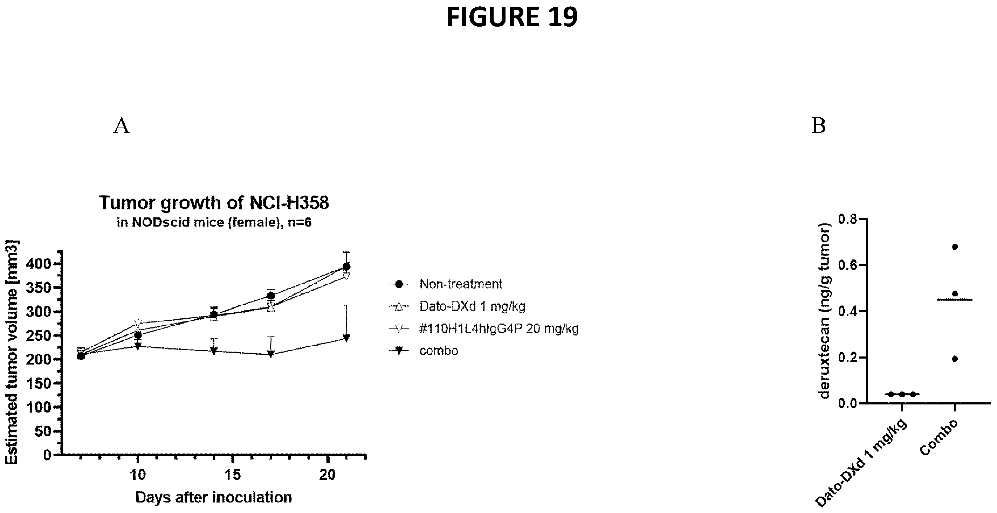

The *in vivo* efficacy of the combination therapy of #110H1L4hIgG4P and Dato-DXd was evaluated in the NCI-H358 subcutaneous xenograft model. Treating tumor-bearing mice with #110H1L4hIgG4P, Dato-DXd, and the combination resulted in tumor growth inhibition of 0%, 5%, and 38%, respectively, compared to the non-treatment control on day 21, as shown in Fig. 19A. The combination therapy synergistically enhanced antitumor effects compared with the monotherapies, as indicated by a combination index of 0.13. As shown in the Fig. 19B, intratumoral DXd levels in samples obtained from mice receiving the Dato-DXd monotherapy is lower than the detection limit (0.0400 ng/g tissue), whereas those in tumor samples obtained from mice receiving the combination therapy were increased to a mean value of 0.450 ng/g of tissue with a standard deviation of 0.244 (Fig. 19B). These results demonstrate that the synergistically-improved anti-tumor effects observed with the combination therapy were associated with extended DXd retention in the NCI-H358 xenograft models.

### Example 3 - Antitumor responses of monotherapy and combination therapy using #110H1L4hIgG4P and Dato-DXd in a HCC-1937 xenograft model (triple negative breast cancer)

### 3)-1 Rationale and objectives

The inventors investigated whether a combination therapy using an anti-CD147 antibody and anti-TROP2 antibody-drug conjugate could suppress tumor growth to a greater extent than the corresponding monotherapies in an HCC-1937 xenograft model.

### 3)-2 Materials and Methods

HCC-1937 cells (ATCC, catalogue#CRL-2336) (a triple negative breast cancer cell line with BRACA1 mutation) were cultured in ATCC-modified RPMI1640 with 10% FBS. Five million cells were injected subcutaneously into the right flank of female, 6-week-old NODscid mice for tumor implantation. Following tumor randomization, the Dato-DXd was administered intravenously once at a dose of 3 mg/kg on day 28 post tumor inoculation when the tumor reached a volume of about 125 mm³. The #110H1L4hIgG4P was administered intravenously at a dose of 20 mg/kg weekly for two weeks, starting on day 29 post tumor inoculation. The tumor volume in each group of mice was determined twice per week.

### 3)-3 Results

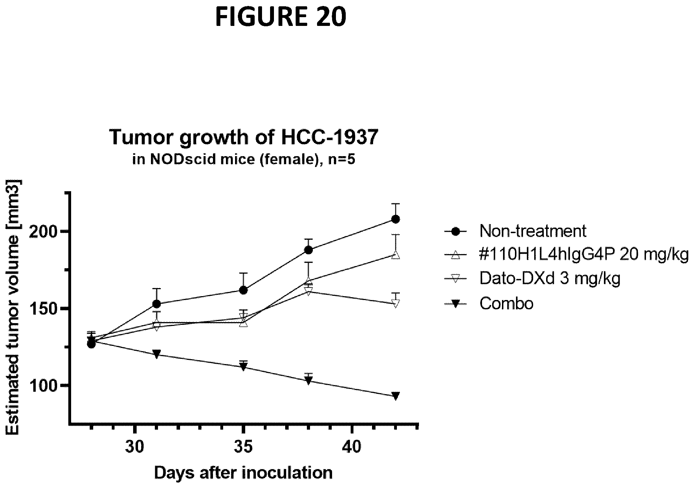

The *in vivo* efficacy of the combination therapy of #110H1L4hIgG4P and Dato-DXd was evaluated in the HCC-1937 subcutaneous xenograft model. Treating tumor-bearing mice with #110H1L4hIgG4P, Dato-DXd, and the combination resulted in a tumor growth inhibition of 11%, 26%, and 55%, respectively, compared to the non-treatment control on day 42, as shown in Fig. 20. The combination therapy synergistically enhanced antitumor effects in the HCC-1937 xenograft model as compared to the corresponding monotherapies, as evidenced by a combination index of 0.62.

### Example 4 - Antitumor responses of monotherapy and combination therapy using #110H1L4hIgG4P and Dato-DXd in a HCC-1806 xenograft model (triple negative breast cancer without BRACA1 mutation)

### 4)-1 Rationale and objectives

The inventors investigated whether a combination therapy using an anti-CD147 antibody and anti-TROP2 antibody-drug conjugate could suppress tumor growth to a greater extent than the corresponding monotherapies in a HCC-1806 xenograft model.

### 4)-2 Materials and Methods

HCC-1806 cells (ATCC, catalogue#CRL-2335) (triple negative breast cancer cell line without a BRACA1 mutation), were cultured in ATCC-modified RPMI1640 with 10% FBS. Five million cells were injected subcutaneously into the right flank of female, 6-week-old NODscid mice for tumour implantation. Following tumor randomization, the Dato-DXd was administered intravenously once at a dose of 3 mg/kg on day 4 post tumor inoculation when the tumor reached a volume of about 170 mm³(n=6). The #110H1L4hIgG4P was administered intravenously at a dose of 20 mg/kg weekly for two weeks, starting on day 5 post tumor inoculation (n=6). The tumor volume in each group of mice was determined twice per week.

### 4)-3 Results

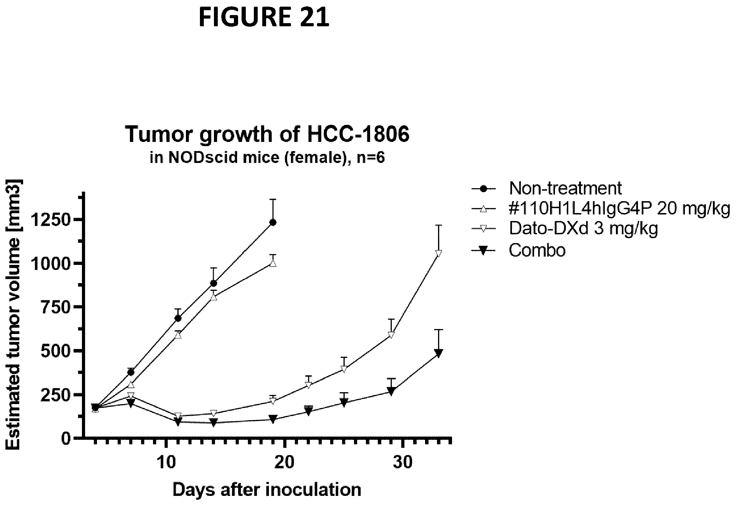

The *in vivo* efficacy of the combination therapy of #110H1L4hIgG4P and Dato-DXd was evaluated in the HCC-1806 subcutaneous xenograft model. Treating tumor-bearing mice with #110H1L4hIgG4P resulted in a tumor growth inhibition of 19% compared to the non-treatment control on day 19 after inoculation, as shown in Fig. 21. Treatment with Dato-DXd, and the combination of #110H1L4hIgG4P and Dato-DXd, showed tumor regression to a similar extent until day 11. From day 11 onwards, tumor regrowth was suppressed to a greater extent in mice receiving the combination therapy compared to those treated with Dato-DXd monotherapy and #110H1L4hIgG4P monotherapy.

### Example 5- Antitumor responses of monotherapy and combination therapy using #110H1L4hIgG4P and sacituzumab govitecan in an HCC-1806 xenograft model (triple negative breast cancer without BRACA1 mutation)

### 5)-1 Rationale and objectives

The inventors investigated whether a combination therapy using an anti-CD147 antibody and anti-TROP2 antibody-drug conjugate could suppress tumor growth to a greater extent than the corresponding monotherapies in an HCC-1806 xenograft model.

### 5)-2 Materials and Methods

HCC-1806 cells (ATCC, catalogue#CRL-2335) (a breast cancer cell line without a BRACA1 mutation), were cultured in ATCC-modified RPMI1640 with 10% FBS. Five million cells were injected subcutaneously into the right flank of female, 6-week-old Scid mice for tumor implantation. Following tumor randomization, the anti-TROP2 antibody-drug conjugate sacituzumab govitecan (Trodelvy^{®}, Gilead Sciences) was administered intravenously once at a dose of 3 mg/kg on day 3 post tumor inoculation when the tumor reached a volume of about 140 mm³(n=6). The #110H1L4hIgG4P was administered intravenously at a dose of 20 mg/kg weekly for two weeks, starting on day 4 post tumor inoculation (n=6). The tumor volume in each group of mice was determined twice per week.

### 5)-3 Results

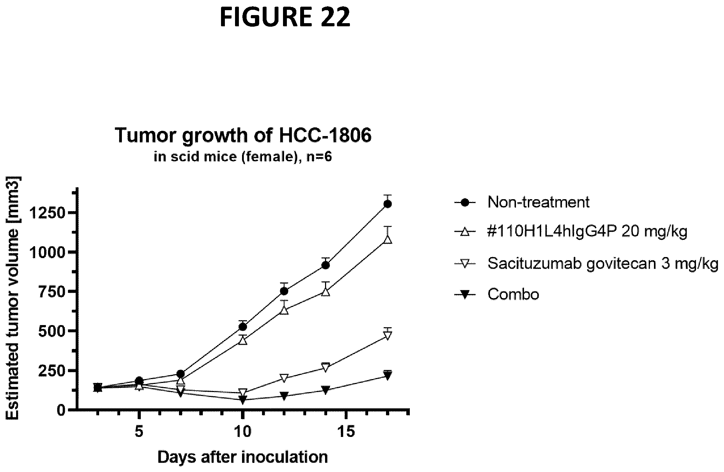

The *in vivo* efficacy of the combination therapy of #110H1L4hIgG4P and sacituzumab govitecan was evaluated in the HCC-1806 subcutaneous xenograft model. Treating tumor-bearing mice with #110H1L4hIgG4P, sacituzumab govitecan, and the combination resulted in a tumor growth inhibition (TGI) of 17%, 64%, and 83%, respectively, compared to the non-treatment control on day 17, as shown in Fig. 22. The combination therapy synergistically enhanced antitumor effects compared to the corresponding monotherapies, as indicated by a combination index of 0.84.

### Example 6 - Antitumor responses of monotherapy and combination therapy using #110H1L4hIgG4P and T-DXd in an SNU-16 xenograft model (gastric cancer).

### 6)-1 Rationale and objectives

The inventors investigated whether a combination therapy using an anti-CD147 antibody and anti-HER2 antibody-drug conjugate can suppress tumor growth to a greater extent than the corresponding monotherapies in an SNU-16 xenograft model.

### 6)-2 Materials and Methods

SNU-16 cells (ATCC, catalogue#CRL-5974) (gastric carcinoma cell line) were cultured in RPMI1640 medium with 10% FBS. Five million cells were injected subcutaneously into the right flank of female, 5-week-old NODscid mice for tumor implantation. Following tumor randomization, a control antibody-drug conjugate(comprising a control anti-LPS antibody conjugated to DXd) or T-DXd was administered intravenously once at a dose of 10 mg/kg on day 10 post tumor inoculation when the tumor reached a volume of about 130 mm³. A control anti-LPS antibody comprising a hIgG4P constant region or #110H1L4hIgG4P was administered intravenously at a dose of 10 mg/kg weekly for two weeks, starting on day 10 post tumor inoculation. The amino acid sequences of the LPS-binding variable domains are described WO2015/046505 and the hIgG4P constant region is set out in SEQ ID NOs: 13 and 14 as provided herein.

### 6)-3 Results

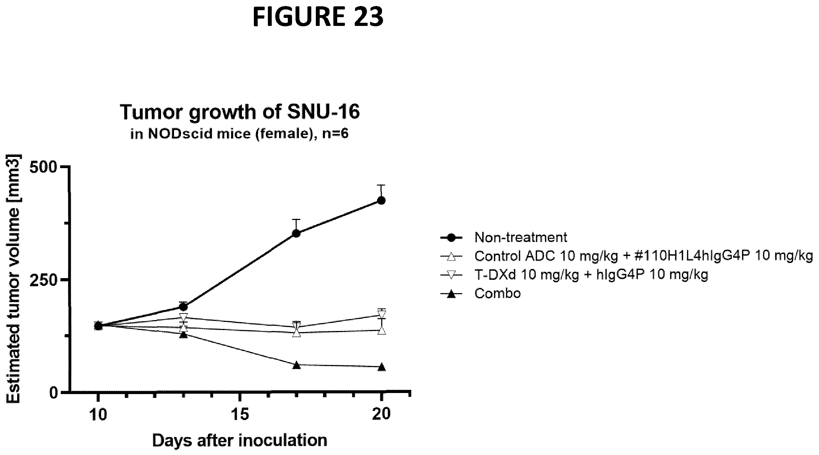

The in vivo efficacy of the combination therapy of #110H1L4hIgG4P and T-DXd was evaluated in the SNU-16 subcutaneous xenograft model. Tumor-bearing mice receiving the control antibody-drug conjugate and #110H1L4hIgG4P treatment, T-DXd and the control anti-LPS hIgG4P antibody treatment, and the T-DXd and #110H1L4hIgG4P combination treatment according to the present disclosure, demonstrated a tumor growth inhibition (TGI) of 68%, 60%, and 87%, respectively, compared to the non-treatment control on day20 as shown in Fig. 23. The administration of T-DXd and hIgG4P, or #110H1L4hIgG4P and control antibody-drug conjugate, resulted in stable disease (as determined by a maintained tumor volume), whilst the combination therapy of T-DXd and #110H1L4hIgG4P according to the present disclosure advantageously demonstrated tumor regression.

### Free Text of Sequence Listing

SEQ ID NO: 1 - Amino acid sequence of variant 1 of human CD147
SEQ ID NO: 2 - Nucleotide sequence of variant 1 of human CD147
SEQ ID NO: 3 - Amino acid sequence of variant 2 of human CD147
SEQ ID NO: 4 - Nucleotide sequence of variant 2 of human CD147
SEQ ID NO: 5 - Amino acid sequence of the heavy chain variable region of rat_CD147_#110(r#110)
SEQ ID NO: 6 - Amino acid sequence of the light chain variable region of rat_CD147_#110(r#110)
SEQ ID NO: 7 - Amino acid sequence of CDRH1 of
   rat_CD147_#110(r#110)
SEQ ID NO: 8 - Amino acid sequence of CDRH2 of
   rat_CD147_#110(r#110)
SEQ ID NO: 9 - Amino acid sequence of CDRH3 of
   rat_CD147_#110(r#110)
SEQ ID NO: 10 - Amino acid sequence of CDRL1 of
   rat_CD147_#110(r#110)
SEQ ID NO: 11 - Amino acid sequence of CDRL2 of
   rat_CD147_#110(r#110)
SEQ ID NO: 12 - Amino acid sequence of CDRL3 of
   rat_CD147_#110(r#110)
SEQ ID NO: 13 - Amino acid sequence of #110H1hIgG4P
SEQ ID NO: 14 - Amino acid sequence of #110H13hIgG4P
SEQ ID NO: 15 - Amino acid sequence of #110L4h
SEQ ID NO: 16 - Amino acid sequence of #110L2h
SEQ ID NO: 17 - Amino acid sequence of #110L12h
SEQ ID NO: 18 - Amino acid sequence of mu3 region of human CD147v1
SEQ ID NO: 19 - Amino acid sequence of the heavy chain variable region of rat_CD147_#84(r#84)
SEQ ID NO: 20 - Amino acid sequence of the light chain variable region of rat_CD147_#84(r#84)
SEQ ID NO: 21 - Amino acid sequence of CDRH1 of
   rat_CD147_#84(r#84)
SEQ ID NO: 22 - Amino acid sequence of CDRH2 of
   rat_CD147_#84(r#84
SEQ ID NO: 23 - Amino acid sequence of CDRH3 of
   rat_CD147_#84(r#84)
SEQ ID NO: 24 - Amino acid sequence of CDRL1 of
   rat_CD147_#84(r#84)
SEQ ID NO: 25 - Amino acid sequence of CDRL2 of
   rat_CD147_#84 (r#84)
SEQ ID NO: 26 - Amino acid sequence of CDRL3 of
   rat_CD147_#84 (r#84)
SEQ ID NO: 27 - Amino acid sequence of #84H1hIgG2
SEQ ID NO: 28 - Amino acid sequence of #84H1hIgG4P
SEQ ID NO: 29 - Amino acid sequence of #84L2h
SEQ ID NO: 30 - Amino acid sequence of a heavy chain CDRH1 of anti-TROP2 antibody [= amino acid residues 50 to 54 of SEQ ID NO: 38]
SEQ ID NO: 31 - Amino acid sequence of a heavy chain CDRH2 of anti-TROP2 antibody [= amino acid residues 69 to 85 of SEQ ID NO: 38]
SEQ ID NO: 32 - Amino acid sequence of a heavy chain CDRH3 of anti-TROP2 antibody [= amino acid residues 118 to 129 of SEQ ID NO: 38]
SEQ ID NO: 33 - Amino acid sequence of a light chain CDRL1 of anti-TROP2 antibody [= amino acid residues 44 to 54 of SEQ ID NO: 39]
SEQ ID NO: 34 - Amino acid sequence of a light chain CDRL2 of anti-TROP2 antibody [= amino acid residues 70 to 76 of SEQ ID NO: 39]
SEQ ID NO: 35 - Amino acid sequence of a light chain CDRL3 of anti-TROP2 antibody [= amino acid residues 109 to 117 of SEQ ID NO: 39]
SEQ ID NO: 36 - Amino acid sequence of a heavy chain variable region of anti-TROP2 antibody [= amino acid residues 20 to 140 of SEQ ID NO: 38]
SEQ ID NO: 37 - Amino acid sequence of a light chain variable region of anti-TROP2 antibody [= amino acid residues 21 to 129 of SEQ ID NO: 39]
SEQ ID NO: 38 - Amino acid sequence of a heavy chain of anti-TROP2 antibody
SEQ ID NO: 39 - Amino acid sequence of a light chain of anti-TROP2 antibody
SEQ ID NO: 40 - Amino acid sequence of a heavy chain of anti-TROP2 antibody [= amino acid residues 20 to 470 of SEQ ID NO: 38]
SEQ ID NO: 41 - Amino acid sequence of a light chain of anti-TROP2 antibody [= amino acid residues 21 to 234 of SEQ ID NO: 39]
SEQ ID NO: 42 - Amino acid sequence of a heavy chain of anti-TROP2 antibody [= amino acid residues 20 to 469 of SEQ ID NO: 38]
SEQ ID NO: 43 - Amino acid sequence of a heavy chain CDRH1 of anti-HER2 antibody [= amino acid residues 26 to 33 of SEQ ID NO 51]
SEQ ID NO: 44 - Amino acid sequence of a heavy chain CDRH2 of anti-HER2 antibody [= amino acid residues 51 to 58 of SEQ ID NO: 51]
SEQ ID NO: 45 - Amino acid sequence of a heavy chain CDRH3 of anti-HER2 antibody [= amino acid residues 97 to 109 of SEQ ID NO: 51]
SEQ ID NO: 46 - Amino acid sequence of a light chain CDRL1 of anti-HER2 antibody [= amino acid residues 27 to 32 of SEQ ID NO: 52]
SEQ ID NO: 47 - Amino acid sequence of a light chain CDRL2 of anti-HER2 antibody [= amino acid residues 50 to 56 of SEQ ID NO: 52]
SEQ ID NO: 48 - Amino acid sequence of a light chain CDRL3 of anti-HER2 antibody [= amino acid residues 89 to 97 of SEQ ID NO: 52]
SEQ ID NO: 49 - Amino acid sequence of a heavy chain variable region of an anti-HER2 antibody [= amino acid residues 1 to 120 of SEQ ID NO: 51]
SEQ ID NO: 50 - Amino acid sequence of a light chain variable region of an anti-HER2 antibody [= amino acid residues 1 to 107 of SEQ ID NO: 52]
SEQ ID NO: 51 - Amino acid sequence of a heavy chain of an anti-HER2 antibody
SEQ ID NO: 52 - Amino acid sequence of a light chain of an anti-HER2 antibody
SEQ ID NO: 53 - Amino acid sequence of linker in antibody-drug conjugate
SEQ ID NO: 54: Amino acid sequence of heavy chain of Sacituzumab govitecan
SEQ ID NO: 55: Amino acid sequence of light chain of Sacituzumab govitecan

## Claims

1. An anti-CD147 antibody for use in medicine,
wherein the anti-CD147 antibody is administered to a subject simultaneously or sequentially with an antibody-drug conjugate,
wherein the antibody moiety of the antibody-drug conjugate comprises an anti-TROP2 antibody, and
wherein the drug moiety of the antibody-drug conjugate comprises a topoisomerase I inhibitor.

2. An anti-CD147 antibody for use in medicine,
wherein the anti-CD147 antibody is administered to a subject simultaneously or sequentially with an antibody-drug conjugate,
wherein the antibody moiety of the antibody-drug conjugate comprises an anti-HER2 antibody, and
wherein the drug moiety of the antibody-drug conjugate comprises a Topoisomerase I inhibitor.

3. A combination of an anti-CD147 antibody and an antibody-drug conjugate for use in medicine,
wherein the anti-CD147 antibody is administered to a subject simultaneously or sequentially with an antibody-drug conjugate,
wherein the antibody moiety of the antibody-drug conjugate comprises an anti-TROP2 or an anti-HER2 antibody, and
wherein the drug moiety of the antibody-drug conjugate comprises a topoisomerase I inhibitor.

4. The anti-CD147 antibody for use according to claim 1 or claim 2, or the combination for use according to claim 3, wherein the use comprises the treatment and/or prevention of a cancer in the subject.

5. The anti-CD147 antibody for use according to any one of claims 1, 2 and 4, or the combination for use according to claim 3 or claim 4, wherein the anti-CD147 antibody is human or humanised.

6. The anti-CD147 antibody for use according to any one of claims 1, 2, 4 and 5, or the combination for use according to any one of claims 3 to 5, wherein the anti-CD147 antibody comprises a heavy chain variable region and a light chain variable region,
wherein the heavy chain variable region comprises CDRH1, CDRH2 and CDRH3,
wherein the CDRH1 consists of an amino acid sequence represented by SEQ ID NO: 7, the CDRH2 consists of an amino acid sequence represented by SEQ ID NO: 8, and the CDRH3 consists of an amino acid sequence represented by SEQ ID NO: 9,
wherein the light chain variable region comprises CDRL1, CDRL2 and CDRL3, and
wherein the CDRL1 consists of an amino acid sequence represented by SEQ ID NO: 10, the CDRL2 consists of an amino acid sequence represented by SEQ ID NO: 11, and the CDRL3 consists of an amino acid sequence represented by SEQ ID NO: 12.

7. The anti-CD147 antibody for use according to any one of claims 1, 2, and 4 to 6, or the combination for use according to any one of claims 3 to 6, wherein the anti-CD147 antibody comprises the following (a) and (b):
(a) a heavy chain variable region selected from the group consisting of the following (a1) to (a4):
(a1) a heavy chain variable region consisting of amino acid residues at positions 20 to 136 in an amino acid sequence represented by SEQ ID NO: 13;
(a2) a heavy chain variable region consisting of amino acid residues at positions 20 to 136 in an amino acid sequence represented by SEQ ID NO: 14;
(a3) an amino acid sequence having at least 80% or more sequence identity with the sequence of a framework region outside each CDR sequence in the sequence of (a1) or (a2); and
(a4) an amino acid sequence in which one or several amino acids are deleted, substituted, or added in the sequence of a framework region outside each CDR sequence in the sequence according to any one of (a1) to (a3); and
(b) a light chain variable region selected from the group consisting of the following (b1) to (b5):
(b1) a light chain variable region consisting of amino acid residues at positions 21 to 128 in an amino acid sequence represented by SEQ ID NO: 15;
(b2) a light chain variable region consisting of amino acid residues at positions 21 to 128 in an amino acid sequence represented by SEQ ID NO: 16;
(b3) a light chain variable region consisting of amino acid residues at positions 21 to 128 in an amino acid sequence represented by SEQ ID NO: 17;
(b4) an amino acid sequence having at least 80% or more sequence identity with the sequence of a framework region outside each CDR sequence in the sequence according to any one of (b1) to (b3); and
(b5) an amino acid sequence in which one or several amino acids are deleted, substituted, or added in the sequence of a framework region outside each CDR sequence in the sequence according to any one of (b1) to (b4).

8. The anti-CD147 antibody for use according to claim 7, or the combination for use according to claim 7, wherein the anti-CD147 antibody comprises a heavy chain variable region and a light chain variable region selected from the following (A) to (C):
(A) a heavy chain variable region consisting of amino acid residues at positions 20 to 136 in the amino acid sequence represented by SEQ ID NO: 13, and a light chain variable region consisting of amino acid residues at positions 21 to 128 in the amino acid sequence represented by SEQ ID NO: 15;
(B) a heavy chain variable region consisting of amino acid residues at positions 20 to 136 in the amino acid sequence represented by SEQ ID NO: 14, and a light chain variable region consisting of amino acid residues at positions 21 to 128 in the amino acid sequence represented by SEQ ID NO: 16; and
(C) a heavy chain variable region consisting of amino acid residues at positions 20 to 136 in the amino acid sequence represented by SEQ ID NO: 14, and a light chain variable region consisting of amino acid residues at positions 21 to 128 in the amino acid sequence represented by SEQ ID NO: 17.

9. The anti-CD147 antibody for use according to claim 8, or the combination for use according to claim 8, wherein the anti-CD147 antibody comprises a heavy chain variable region consisting of amino acid residues at positions 20 to 136 in the amino acid sequence represented by SEQ ID NO: 13, and a light chain variable region consisting of amino acid residues at positions 21 to 128 in the amino acid sequence represented by SEQ ID NO: 15.

10. The anti-CD147 antibody for use according to claim 7 or claim 8, or the combination for use according to claim 7 or claim 8, wherein the anti-CD147 antibody comprises a heavy chain and a light chain selected from one of the following (D) to (F):
(D) a heavy chain consisting of amino acid residues at positions 20 to 463 in the amino acid sequence represented by SEQ ID NO: 13, and a light chain consisting of amino acid residues at positions 21 to 234 in the amino acid sequence represented by SEQ ID NO: 15;
(E) a heavy chain consisting of amino acid residues at positions 20 to 463 in the amino acid sequence represented by SEQ ID NO: 14, and a light chain consisting of amino acid residues at positions 21 to 234 in the amino acid sequence represented by SEQ ID NO: 16; and
(F) a heavy chain consisting of amino acid residues at positions 20 to 463 in the amino acid sequence represented by SEQ ID NO: 14, and a light chain consisting of amino acid residues at positions 21 to 234 in the amino acid sequence represented by SEQ ID NO: 17.

11. The anti-CD147 antibody for use according to claim 10, or the combination for use according to claim 10, wherein the anti-CD147 antibody comprises a heavy chain consisting of amino acid residues at positions 20 to 463 in the amino acid sequence represented by SEQ ID NO: 13, and a light chain consisting of amino acid residues at positions 21 to 234 in the amino acid sequence represented by SEQ ID NO: 15.

12. The anti-CD147 antibody for use according to any one of claims 1, 2, 4 and 5, or the combination for use according to any one of claims 3 to 5, wherein the anti-CD147 antibody competes with an anti-CD147 antibody comprising a heavy chain variable region consisting of an amino acid sequence represented by SEQ ID NO: 5, and a light chain variable region consisting of an amino acid sequence represented by SEQ ID NO: 6.

13. The anti-CD147 antibody for use according to any one of claims 1, 2, 4 and 5, or the combination for use according to any one of claims 3 to 5, wherein the anti-CD147 antibody binds to an epitope to which the following antibody binds:
an anti-CD147 antibody comprising a heavy chain variable region consisting of an amino acid sequence represented by SEQ ID NO: 5, and a light chain variable region consisting of an amino acid sequence represented by SEQ ID NO: 6.

14. The anti-CD147 antibody for use according to any one of claims 1, 2, 4 to 9, and 12 to 13, or the combination for use according to any one of claims 3 to 9, and 12 to 13, wherein the anti-CD147 antibody comprises an antigen-binding fragment that is selected from the group consisting of Fab, F(ab')2, Fab', diabody and Fv.

15. The anti-CD147 antibody for use according to any one of claims 1, 2, 4 to 9, and 12 to 13, or the combination for use according to any one of claims 3 to 9, and 12 to 13, wherein the anti-CD147 antibody comprises an antigen-binding fragment that is scFv.

16. The anti-CD147 antibody for use according to any one of claims 1, 2, 4 to 6, and 12 to 13, or the combination for use according to any one of claims 3 to 6, and 12 to 13, wherein the anti-CD147 antibody comprises an antigen-binding fragment that is VHH.

17. The anti-CD147 antibody for use according to any one of claims 1, 2 and 4 to 16, or the combination for use according to any one of claims 3 to 16, wherein a topoisomerase I inhibitor represented by the following formula is released from the antibody-drug conjugate to express an antitumor activity:

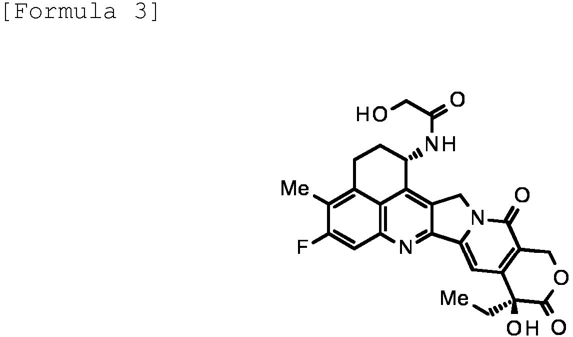

18. The anti-CD147 antibody for use according to any one of claims 1, 2 and 4 to 17, or the combination for use according to any one of claims 3 to 17,
wherein antibody-drug conjugate comprises a linker and a drug, the linker and drug forming a drug-linker,
wherein the drug represented by the following formula is conjugated to the antibody of the antibody-drug conjugate via the linker with the nitrogen atom of the amino group at position 1 as a connecting position,
preferably, wherein the drug-linker is conjugated to the antibody via a thioether bond,
and more preferably, wherein the linker comprises a tetrapeptide sequence of amino acid residues -Gly-Gly-Phe-Gly- (SEQ ID NO: 53):

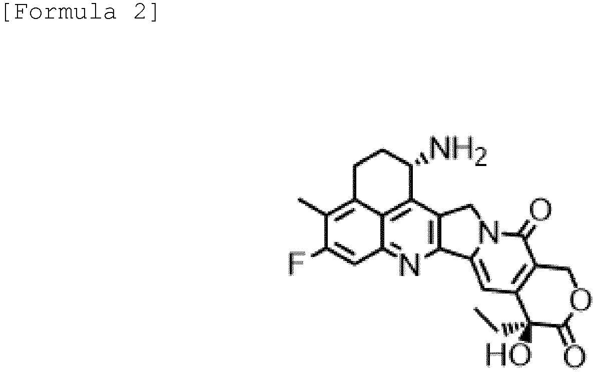

19. The anti-CD147 antibody for use according to any one of claims 1 and 4 to 18 as dependent on claim 1, or the combination for use according to any one of claims 3 and 4 to 18 as dependent on claim 3, wherein the anti-TROP2 antibody-drug conjugate comprises a linker and a drug, the anti-TROP2 antibody being connected to the drug via the linker, the linker and drug forming a drug-linker, and wherein the drug-linker is represented by the following formula: 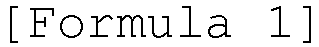 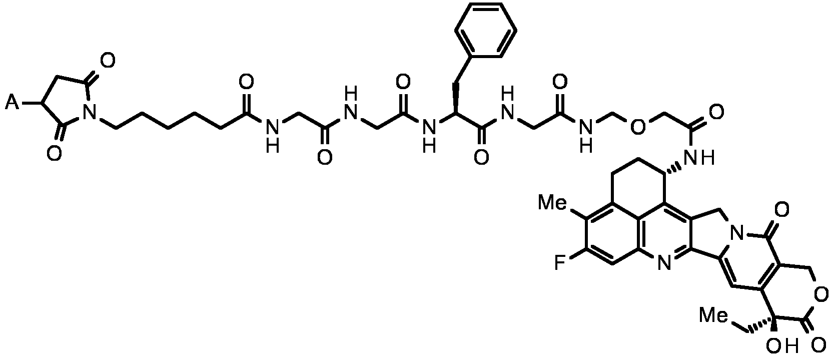 wherein A represents a connecting position to the anti-TROP antibody, and wherein the drug-linker is conjugated to the anti-TROP2 antibody via a thioether bond.

20. The anti-CD147 antibody for use according to any one of claims 1, 4 to 18 as dependent on claim 1, and claim 19, or the combination for use according to any one of 3, 4 to 18 as dependent on claim 3, and claim 19, wherein the anti-TROP2 antibody comprises a heavy chain variable region and a light chain variable region,
wherein the heavy chain variable region comprises CDRH1, CDRH2 and CDRH3,
wherein the CDRH1 consists of an amino acid sequence represented by SEQ ID NO: 30, the CDRH2 consists of an amino acid sequence represented by SEQ ID NO: 31, and the CDRH3 consists of an amino acid sequence represented by SEQ ID NO: 32,
wherein the light chain variable region comprises CDRL1, CDRL2 and CDRL3, and
wherein the CDRL1 consists of an amino acid sequence represented by SEQ ID NO: 33, the CDRL2 consists of an amino acid sequence represented by SEQ ID NO: 34, and the CDRL3 consists of an amino acid sequence represented by SEQ ID NO: 35.

21. The anti-CD147 antibody for use according to claim 20, or the combination for use according to claim 20, wherein the anti-TROP2 antibody comprises:
a heavy chain variable region consisting of an amino acid sequence represented by SEQ ID NO: 36, and
a light chain variable region consisting of an amino acid sequence represented by SEQ ID NO: 37.

22. The anti-CD147 antibody for use according to claim 20 or 21, or the combination for use according to claim 20 or 21, wherein the anti-TROP2 antibody comprises:
a heavy chain consisting of an amino acid sequence represented by SEQ ID NO: 38 and a light chain consisting of an amino acid sequence represented by SEQ ID NO: 39.

23. The anti-CD147 antibody for use according to any one of claims 19 to 22, or the combination for use according to any one of claims 19 to 22, wherein the average number of units of the drug-linker conjugated per anti-TROP2 antibody molecule in the anti-TROP2 antibody-drug conjugate is in the range of from 2 to 8, and preferably, in the range of from 3.5 to 4.5.

24. The anti-CD147 antibody for use according to any one of claims 19 to 23, or the combination for use according to any one of claims 19 to 23, wherein the anti-TROP2 antibody-drug conjugate is datopotamab deruxtecan.

25. The anti-CD147 antibody for use according to any one of claims 2 and 4 to 18 as dependent on claim 2, or the combination for use according to any one of claims 3 and 4 to 18 as dependent on claim 3, wherein the anti-HER2 antibody-drug conjugate comprises a linker and a drug, the anti-HER2 antibody being connected to the drug via the linker, the linker and drug forming a drug-linker, and wherein the drug-linker is represented by the following formula: 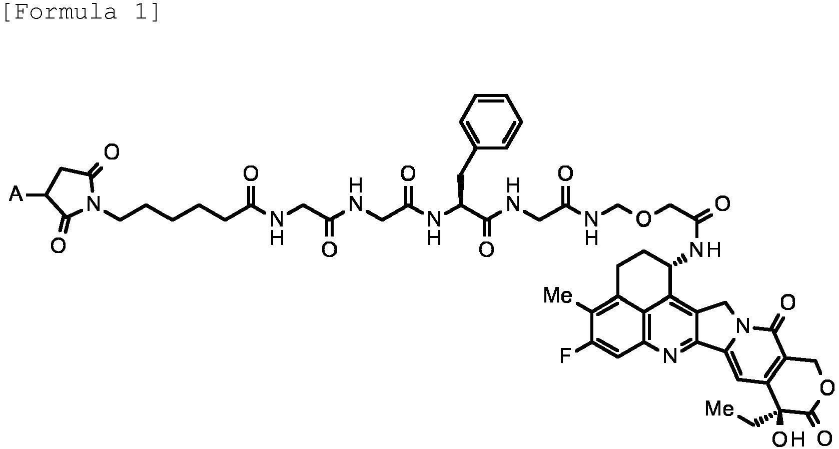 wherein A represents a connecting position to the anti-HER2 antibody, and wherein the drug-linker is conjugated to the anti-HER2 antibody via a thioether bond.

26. The anti-CD147 antibody for use according to any one of claims 2, 4 to 18 as dependent on claim 2, and claim 25, or the combination for use according to any one of claims 2, 4 to 18 as dependent on claim 2, and claim 25, wherein the anti-HER2 antibody comprises a heavy chain variable region and a light chain variable region,
wherein the heavy chain variable region comprises CDRH1, CDRH2 and CDRH3,
wherein the CDRH1 consists of an amino acid sequence represented by SEQ ID NO: 43, the CDRH2 consists of an amino acid sequence represented by SEQ ID NO: 44, and the CDRH3 consists of an amino acid sequence represented by SEQ ID NO: 45,
wherein the light chain variable region comprises CDRL1, CDRL2 and CDRL3, and
wherein the CDRL1 consists of an amino acid sequence represented by SEQ ID NO: 46, the CDRL2 consists of an amino acid sequence represented by SEQ ID NO: 47, and the CDRL3 consists of an amino acid sequence represented by SEQ ID NO: 48.

27. The anti-CD147 antibody for use according to claim 26, or the combination for use according to claim 26, wherein the anti-HER2 antibody comprises:
a heavy chain variable region consisting of an amino acid sequence represented by SEQ ID NO: 49, and
a light chain variable region consisting of an amino acid sequence represented by SEQ ID NO: 50.

28. The anti-CD147 antibody for use according to claim 26 or 27, or the combination for use according to claim 26 or 27, wherein the anti-HER2 antibody comprises:
a heavy chain consisting of an amino acid sequence represented by SEQ ID NO: 51 and a light chain consisting of an amino acid sequence represented by SEQ ID NO: 52.

29. The anti-CD147 antibody for use according to any one of claims 25 to 28, or the combination for use according to any one of claims 25 to 28, wherein the average number of units of the drug-linker conjugated per anti-HER2 antibody molecule in the anti-HER2 antibody-drug conjugate is in the range of from 2 to 8, preferably in the range of from 7 to 8, and more preferably, in the range of from 7.5 to 8.

30. The anti-CD147 antibody for use according to any one of claims 25 to 29, or the combination for use according to any one of claims 25 to 29, wherein the anti-HER2 antibody-drug conjugate is trastuzumab deruxtecan.

31. The anti-CD147 antibody for use according to any one of claims 1, 2 and 4 to 16, or the combination for use according to any one of claims 3 to 16, wherein the anti-TROP2 antibody-drug conjugate or the anti-HER2 antibody drug conjugate comprises a linker and a drug, the anti-TROP2 antibody or the anti-HER2 antibody being connected to the drug via the linker, the linker and drug forming a drug-linker, and wherein the drug-linker is represented by the following formula: 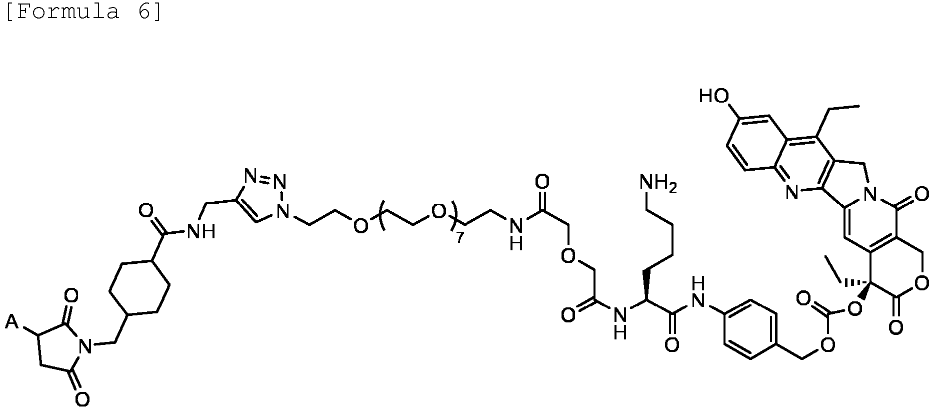 wherein A represents a connecting position to the anti-TROP antibody or the anti-HER2 antibody, and wherein the drug-linker is conjugated to the anti-TROP2 antibody or the anti-HER2 antibody via a thioether bond.

32. The anti-CD147 antibody for use according to claim 31, or the combination for use according to claim 31, wherein A represents the connecting position to an anti-TROP antibody, and wherein the anti-TROP2 antibody-drug conjugate comprises a heavy chain amino acid sequence according to SEQ ID NO: 54, and a light chain amino acid sequence according to SEQ ID NO: 55 (sacituzumab govitecan).

33. The anti-CD147 antibody for use according to any one of claims 1, 2 and 4 to 32, or the combination for use according to any one of claims 3 to 32, wherein the anti-CD147 antibody, anti-TROP2 antibody and/or anti-HER2 antibody each comprises a heavy chain, and wherein 1 or 2 amino acid residues are deleted from the carboxyl terminus of the heavy chain,
optionally, wherein the anti-CD147 antibody, anti-TROP2 antibody and/or anti-HER2 antibody each comprises a first and a second heavy chain, and wherein each of the first and the second heavy chains has a deletion of one amino acid residue at the carboxyl terminus.

34. The anti-CD147 antibody for use according to any one of claims 1, 2 and 4 to 33, or the combination for use according to any one of claims 3 to 33, wherein the anti-CD147 antibody, anti-TROP2 antibody and/or anti-HER2 antibody each comprises a heavy chain, and wherein an amino acid residue at the carboxyl terminus of the heavy chain is amidated.

35. The anti-CD147 antibody for use according to any one of claims 4 to 24, and 31 to 34, or the combination for use according to any one of claims 4 to 24, and 31 to 34, wherein the antibody-drug conjugate comprises an anti-TROP2 antibody-drug conjugate, and wherein the cancer is selected from the group consisting of: breast cancer (including triple negative breast cancer and hormone receptor (HR)-positive, HER2-negative breast cancer), lung cancer (including small cell lung cancer and non-small cell lung cancer), colorectal cancer (also called colon and rectal cancer, and including colon cancer and rectal cancer), gastric cancer (also called gastric adenocarcinoma), esophageal cancer, head-and-neck cancer (including salivary gland cancer and pharyngeal cancer), esophagogastric junction adenocarcinoma, biliary tract cancer (including bile duct cancer), Paget's disease, pancreatic cancer, ovarian cancer, uterine carcinosarcoma, urothelial cancer, prostate cancer, bladder cancer, gastrointestinal stromal tumor, cervical cancer (including uterine cervix cancer), squamous cell carcinoma, peritoneal cancer, liver cancer, hepatocellular cancer, corpus uteri carcinoma, kidney cancer, vulval cancer, thyroid cancer, penis cancer, leukemia, malignant lymphoma, plasmacytoma, myeloma, glioblastoma multiforme, osteosarcoma, sarcoma, endometrial cancer, and melanoma;
preferably wherein the cancer is selected from non-small cell lung cancer, triple negative breast cancer, hepatocellular cancer, pancreatic ductal adenocarcinoma, and colorectal cancer;
and more preferably, wherein the cancer is selected from triple negative breast cancer and non-small cell lung cancer.

36. The anti-CD147 antibody for use according to any one of claims 4 to 18, 25 to 31 and 33 to 34, or the combination for use according to any one of claims 4 to 18, 25 to 31 and 33 to 34, wherein the antibody-drug conjugate comprises an anti-HER2 antibody-drug conjugate, and wherein the cancer is selected from the group consisting of: breast cancer (including hormone receptor (HR)-positive breast cancer), lung cancer (including small cell lung cancer and non-small cell lung cancer), colorectal cancer (also called colon and rectal cancer, and including colon cancer and rectal cancer), gastric cancer (also called gastric adenocarcinoma), esophageal cancer, head-and-neck cancer (including salivary gland cancer and pharyngeal cancer), esophagogastric junction adenocarcinoma, biliary tract cancer (including bile duct cancer), Paget's disease, pancreatic cancer including pancreatic ductal adenocarcinoma, ovarian cancer, uterine carcinosarcoma, urothelial cancer, prostate cancer, bladder cancer, gastrointestinal stromal tumor, cervical cancer (including uterine cervix cancer), squamous cell carcinoma, peritoneal cancer, liver cancer, hepatocellular cancer, corpus uteri carcinoma, kidney cancer, vulval cancer, thyroid cancer, penis cancer, leukemia, malignant lymphoma, plasmacytoma, myeloma, glioblastoma multiforme, osteosarcoma, sarcoma, endometrial cancer, and melanoma; preferably wherein the cancer is selected from the group consisting of HER2-positive breast cancer, lung cancer including non-small cell lung cancer, gastric cancer, hepatocellular cancer, pancreatic ductal adenocarcinoma, and colorectal cancer;
and more preferably, wherein the cancer is gastric cancer.

37. The anti-CD147 antibody for use according to any one of claims 4 to 34, or the combination for use according to any one of claims 4 to 34, wherein the cancer is selected from: non-small cell lung cancer; breast cancer, preferably triple negative breast cancer, and more preferably, triple negative breast cancer without a BRACA1 mutation; and gastric cancer.

38. The anti-CD147 antibody for use according to any one of claims 1, 2 and 4 to 37, or the combination for use according to any one of claims 3 to 37, wherein the anti-CD147 antibody and the antibody-drug conjugate are contained separately in different formulations for simultaneous or sequential administration, or wherein the anti-CD147 antibody and the antibody-drug conjugate are contained in the same formulation for simultaneous administration.

39. The anti-CD147 antibody for use according to any one of claims 1, 2 and 4 to 38, or the combination for use according to any one of claims 3 to 38, wherein the anti-CD147 antibody is administered to the subject at a dosage of from about 0.1 to about 50 mg/kg, or from about 1 to about 40 mg/kg, or about 20 mg/kg, about 40 mg/kg, about 45 mg/kg, or about 50 mg/kg, optionally,
wherein the anti-CD147 antibody is administered to the subject daily, every other day, twice per week, three times per week, four times per week, five times per week, six times per week, once per week, once every two weeks, once every three weeks, once every four weeks, once every five weeks, once every six weeks, once every seven weeks, once every eight weeks, once every nine weeks, once every ten weeks, once every eleven weeks, once every twelve weeks, twice per year, or once per year.

40. A pharmaceutical composition comprising:
i) an anti-CD147 antibody; and
ii) an antibody-drug conjugate;
wherein the antibody moiety of the antibody-drug conjugate comprises an anti-TROP2 antibody or an anti-HER2 antibody, and
wherein the drug moiety of the antibody-drug conjugate comprises a topoisomerase I inhibitor.

41. A kit suitable for the prevention and/or treatment of a cancer comprising:
i) an anti-CD147 antibody; and
ii) an antibody-drug conjugate;
wherein the antibody moiety of the antibody-drug conjugate comprises an anti-TROP2 antibody or an anti-HER2 antibody, and
wherein the drug moiety of the antibody-drug conjugate comprises a topoisomerase I inhibitor.

42. The pharmaceutical composition according to claim 40, or the kit according to claim 41, wherein the anti-CD147 antibody is as defined in any one of claims 5 to 16 and 33 to 34, wherein the anti-TROP2 antibody-drug conjugate is as defined in any one of claims 17 to 24 and 31 to 34, and/or wherein the anti-HER2 antibody-drug conjugate is as defined in any one of claims 17, 18, 25 to 31, 33 and **34.**
